# EUROPEAN PATENT SPECIFICATION

(11) **EP 1 907 403 B1**
(45) Date of publication and mention of the grant of the patent: **25.12.2013**
(21) Application number: 06787506.2
(22) Date of filing: 14.07.2006
(51) Int. Cl.: C07F 9/572, A61P 31/12, C07D 417/14, A61K 31/4709, A61K 31/662, C07K 5/087, A61K 38/21

(54) **ANTIVIRAL COMPOUNDS**
ANTIVIRALE VERBINDUNGEN
COMPOSES ANTIVIRAUX

(30) Priority: 14.07.2005 US 699096 P; 18.07.2005 US 700559 P
(43) Date of publication of application: 09.04.2008
(73) Proprietor: GILEAD SCIENCES, INC., Foster City CA 94404 (US)
(72) Inventor: CHO, Aesop, Mountain View, CA 94040 (US); KIM, Choung, U., San Carlos, CA 94070 (US); SHENG, Xiaoning, C., Foster City, CA 94404 (US)
(74) Representative: Reitstötter - Kinzebach
(86) International application number: PCT/US2006/027605
(87) International publication number: WO 2007/009109

(56) References cited:
- WO-A1-03/064416
- WO-A1-03/064456
- WO-A1-03/099274
- WO-A1-03/099316
- WO-A1-2004/103996
- WO-A1-2005/046712
- WO-A1-2005/051410
- WO-A1-2006/000085
- WO-A2-02/060926
- WO-A2-2005/054430
- WO-A2-2006/020276
- US-A1- 2003 186 895
- US-A1- 2003 187 018
- US-A1- 2004 077 551
- NI Z-J ET AL: "PROGRESS AND DEVELOPMENT OF SMALL MOLECULE HCV ANTIVIRALS" CURRENT OPINION IN DRUG DISCOVERY AND DEVELOPMENT, CURRENT DRUGS, LONDON, GB, vol. 7, no. 4, July 2004 (2004-07), pages 446-459, XP009037092 ISSN: 1367-6733
- ROENN, ROBERT ET AL: "Exploration of acyl sulfonamides as carboxylic acid replacements in protease inhibitors of the hepatitis C virus full-length NS3" BIOORGANIC & MEDICINAL CHEMISTRY , 14(2), 544-559 CODEN: BMECEP; ISSN: 0968-0896, 2006, XP002408481

## Description

### BACKGROUND OF THE INVENTION

Improving the delivery of drugs and other agents to target cells and tissues has been the focus of considerable research for many years. Though many attempts have been made to develop effective methods for importing biologically active molecules into cells, both *in vivo* and *in vitro,* none has proved to be entirely satisfactory. Optimizing the association of the inhibitory drug with its intracellular target, while minimizing intercellular redistribution of the drug, e.g., to neighboring cells, is often difficult or inefficient.

Most agents currently administered to a patient parenterally are not targeted, resulting in systemic delivery of the agent to cells and tissues of the body where it is unnecessary, and often undesirable. This may result in adverse drug side effects, and often limits the dose of a drug (e.g., glucocorticoids and other anti-inflammatory drugs) that can be administered. By comparison, although oral administration of drugs is generally recognized as a convenient and economical method of administration, oral administration can result in either (a) uptake of the drug through the cellular and tissue barriers, e.g., blood/brain, epithelial, cell membrane, resulting in undesirable systemic distribution, or (b) temporary residence of the drug within the gastrointestinal tract. Accordingly, a major goal has been to develop methods for specifically targeting agents to cells and tissues. Benefits of such treatment includes avoiding the general physiological effects of inappropriate delivery of such agents to other cells and tissues, such as uninfected cells.

Hepatitis C is recognized as a chronic viral disease of the liver which is characterized by liver disease. Although drugs targeting the liver are in wide use and have shown effectiveness, toxicity and other side effects have limited their usefulness.

Assay methods capable of determining the presence, absence or amounts of HCV are of practical utility in the search for inhibitors as well as for diagnosing the presence of HCV.

Inhibitors of HCV are useful to limit the establishment and progression of infection by HCV as well as in diagnostic assays for HCV.

Generally, there is a need for new HCV therapeutic agents. Certain agents may have improved inhibitory or pharmacokinetic properties, such as enhanced activity against development of viral resistance, improved oral bioavailability, greater potency or extended effective half-life *in vivo.* Particular compounds may have fewer side effects, less complicated dosing schedules, or be orally active.

WO 03/099274, WO 2004/103996, US 2004/077551 and WO 03/099316 disclose compounds which inhibit the functioning of the NS3 protease and which are therefore useful for treating hepatitis C virus.

### SUMMARY OF THE INVENTION

In one embodiment the present invention provides compounds, and compositions useful for inhibition of HCV, or that have therapeutic activity against HCV. Accordingly, the invention provides a compound, including enantiomers thereof, of formula II: 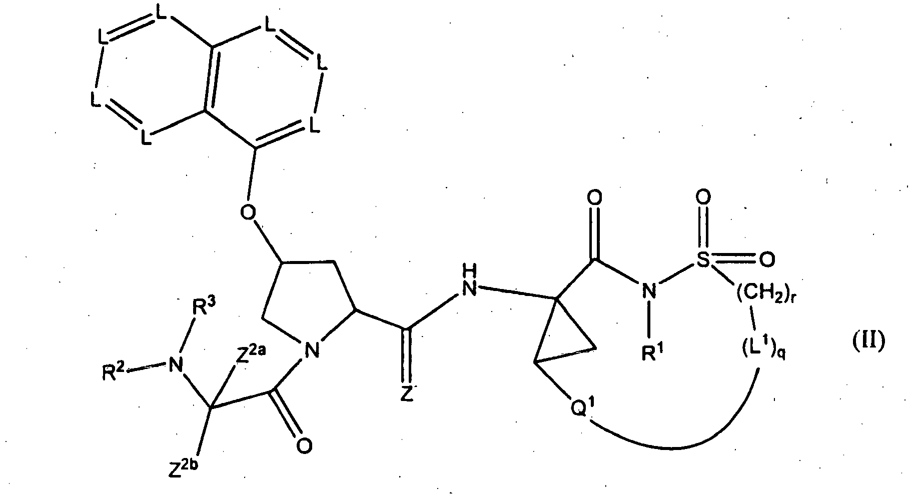 or a pharmaceutically acceptable salt, or solvate thereof, wherein:
R¹ is independently selected from H, alkyl, alkenyl, alkynyl, aryl, cycloalkyl, heterocycle, halogen, haloalkyl, alkylsulfonamido, arylsulfonamido, - C(O)NHS(O)₂-, or -S(O)₂-, optionally substituted with one or more A³;
R² is selected from
   a) -C(Y¹)(A³)
   b) (C2-10)alkyl, (C3-7)cycloalkyl or (C1-4)alkyl-(C3-7)cycloalkyl,
      where said cycloalkyl and alkyl-cycloalkyl may be mono-, di- or tri-substituted with (C1-3)alkyl, or
      where said alkyl, cycloalkyl and alkyl-cycloalkyl may be mono- or disubstituted with substituents selected from hydroxy and O-(C1-4)alkyl, or
      where each of said alkyl-groups may be mono-, di- or tri-substituted with halogen, or
      where each of said cycloalkyl groups being 5-, 6- or 7-membered, one or two -CH2-groups not being directly linked to each other may be replaced by -O- such that the O-atom is linked to the N atom to which R² is attached via at least two C-atoms, or
   c) phenyl, (C1-3)alkyl-phenyl, heteroaryl or (C1-3)alkyl-heteroaryl, wherein the heteroaryl-groups are 5- or 6-membered having from 1 to 3 heteroatoms selected from N, O and S, wherein said phenyl and heteroaryl groups may be mono-, di- or trisubstituted with substituents selected from halogen, - OH, (C1-4)alkyl, O-(C1-4)alkyl, S-(C1-4)alkyl, -NH2, -NH((C1-4)alkyl) and -N((Cl-4)alkyl)2, -CONH2 and -CONH-(C1-4)alkyl;
R³ is PRT, H or (C1-6)alkyl;
L is independently selected from C or N, providing there are no more than three consecutive N, each C being substituted with one or more A³;
L¹ is independently selected from C or N, providing there are no more than three consecutive N, each substituted with one or more A³;
Z is O, S, C or N, substituted with A³;
Z^{2a} is H, (C1-10)alkyl, (C2-10)allcenyl, (C2-10)alkynyl, wherein any carbon atom may be replaced with a heteroatom selected from O, S or N, or Z^{2a} optionally forms a carbocyle or heterocycle with Q¹, or any A³;
Z^{2b} is H, (C1-6)alkyl, (C2-8)alkenyl, (C2-8)alkynyl;
Q¹ is (C1)alkylene, (C2-8)alkenylene, or (C2-8)alkynylene;
A³ is independently selected from PRT H, -OH, -C(O)OH, - (CH₂)ₘ-, -C(O)O-, -NH-, cyano, alkyl, alkenyl, alkynyl, amino, amido, imido, imino, halogen, CF₃, CH₂CF₃, cycloalkyl, nitro, aryl, aralkyl, alkoxy, aryloxy, heterocycle, heteroaryl, -C(A²)₃, -C(A²)₂-C(O)A², -C(O)A², -C(O)OA², -O(A²), -N(A²)₂, -S(A²), -CH₂P(O)(A²)(OA²), -CH₂P(O)(A²)(N(A²)₂), -CH₂(O)(OA²)(OA²), -OCH₂P(O)(OA²)(OA²), -OCH₂P(O)(A²)(OA²), -OCH₂P(O)(A²)(N(A²)₂), -C(O)OCH₂P(O)(OA²)(OA²), -C(O)OCH₂P(O)(A²)(OA²), -C(O)OCH₂P(O)(A²)(N(A²)₂), -CH₂P(O)(OA²)(N(A²)₂), -OCH₂P(O)(OA²)(N(A²)₂), -C(O)OCH₂P(O)(OA²)(N(A²)₂), -CH₂P(O)(N(A²)₂)(N(A²)₂), -C(O)OCH₂P(O)(N(A²)₂)(N(A²)₂), -OCH₂P(O)(N(A²)₂)(N(A²)₂), -(CH₂)ₘ-heterocycle, -(CH₂)ₘC(O)Oalkyl, -O-(CH₂)ₘ-O-C(O)-Oalkyl, -O-(CH₂)ᵣ-O-C(O)-(CH₂)ₘ -alkyl, -(CH₂)ₘO-C(O)-O-alkyl, -(CH₂)ₘO-C(O)-O-cycloalkyl, -N(H)C(Me)C(O)O-alkyl, or alkoxy arylsulfonamide,
   wherein each A³ may be optionally substituted with 1 to 4
   -R¹, -P(O)(OA²)(OA²), -P(O)(OA²)(N(A²)₂), -P(O)(A²)(OA²), -P(O)(A²)(N(A²)₂), or -P(O)(N(A²)₂)(N(A²)₂), halogen, alkyl, alkenyl, alkynyl, aryl, carbocycle, heterocycle, aralkyl, aryl sulfonamide, aryl alkylsulfonamide, aryloxy sulfonamide, aryloxy alkylsulfonamide, aryloxy arylsulfonamide, alkyl sulfonamide, alkyloxy sulfonamide, alkyloxy alkylsulfonamide, -(CH₂)ₘheterocycle, -(CH₂)ₘ-C(O)O-alkyl, -O(CH₂)ₘOC(O)Oalkyl, -O-(CH₂)ₘ-O-C(O) -(CH₂)ₘ-alkyl, - (CH₂)m-O-C(O)-O-alkyl, -(CH₂)ₘ-O-C(O)-O-cycloalkyl, -N(H)C(CH₃)C(O)O-alkyl, alkoxy, -N(R³)(R³) or alkoxy arylsulfonamide, optionally substituted with -R¹, or
A³ forms a carbocyclic or heterocyclic ring with any other A³ or Q¹;
Y¹ is O, S, N(R₂), N(OR₂) or N(N(R₂))₂;
A² is independently selected from H, alkyl, alkenyl, alkynyl, amino, amino acid, alkoxy, aryloxy, cyano, haloalkyl, cycloalkyl, aryl, heteroaryl, alkylsulfonamide, or arylsulfonamide, optionally substituted with A³;
PRT is selected from -CH₂OC(=O)R⁹ and -CH₂OC(=O)OR⁹ where R⁹ is C₁-C₆ alkyl, C₁-C₆ substituted alkyl, C₆-C₂₀ aryl or C₆-C₂₀ substituted aryl
m is to 6;
r is 1 to 2; and
q is 1 to 10.

In an embodiment the invention relates to the accumulation or retention of therapeutic compounds inside cells. The invention is more particularly related to attaining high concentrations of phosphonate molecules in liver cells. Such effective targeting may be applicable to a variety of therapeutic formulations and procedures.

Compositions of the invention include anti-viral compounds having usually at least one phosphonate group. Accordingly, in one embodiment, the invention provides a compound of the invention which is linked to one or more phosphonate groups.

### DEFINITIONS

Unless stated otherwise, the following terms and phrases as used herein are intended to have the following meanings:
When tradenames are used herein, applicants intend to independently include the tradename product and the active pharmaceutical ingredient(s) of the tradename product.

"Bioavailability" is the degree to which the pharmaceutically active agent becomes available to the target tissue after the agent's introduction into the body. Enhancement of the bioavailability of a pharmaceutically active agent can provide a more efficient and effective treatment for patients because, for a given dose, more of the pharmaceutically active agent will be available at the targeted tissue sites.

The terms "phosphonate" and "phosphonate group" include functional groups or moieties within a molecule that comprises a phosphorous that is 1) single-bonded to a carbon, 2) double-bonded to a heteroatom , 3) single-bonded to a heteroatom, and 4) single-bonded to another heteroatom, wherein each heteroatom can be the same or different. The terms "phosphonate" and "phosphonate group" also include functional groups or moieties that comprise a phosphorous in the same oxidation state as the phosphorous described above, as well as functional groups or moieties that comprise a prodrug moiety that can separate from a compound so that the compound retains a phosphorous having the characteriatics described above. For example, the terms "phosphonate" and "phosphonate group" include phosphonic acid, phosphonic monoester, phosphonic diester, phosphonamidate, and phosphonthioate functional groups. In one specific embodiment of the invention, the terms "phosphonate" and "phosphonate group" include functional groups or moieties within a molecule that comprises a phosphorous that is 1) single-bonded to a carbon, 2) double-bonded to an oxygen, 3) single-bonded to an oxygen, and 4) single-bonded to another oxygen, as well as functional groups or moieties that comprise a prodrug moiety that can separate from a compound so that the compound retains a phosphorous having such characteriatics. In another specific embodiment of the invention, the terms "phosphonate" and "phosphonate group" include functional groups or moieties within a molecule that comprises a phosphorous that is 1) single-bonded to a carbon, 2) double-bonded to an oxygen, 3) single-bonded to an oxygen or nitrogen, and 4) single-bonded to another oxygen or nitrogen, as well as functional groups or moieties that comprise a prodrug moiety that can separate from a compound so that the compound retains a phosphorous having such characteriatics.

The term "PRT" is selected from the terms "prodrug moiety" and "protecting group" as defined herein.

The term "prodrug" as used herein refers to any compound that when administered to a biological system generates the drug substance, i.e. active ingredient, as a result of spontaneous chemical reaction(s), enzyme catalyzed chemical reaction(s), photolysis, and/or metabolic chemical reaction(s). A prodrug is thus a covalently modified analog or latent form of a therapeutically-active compound.

"Prodrug moiety" refers to a labile functional group which separates from the active inhibitory compound during metabolism, systemically, inside a cell, by hydrolysis, enzymatic cleavage, or by some other process (Bundgaard, Hans, "Design and Application of Prodrugs" in A Textbook of Drug Design and Development (1991), P. Krogsgaard-Larsen and H. Bundgaard, Eds. Harwood Academic Publishers, pp. 113-191). Enzymes which are capable of an enzymatic activation mechanism with the phosphonate prodrug compounds of the invention include, but are not limited to, amidases, esterases, microbial enzymes, phospholipases, cholinesterases, and phosphases. Prodrug moieties can serve to enhance solubility, absorption and lipophilicity to optimize drug delivery, bioavailability and efficacy. A prodrug moiety may include an active metabolite or drug itself.

Exemplary prodrug moieties include the hydrolytically sensitive or labile acyloxymethyl esters -CH₂OC(=O)R⁹ and acyloxymethyl carbonates -CH₂OC(=O)OR⁹ where R⁹ is C₁-C₆ alkyl, C₁-C₆ substituted alkyl, C₆-C₂₀ aryl or C₆-C₂₀ substituted aryl. The acyloxyalkyl ester was first used as a prodrug strategy for carboxylic acids and then applied to phosphates and phosphonates by Farqullar et al. (1983) J. Pharm. Sci. 72: 324; also US Patent Nos. 4816570, 4968788, 5663159 and 5792756. Subsequently, the acyloxyalkyl ester was used to deliver phosphonic acids across cell membranes and to enhance oral bioavailability. A close variant of the acyloxyalkyl ester, the alkoxycarbonyloxyalkyl ester (carbonate), may also enhance oral bioavailability as a prodrug moiety in the compounds of the combinations of the invention. An exemplary acyloxymethyl ester is pivaloyloxymethoxy, (POM) -CH₂OC(=O)C(CH₃)₃. An exemplary acyloxymethyl carbonate prodrug moiety is pivaloyloxymethylcarbonate (POC) -CH₂OC(=O)OC(CH₃)₃.

The phosphonate group may be a phosphonate prodrug moiety. The prodrug moiety may be sensitive to hydrolysis, such as, but not limited to a pivaloyloxymethyl carbonate (POC) or POM group. Alternatively, the prodrug moiety may be sensitive to enzymatic potentiated cleavage, such as a lactate ester or a phosphonamidate-ester group.

Aryl esters of phosphorus groups, especially phenyl esters, are reported to enhance oral bioavailability (De Lombaert et al. (1994) J. Med. Chem. 37: 498). Phenyl esters containing a carboxylic ester ortho to the phosphate have also been described (Khanmei and Torrence, (1996) J. Med. Chem. 39:4109-4115). Benzyl esters are reported to generate the parent phosphonic acid. In some cases, substituents at the *ortho*-or *para*-position may accelerate the hydrolysis. Benzyl analogs with an acylated phenol or an alkylated phenol may generate the phenolic compound through the action of enzymes, *e.g.,* esterases, oxidases, etc., which in turn undergoes cleavage at the benzylic C-O bond to generate the phosphoric acid and the quinone methide intermediate. Examples of this class of prodrugs are described by Mitchell et al. (1992) J. Chem. Soc. Perkin Trans. II 2345; Glazier WO 91/19721. Still other benzylic prodrugs have been described containing a carboxylic ester-containing group attached to the benzylic methylene (Glazier WO 91/19721). Thio-containing prodrugs are reported to be useful for the intracellular delivery of phosphonate drugs. These proesters contain an ethylthio group in which the thiol group is either esterified with an acyl group or combined with another thiol group to form a disulfide. Deesterification or reduction of the disulfide generates the free thio intermediate which subsequently breaks down to the phosphoric acid and episulfide (Puech et al. (1993) Antiviral Res., 22:155-174; Benzaria et al. (1996) J. Med. Chem. 39: 4958). Cyclic phosphonate esters have also been described as prodrugs of phosphorus-containing compounds (Erion et al., US Patent No. 6312662).

"Protecting group" refers to a moiety of a compound that masks or alters the properties of a functional group or the properties of the compound as a whole. Chemical protecting groups and strategies for protection/deprotection are well known in the art. *See e.g.,* Protective Groups in Organic Chemistry, Theodora W. Greene, John Wiley & Sons, Inc., New York, 1991. Protecting groups are often utilized to mask the reactivity of certain functional groups, to assist in the efficiency of desired chemical reactions, *e.g*., making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

Protected compounds may also exhibit altered, and in some cases, optimized properties *in vitro* and *in vivo,* such as passage through cellular membranes and resistance to enzymatic degradation or sequestration. In this role, protected compounds with intended therapeutic effects may be referred to as prodrugs. Another function of a protecting group is to convert the parental drug into a prodrug, whereby the parental drug is released upon conversion of the prodrug *in vivo.* Because active prodrugs may be absorbed more effectively than the parental drug, prodrugs may possess greater potency *in vivo* than the parental drug. Protecting groups are removed either *in vitro,* in the instance of chemical intermediate, or *in vivo,* in the case of prodrugs. With chemical intermediates, it is not particularly important that the resulting products after deprotection, *e.g*., alcohols, be physiologically acceptable, although in general it is more desirable if the products are pharmacologically innocuous.

Any reference to any of the compounds of the invention also includes a reference to a physiologically acceptable salt thereof. Examples of physiologically acceptable salts of the compounds of the invention include salts derived from an appropriate base, such as an alkali metal (for example, sodium), an alkaline earth (for example, magnesium), ammonium and NX₄⁺ (wherein X is C₁-C₄ alkyl). Physiologically acceptable salts of an hydrogen atom or an amino-group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound of an hydroxy group include the anion of said compound in combination with a suitable cation such as Na⁺ and NX₄⁺ (wherein X is independently selected from H or a C₁-C₄ alkyl group).

For therapeutic use, salts of active ingredients of the compounds of the invention will be physiologically acceptable, *i.e*. they will be salts derived from a physiologically acceptable acid or base. However, salts of acids or bases which are not physiologically acceptable may also find use, for example, in the preparation or purification of a physiologically acceptable compound. All salts, whether or not derived form a physiologically acceptable acid or base, are within the scope of the present invention.

"Alkyl" is C₁-C₁₈ hydrocarbon containing normal, secondary, tertiary or cyclic carbon atoms. Examples are methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl,-C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃),1-hexyl (-CH₂CH₂CH₂CH₂CH₂CH₃), 2-hexyl (-CH(CH3)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃.

"Alkenyl" is C₂-C₁₈ hydrocarbon containing normal, secondary, tertiary or cyclic carbon atoms with at least one site of unsaturation, *i.e*. a carbon-carbon, *sp*² double bond. Examples include, but are not limited to, ethylene or vinyl (-CH=CH₂), allyl (-CH₂CH=CH₂), cyclopentenyl (-C₅H₇), and 5-hexenyl (-CH₂ CH₂CH₂CH₂CH=CH₂).

"Alkynyl" is C₂-C₁₈ hydrocarbon containing normal, secondary, tertiary or cyclic carbon atoms with at least one site of unsaturation, *i.e*. a carbon-carbon, *sp* triple bond. Examples include, but are not limited to, acetylenic (-C≡CH) and propargyl (-CH₂C≡CH),

"Alkylene" refers to a saturated, branched or straight chain or cyclic hydrocarbon radical of 1-18 carbon atoms, and having two monovalent radical centers derived by the removal of two hydrogen atoms from the same or two different carbon atoms of a parent alkane. Typical alkylene radicals include, but are not limited to, methylene (-CH₂-) 1,2-ethyl (-CH₂CH₂-), 1,3-propyl (-CH₂CH₂CH₂-), 1,4-butyl (-CH₂CH₂CH₂CH₂-), and the like.

"Alkenylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to, 1,2-ethylene (-CH=CH-).

"alkynylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkyne. Typical alkynylene radicals include, but are not limited to, acetylene (-C≡C-), propargyl (-CH₂C≡C-), and 4-pentynyl (-CH₂CH₂CH₂C≡CH-),

"Aryl" means a monovalent aromatic hydrocarbon radical of 6-20 carbon atoms derived by the removal of one hydrogen atom from a single carbon atom of a parent aromatic ring system. Typical aryl groups include, but are not limited to, radicals derived from benzene, substituted benzene, naphthalene, anthracene, biphenyl, and the like.

"Arylalkyl" refers to an acyclic alkyl radical in which one of the hydrogen atoms bonded to a carbon atom, typically a terminal or sp³ carbon atom, is replaced with an aryl radical. Typical arylalkyl groups include, but are not limited to, benzyl, 2-phenylethan-1-yl,, naphthylmethyl, 2-naphthylethan-1-yl, naphthobenzyl, 2-naphthophenylethan-1-yl and the like. The arylalkyl group comprises 6 to 20 carbon atoms, *e.g*., the alkyl moiety, including alkanyl, alkenyl or alkynyl groups, of the arylalkyl group is 1 to 6 carbon atoms and the aryl moiety is 5 to 14 carbon atoms.

"Substituted alkyl", "substituted aryl", and "substituted arylalkyl" mean alkyl, aryl, and arylalkyl respectively, in which one or more hydrogen atoms are each independently replaced with a non-hydrogen substituent. Typical substituents include, but are not limited to, -X, -R, -O-, -OR, -SR, -S-, -NR₂, -NR₃, =NR, -CX₃, -CN, -OCN, -SCN, -N=C=O, -NCS, -NO, -NO₂, =N₂, -N₃, NC(=O)R, -C(=O)R, -C(=O)NRR -S(=O)₂O⁻, -S(=O)₂OH, -S(=O)₂R, -OS(=O)₂OR, -S(=O)₂NR, -S(=O)R, -OP(=O)O₂RR,-P(=O)O₂RR -P(=O)(O⁻)₂, -P(=O)(OH)₂, -C(=O)R, -C(=O)X, -C(S)R, -C(O)OR, -C(O)O-, -C(S)OR, -C(O)SR, -C(S)SR, -C(O)NRR, -C(S)NRR, -C(NR)NRR, where each X is independently a halogen: F, Cl, Br, or I; and each R is independently -H, alkyl, aryl, heterocycle, protecting group or prodrug moiety. Alkylene, alkenylene, and alkynylene groups may also be similarly substituted.

"Heterocycle" as used herein includes by way of example and not limitation these heterocycles described in Paquette, Leo A.; Principles of Modern Heterocyclic Chemistry (W.A. Benjamin, New York, 1968), particularly Chapters 1, 3, 4, 6, 7, and 9; The Chemistry of Heterocyclic Compounds, A Series of Monographs" (John Wiley & Sons, New York, 1950 to present), in particular Volumes 13, 14, 16, 19, and 28; and J. Am. Chem. Soc. (1960) 82:5566. In one specific embodiment of the invention "heterocycle" includes a "carbocycle" as defined herein, wherein one or more (*e.g*. 1, 2, 3, or 4) carbon atoms have been replaced with a heteroatom (*e.g*. O, N, or S).

Examples of heterocycles include by way of example and not limitation pyridyl, dihydroypyridyl, tetrahydropyridyl (piperidyl), thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thienyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, isatinoyl, and bis-tetrahydrofuranyl:

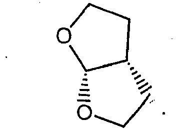

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline. Still more typically, nitrogen bonded heterocycles include 1-aziridyl, 1-azetedyl, 1-pyrrolyl, 1-imidazolyl, 1-pyrazolyl, and 1-piperidinyl.

"Carbocycle" refers to a saturated, unsaturated or aromatic ring having 3 to 7 carbon atoms as a monocycle, 7 to 12 carbon atoms as a bicycle, and up to about 20 carbon atoms as a polycycle. Monocyclic carbocycles have 3 to 6 ring atoms, still more typically 5 or 6 ring atoms. Bicyclic carbocycles have 7 to 12 ring atoms, e.g., arranged as a bicyclo [4,5], [5,5], [5,6] or [6,6] system, or 9 or 10 ring atoms arranged as a bicyclo [5,6] or [6,6] system. Examples of monocyclic carbocycles include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, phenyl, spiryl and naphthyl.

"linked" or "link" refers to a chemical moiety comprising a covalent bond or a chain or group of atoms that covalently attaches a phosphonate group to a drug. Linkers include portions of substituents A¹ and A³, which include moieties such as: repeating units of alkyloxy (*e.g*., polyethylenoxy, PEG, polymethyleneoxy) and alkylamino (*e.g*., polyethyleneamino, Jeffamine™); and diacid ester and amides including succinate, succinamide, diglycolate, malonate, and caproamide.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, *e.g*., melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

The term "treatment" or "treating," to the extent it relates to a disease or condition includes preventing the disease or condition from occurring, inhibiting the disease or condition, eliminating the disease or condition; and/or relieving one or more symptoms of the disease or condition.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, *i*.*e*., they have the ability to rotate the plane of plane-polarized light. In describing an optically active compound, the prefixes D and L or R and S are used to denote the absolute configuration of the molecule about its chiral center(s). The prefixes d and 1 or + and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

### Protecting Groups

In the context of the present invention, protecting groups include prodrug moieties and chemical protecting groups.

Protecting groups are available, commonly known and used, and are optionally used to prevent side reactions with the protected group during synthetic procedures, *i.e.* routes or methods to prepare the compounds of the invention. For the most part the decision as to which groups to protect, when to do so, and the nature of the chemical protecting group "PG" will be dependent upon the chemistry of the reaction to be protected against (*e.g*., acidic, basic, oxidative, reductive or other conditions) and the intended direction of the synthesis. The PG groups do not need to be, and generally are not, the same if the compound is substituted with multiple PG. In general, PG will be used to protect functional groups such as carboxyl, hydroxyl, thio, or amino groups and to thus prevent side reactions or to otherwise facilitate the synthetic efficiency. The order of deprotection to yield free, deprotected groups is dependent upon the intended direction of the synthesis and the reaction conditions to be encountered, and may occur in any order as determined by the artisan.

Various functional groups of the compounds of the invention may be protected. For example, protecting groups for -OH groups (whether hydroxyl, carboxylic acid, phosphonic acid, or other functions) include "ether- or ester-forming groups". Ether- or ester-forming groups are capable of functioning as chemical protecting groups in the synthetic schemes set forth herein. However, some hydroxyl and thio protecting groups are neither ether- nor ester-forming groups, as will be understood by those skilled in the art, and are included with amides, discussed below.

A very large number of hydroxyl protecting groups and amide-forming groups and corresponding chemical cleavage reactions are described in Protective Groups in Organic Synthesis, Theodora W. Greene (John Wiley & Sons, Inc., New York, 1991, ISBN 0-471-62301-6) ("Greene"). See also Kocienski, Philip J.; Protecting Groups (Georg Thieme Verlag Stuttgart, New York, 1994). In particular Chapter 1, Protecting Groups: An Overview, pages 1-20, Chapter 2, Hydroxyl Protecting Groups, pages 21-94, Chapter 3, Diol Protecting Groups, pages 95-117, Chapter 4, Carboxyl Protecting Groups, pages 118-154, Chapter 5, Carbonyl Protecting Groups, pages 155-184. For protecting groups for carboxylic acid, phosphonic acid, phosphonate, sulfonic acid and other protecting groups for acids see Greene as set forth below. Such groups include by way of example and not limitation, esters, amides, hydrazides, and the like.

### Ether- and Ester-forming protecting groups

Ester-forming groups include: (1) phosphonate ester-forming groups, such as phosphonamidate esters, phosphorothioate esters, phosphonate esters, and phosphon-bis-amidates; (2) carboxyl ester-forming groups, and (3) sulphur ester-forming groups, such as sulphonate, sulfate, and sulfinate.

The phosphonate moieties of the compounds of the invention may or may not be prodrug moieties, *i.e*. they may or may be susceptible to hydrolytic or enzymatic cleavage or modification. Certain phosphonate moieties are stable under most or nearly all metabolic conditions. For example, a dialkylphosphonate, where the alkyl groups are two or more carbons, may have appreciable stability *in vivo* due to a slow rate of hydrolysis.

Within the context of phosphonate prodrug moieties, a large number of structurally-diverse prodrugs have been described for phosphonic acids (Freeman and Ross in Progress in Medicinal Chemistry 34: 112-147 (1997). An exemplary phosphonate ester-forming group is the phenyl carbocycle in substructure A₃ having the formula: 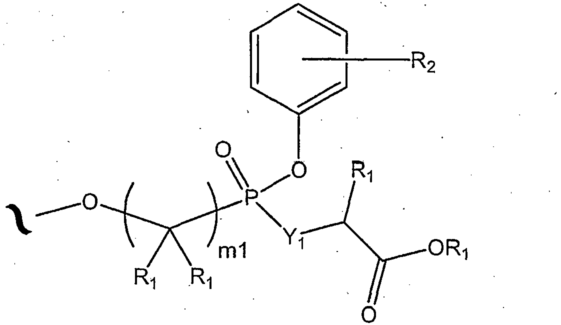 wherein R₁ may be H or C₁-C₁₂ alkyl; m1 is 1, 2, 3, 4, 5, 6, 7 or 8, and the phenyl carbocycle is substituted with 0 to 3 R₂ groups. Where Y₁ is O, a lactate ester is formed, and where Y₁ is N(R₂), N(OR₂) or N(N(R₂)₂, a phosphonamidate ester results.

In its ester-forming role, a protecting group typically is bound to any acidic group such as, by way of example and not limitation, a -CO₂H or -C(S)OH group, thereby resulting in -CO₂R^{x} where R^{x} is defined herein. Also, R^{x} for example includes the enumerated ester groups of WO 95/07920.

Examples of protecting groups include:
C₃-C₁₂ heterocycle (described above) or aryl. These aromatic groups optionally are polycyclic or monocyclic. Examples include phenyl, spiryl, 2- and 3-pyrrolyl, 2- and 3-thienyl, 2- and 4-imidazolyl, 2-, 4- and 5-oxazolyl, 3- and 4-isoxazolyl, 2-, 4- and 5-thiazolyl, 3-, 4- and 5-isothiazolyl, 3- and 4-pyrazolyl, 1-, 2-, 3-and 4-pyridinyl, and 1-, 2-, 4- and 5-pyrimidinyl,
C₃-C₁₂ heterocycle or aryl substituted with halo, R¹, R¹-O-C₁-C₁₂ alkylene, C₁-C₁₂ alkoxy, CN, NO₂, OH, carboxy, carboxyester, thiol, thioester, C₁-C₁₂ haloalkyl (1-6 halogen atoms), C₂-C₁₂ alkenyl or C₂-C₁₂ alkynyl. Such groups include 2-, 3- and 4-alkoxyphenyl (C₁-C₁₂ alkyl), 2-, 3- and 4-methoxyphenyl, 2-, 3-and 4-ethoxyphenyl, 2,3-, 2,4-, 2,5-, 2,6-, 3,4- and 3,5-diethoxyphenyl, 2- and 3-carboethoxy-4-hydroxyphenyl, 2- and 3-ethoxy-4-hydroxyphenyl, 2- and 3-ethoxy-5-hydroxyphenyl, 2- and 3-ethoxy-6-hydroxyphenyl, 2-, 3- and 4-O-acetylphenyl, 2-, 3-and 4-dimethylaminophenyl, 2-, 3- and 4-methylmercaptophenyl, 2-, 3- and 4-halophenyl (including 2-, 3- and 4-fluorophenyl and 2-, 3- and 4-chlorophenyl), 2,3-, 2,4-, 2,5-, 2,6-, 3,4- and 3,5-dimethylphenyl, 2,3-, 2,4-, 2,5-, 2,6-, 3,4- and 3,5-biscarboxyethylphenyl, 2,3-, 2,4-, 2,5-, 2,6-, 3,4- and 3,5-dimethoxyphenyl, 2,3-, 2,4-, 2,5-, 2,6-, 3,4- and 3,5-dihalophenyl (including 2,4-difluorophenyl and 3,5-difluorophenyl), 2-, 3- and 4-haloalkylphenyl (1 to 5 halogen atoms, C₁-C₁₂ alkyl including 4-trifluoromethylphenyl), 2-, 3- and 4-cyanophenyl, 2-, 3- and 4-nitrophenyl, 2-, 3- and 4-haloalkylbenzyl (1 to 5 halogen atoms, C₁-C₁₂ alkyl including 4-trifluoromethylbenzyl and 2-, 3- and 4-trichloromethylphenyl and 2-, 3-and 4-trichloromethylphenyl), 4-N-methylpiperidinyl, 3-N-methylpiperidinyl, 1-ethylpiperazinyl, benzyl, alkylsalicylphenyl (C₁-C₄ alkyl, including 2-, 3- and 4-ethylsalicylphenyl), 2-,3- and 4-acetylphenyl, 2,8-dihydroxynaphthyl (-C₁₀H₆-OH) and aryloxy ethyl [C₆-C₉ aryl (including phenoxy ethyl)], 2,2'-dihydroxybiphenyl, 2-, 3- and 4-N,N-dialkylaminophenol, -C₆H₄CH₂-N(CH₃)₂, trimethoxybenzyl, triethoxybenzyl, 2-alkyl pyridinyl (C₁₋₄ alkyl); 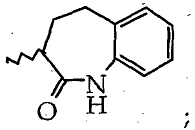 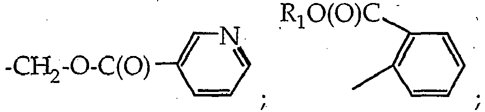 C₄ - C₈ esters of 2-carboxyphenyl; and C₁-C₄ alkylene-C₃-C₆ aryl (including benzyl, -CH₂-pyrrolyl, -CH₂-thienyl, -CH₂-imidazolyl, -CH₂-oxazolyl, -CH₂-isoxazolyl, -CH₂-thiazolyl, -CH₂-isothiazolyl, -CH₂-pyrazolyl, -CH₂-pyridinyl and -CH₂-pyrimidinyl) substituted in the aryl moiety by 3 to 5 halogen atoms or 1 to 2 atoms or groups selected from halogen, C₁-C₁₂ alkoxy (including methoxy and ethoxy), cyano, nitro, OH, C₁-C₁₂ haloalkyl (1 to 6 halogen atoms; including -CH₂CCl₃), C₁-C₁₂ alkyl (including methyl and ethyl), C₂-C₁₂ alkenyl or C₂-C₁₂ alkynyl; alkoxy ethyl [C₁-C₆ alkyl including -CH₂-CH₂-O-CH₃ (methoxy ethyl)]; alkyl substituted by any of the groups set forth above for aryl, in particular OH or by 1 to 3 halo atoms (including -CH₃, -CH(CH₃)₂, -C(CH₃)₃, - CH₂CH₃, -(CH₂)₂CH₃, -(CH₂)₃CH₃, -(CH₂)₄CH₃, -(CH₂)₅CH₃, -CH₂CH₂F, - CH₂CH₂Cl, -CH₂CF₃, and -CH₂CCl₃); 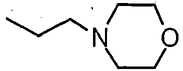 -N-2-propylmoipholino, 2,3-dihydro-6-hydroxyindene, sesamol, catechol monoester, -CH₂-C(O)-N(R¹)₂, - CH₂-S(O)(R¹), -CH₂-S(O)₂(R¹), -CH₂-CH(OC(O)CH₂R¹)-CH₂(OC(O)CH₂R¹), cholesteryl, enolpyruvate (HOOC-C(=CH₂)-), glycerol;
a 5 or 6 carbon monosaccharide, disaccharide or oligosaccharide (3 to 9 monosaccharide residues);
triglycerides such as α-D-β-diglycerides (wherein the fatty acids composing glyceride lipids generally are naturally occurring saturated or unsaturated C₆₋₂₆, C₆₁₈ or C₆₋₁₀ fatty acids such as linoleic, lauric, myristic, palmitic, stearic, oleic, palmitoleic, linolenic and the like fatty acids) linked to acyl of the parental compounds herein through a glyceryl oxygen of the triglyceride;
   phospholipids linked to the carboxyl group through the phosphate of the phospholipid;
   phthalidyl (shown in Fig. 1 of Clayton et al., Antimicrob. Agents Chemo. (1974) 5(6):670-671);
   cyclic carbonates such as (5-R_{d}-2-oxo-1,3-dioxolen-4-yl) methyl esters (Sakamoto et al., Chem. Pharm. Bull. (1984) 32(6)2241-2248) where R_{d} is R₁, R₄ or aryl; and

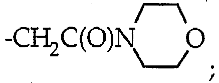

The hydroxyl groups of the compounds of this invention optionally are substituted with one of groups III, IV or V disclosed in WO 94/21604, or with isopropyl.

Table A lists examples of protecting group ester moieties that for example can be bonded via oxygen to -C(O)O- and -P(O)(O-)₂ groups. Several amidates also are shown, which are bound directly to -C(O)- or -P(O)₂. Esters of structures 1-5, 8-10 and 16, 17, 19-22 are synthesized by reacting the compound herein having a free hydroxyl with the corresponding halide (chloride or acyl chloride and the like) and N ,N-dicyclohexyl-N-morpholine carboxamidine (or another base such as DBU, triethylamine, CsCO₃, N,N-dimethylaniline and the like) in DMF (or other solvent such as acetonitrile or N-methylpyrrolidone). When the compound to be protected is a phosphonate, the esters of structures 5-7, 11, 12, 21, and 23-26 are synthesized by reaction of the alcohol or alkoxide salt (or the corresponding amines in the case of compounds such as 13, 14 and 15) with the monochlorophosphonate or dichlorophosphonate (or another activated phosphonate).

**TABLE A**

| | | | | | |
|---|---|---|---|---|---|
| 1. | -CH₂-C(O)-N(R₁)₂ * | | 10. | -CH₂-O-C(O)-C(CH₃)₃ | |
| 2. | -CH₂-S(O)(R₁) | | 11. | -CH₂-CCl₃ | |
| 3. | -CH₂-S(O)₂(R₁) | | 12. | -C₆H₅ | |
| 4. | -CH₂-O-C(O)-CH₂-C₆H₅ | | 13. | -NH-CH₂-C(O)O-CH₂CH₃ | |
| 5. | 3-cholesteryl | | 14. | -N(CH₃)-CH₂-C(O)O-CH₂CH₃ | |
| 6. | 3-pyridyl | | 15. | -NHR₁ | |
| 7. | N-ethylmorpholino | | 16. | -CH₂-O-C(O)-C₁₀H₁₅ | |
| 8. | -CH₂-O-C(O)-C₆H₅ | | 17. | -CH₂-O-C(O)-CH(CH₃)₂ | |
| 9. | -CH₂-O-C(O)-CH₂CH₃ | | 18. | -CH₂-C#H(OC(O)CH₂R₁)-CH₂--(OC(O)CH₂R₁)* | |
| 19. | 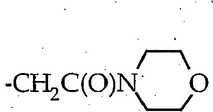 | 20. | 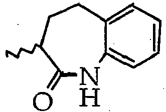 | 21. | 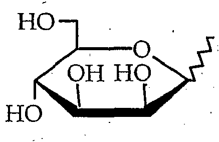 |
| 22. | 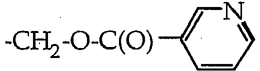 | 23. | 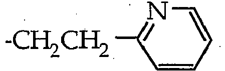 | | |
| 24. | 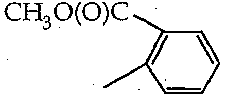 | | | | |
| 25. | 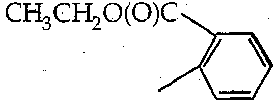 | 26. | 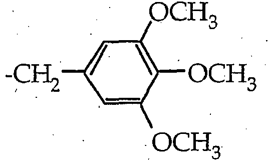 | | |

| | | | | | |
|---|---|---|---|---|---|
| # - chiral center is (R), (S) or racemate. | | | | | |

Other esters that are suitable for use herein are described in EP 632048.

Protecting groups also includes "double ester" forming profunctionalities such as -CH₂OC(O)OCH₃, 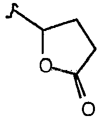 -CH₂SCOCH₃, -CH₂OCON(CH₃)₂, or alkyl-or aryl-acyloxyalkyl groups of the structure -CH(R¹ or W⁵)O((CO)R³⁷) or -CH(R¹ or W⁵)((CO)OR³⁸) (linked to oxygen of the acidic group) wherein R³⁷ and R³⁸ are alkyl, aryl, or alkylaryl groups (see U.S. Patent No. 4968788). Frequently R³⁷ and R³⁸ are bulky groups such as branched alkyl, ortho-substituted aryl, meta-substituted aryl, or combinations thereof, including normal, secondary, iso- and tertiary alkyls of 1-6 carbon atoms. An example is the pivaloyloxymethyl group. These are of particular use with prodrugs for oral administration. Examples of such useful protecting groups are alkylacyloxymethyl esters and their derivatives, including - CH(CH₂CH₂OCH₃)OC(O)C(CH₃)₃, 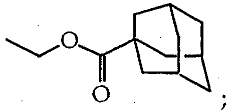 -CH₂OC(O)C₁₀H₁₅, - CH₂OC(O)C(CH₃)₃, -CH(CH₂OCH₃)OC(O)C(CH₃)₃, - CH(CH(CH₃)₂)OC(O)C(CH₃)₃, -CH₂OC(O)CH₂CH(CH₃)₂, -CH₂OC(O)C₆H₁₁, - CH₂OC(O)C₆H₅, -CH₂OC(O)C₁₀H₁₅, -CH₂OC(O)CH₂CH₃, -CH₂OC(O)CH(CH₃)₂, - CH₂OC(O)C(CH₃)₃ and -CH₂OC(O)CH₂C₆H₅.

In some embodiments the protected acidic group is an ester of the acidic group,and is the residue of a hydroxyl-containing functionality. In other embodiments, an amino compound is used to protect the acid functionality. The residues of suitable hydroxyl or amino-containing functionalities are set forth above or are found in WO 95/07920. Of particular interest are the residues of amino acids, amino acid esters, polypeptides, or aryl alcohols. Typical amino acid, polypeptide and carboxyl-esterified amino acid residues are described on pages 11-18 and related text of WO 95/07920 as groups L1 or L2. WO 95/07920 expressly teaches the amidates of phosphonic acids, but it will be understood that such amidates are formed with any of the acid groups set forth herein and the amino acid residues set forth in WO 95/07920.

Typical esters for protecting acidic functionalities are also described in WO 95/07920, again understanding that the same esters can be formed with the acidic groups herein as with the phosphonate of the '920 publication. Typical ester groups are defined at least on WO 95/07920 pages 89-93 (under R³¹ or R³⁵), the table on page 105, and pages 21-23 (as R). Of particular interest are esters of unsubstituted aryl such as phenyl or arylalkyl such benzyl, or hydroxy-, halo-, alkoxy,-, carboxy- and/or alkylestercarboxy-substituted aryl or alkylaryl, especially phenyl, ortho-ethoxyphenyl, or C₁-C₄ alkylestercarboxyphenyl (salicylate C₁-C₁₂ alkylesters).

The protected acidic groups, particularly when using the esters or amides of WO 95/07920, are useful as prodrugs for oral administration. However, it is not essential that the acidic group be protected in order for the compounds of this invention to be effectively administered by the oral route. When the compounds of the invention having protected groups, in particular amino acid amidates or substituted and unsubstituted aryl esters are administered systemically or orally they are capable of hydrolytic cleavage *in vivo* to yield the free acid.

One or more of the acidic hydroxyls are protected. If more than one acidic hydroxy is protected then the same or a different protecting group is employed, e.g., the esters may be different or the same, or a mixed amidate and ester may be used.

Typical hydroxy protecting groups described in Greene (pages 14-118) include substituted methyl and alkyl ethers, substituted benzyl ethers, silyl ethers, esters including sulfonic acid esters, and carbonates. For example:
- Ethers (methyl, *t*-butyl, allyl);
- Substituted Methyl Ethers (Methoxymethyl, Methylthiomethyl, *t-*Butylthiomethyl, (Phenyldimethylsilyl)methoxymethyl, Benzyloxymethyl, *p-*Methoxybenzyloxymethyl, (4-Methoxyphenoxy)methyl, Guaiacolmethyl, *t-*Butoxymethyl, 4-Pentenyloxymethyl, Siloxymethyl, 2-Methoxyethoxymethyl, 2,2,2-Trichloroethoxymethyl, Bis(2-chloroethoxy)methyl, 2-(Trimethylsilyl)ethoxymethyl, Tetrahydropyranyl, 3-Bromotetrahydropyranyl, Tetrahydropthiopyranyl, 1-Methoxycyclohexyl, 4-Methoxytetrahydropyranyl, 4-Methoxytetrahydrothiopyranyl, 4-Methoxytetrahydropthiopyranyl S,S-Dioxido, 1-[(2-Chloro-4-methyl)phenyl]-4-methoxypiperidin-4-yl, 1,4-Dioxan-2-yl, Tetrahydrofuranyl, Tetrahydrothiofuranyl, 2,3,3a,4,5,6,7,7a-Octahydro-7,8,8-trimethyl-4,7-methanobenzofuran-2-yl));

- Substituted Ethyl Ethers (1-Ethoxyethyl, 1-(2-Chloroethoxy)ethyl, 1-Methyl-1-methoxyethyl, 1-Methyl-1-benzyloxyethyl,1-Methyl-1-benzyloxy-2-fluoroethyl, 2,2,2-Trichloroethyl, 2-Trimethylsilylethyl, 2-(Phenylselenyl)ethyl,
- *p*-Chlorophenyl, *p*-Methoxyphenyl, 2,4-Dinitrophenyl, Benzyl);
- Substituted Benzyl Ethers (*p*-Methoxybenzyl, 3,4-Dimethoxybenzyl, *o-*Nitrobenzyl, *p*-Nitrobenzyl, *p*-Halobenzyl, 2,6-Dichlorobenzyl, *p*-Cyanobenzyl, *p-*Phenylbenzyl, 2- and 4-Picolyl, 3-Methyl-2-picolyl *N*-Oxido, Diphenylmethyl, *p*,*p*'-Dinitrobenzhydryl, 5-Dibenzosuberyl, Triphenylmethyl, α-Naphthyldiphenylmethyl, *p*-methoxyphenyldiphenylmethyl, Di(*p-*methoxyphenyl)phenylmethyl, Tri(*p*-methoxyphenyl)methyl, 4-(4'-Bromophenacyloxy)phenyldiphenylmethyl, 4,4',4"-Tris(4,5-dichlorophthalimidophenyl)methyl, 4,4',4"-Tris(levulinoyloxyphenyl)methyl, 4,4',4"-Tris(benzoyloxyphenyl)methyl, 3-(Imidazol-1-ylmethyl)bis(4',4"-dimethoxyphenyl)methyl, 1,1-Bis(4-methoxyphenyl)-1'-pyrenylmethyl, 9-Anthryl, 9-(9-Phenyl)xanthenyl, 9-(9-Phenyl-10-oxo)anthryl, 1,3-Benzodithiolan-2-yl, Benzisothiazolyl *S*,*S*-Dioxido);
- Silyl Ethers (Trimethylsilyl, Triethylsilyl, Triisopropylsilyl, Dimethylisopropylsilyl, Diethylisopropylsilyl, Dimethylthexylsilyl, *t-*Butyldimethylsilyl, *t*-Butyldiphenylsilyl, Tribenzylsilyl, Tri-*p*-xylylsilyl, Triphenylsilyl, Diphenylmethylsilyl, *t*-Butylmethoxyphenylsilyl);
- Esters (Formate, Benzoylformate, Acetate, Choroacetate, Dichloroacetate, Trichloroacetate, Trifluoroacetate; Methoxyacetate, Triphenylmethoxyacetate, Phenoxyacetate, *p*-Chlorophenoxyacetate, *p*-poly-Phenylacetate, 3-Phenylpropionate, 4-Oxopentanoate (Levulinate), 4,4-(Ethylenedithio)pentanoate, Pivaloate, Adamantoate, Crotonate, 4-Methoxycrotonate, Benzoate, *p*-Phenylbenzoate, 2,4,6-Trimethylbenzoate (Mesitoate));
- Carbonates (Methyl, 9-Fluorenylmethyl, Ethyl, 2,2,2-Trichloroethyl, 2-(Trimethylsilyl)ethyl, 2-(Phenylsulfonyl)ethyl, 2-(Triphenylphosphonio)ethyl, Isobutyl, Vinyl, Allyl, *p*-Nitrophenyl, Benzyl, *p*-Methoxybenzyl, 3,4-Dimethoxybenzyl, *o*-Nitrobenzyl, *p*-Nitrobenzyl, *S*-Benzyl Thiocarbonate, 4-Ethoxy-1-naphthyl, Methyl Dithiocarbonate);
- Groups With Assisted Cleavage (2-Iodobenzoate, 4-Azidobutyrate, 4-Nitro-4-methylpentanoate, o-(Dibromomethyl)benzoate, 2-Formylbenzenesulfonate, 2-(Methylthiomethoxy)ethyl Carbonate, 4-(Methylthiomethoxy)butyrate, 2-(Methylthiomethoxymethyl)benzoate); Miscellaneous Esters (2,6-Dichloro-4-methylphenoxyacetate, 2,6-Dichloro-4-(1,1,3,3 tetramethylbutyl)phenoxyacetate, 2,4-Bis(1,1-dimethylpropyl)phenoxyacetate, Chlorodiphenylacetate, Isobutyrate, Monosuccinate, (*E*)-2-Methyl-2-butenoate (Tigloate), *o-*o (Methoxycarbonyl)benzoate, *p*-poly-Benzoate, α-Naphthoate, Nitrate, Alkyl *N*,*N*,*N'*,*N'*-Tetramethylphosphorodiamidate, *N*-Phenylcarbamate, Borate, Dimethylphosphinothioyl, 2,4-Dinitrophenylsulfenate); and
- Sulfonates (Sulfate, Methanesulfonate (Mesylate), Benzylsulfonate, Tosylate).

Typical 1,2-diol protecting groups (thus, generally where two OH groups are taken together with the protecting functionality) are described in Greene at pages 118-142 and include Cyclic Acetals and Ketals (Methylene, Ethylidene, 1-*t-*Butylethylidene,1-Phenylethylidene, (4-Methoxyphenyl)ethylidene, 2,2,2-Trichloroethylidene, Acetonide (Isopropylidene), Cyclopentylidene, Cyclohexylidene, Cycloheptylidene, Benzylidene, *p*-Methoxybenzylidene, 2,4-Dimethoxybenzylidene, 3,4-Dimethoxybenzylidene, 2-Nitrobenzylidene); Cyclic Ortho Esters (Methoxymethylene, Ethoxymethylene, Dimethoxymethylene, 1-Methoxyethylidene, 1-Ethoxyethylidine, 1,2-Dimethoxyethylidene, α-Methoxybenzylidene, 1-(*N*,*N*-Dimethylamino)ethylidene Derivative, α -(*N*,*N-*Dimethylamino)benzylidene Derivative, 2-Oxacyclopentylidene); Silyl Derivatives (Di-*t*-butylsilylene Group, 1,3-(1,1,3,3-Tetraisopropyldisiloxanylidene), and Tetra-*t-*butoxydisiloxane-1,3-diylidene), Cyclic Carbonates, Cyclic Boronates, Ethyl Boronate and Phenyl Boronate.

More typically, 1,2-diol protecting groups include those shown in Table B, still more typically, epoxides, acetonides, cyclic ketals and aryl acetals.

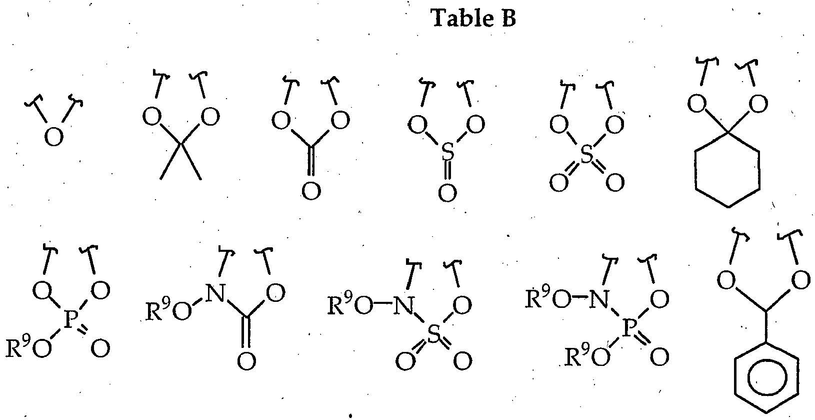

wherein R9 is C₁-C₆ alkyl.

### Amino protecting groups

Another set of protecting groups include any of the typical amino protecting groups described by Greene at pages 315-385. They include:
- Carbamates: (methyl and ethyl, 9-fluorenylmethyl, 9(2-sulfo)fluorenylmethyl, 9-(2,7-dibromo)fluorenylmethyl, 2,7-di-*t*-butyl-[9-(10,10-dioxo-10,10,10,10-tetrahydrothioxanthyl)]methyl, 4-methoxyphenacyl);
- Substituted Ethyl: (2,2,2-trichoroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, *2-(N,N-*dicyclohexylcarboxamido)ethyl, *t*-butyl, 1-adamantyl, vinyl, allyl, 1-isopropylallyl, cinnamyl, 4-nitrocinnamyl, 8-quinolyl, *N*-hydroxypiperidinyl, alkyldithio, benzyl, *p*-methoxybenzyl, p-nitrobenzyl, *p*-bromopenzyl, *p-*chlorobenzyl, 2,4-dichlorobenzyl, 4-methylsulfinylbenzyl, 9-anthrylmethyl, diphenylmethyl);
- Groups With Assisted Cleavage: (2-methylthioethyl, 2-methylsulfonylethyl, 2-(*p-*toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, *m*-chloro-*p*-acyloxybenzyl, *p*-(dihydroxyboryl)benzyl, 5-benzisoxazolylmethyl, 2-(trifluoromethyl)-6-chromonylmethyl);
- Groups Capable of Photolytic Cleavage: (*m*-nitrophenyl, 3,5-dimethoxybenzyl, *o-*nitrobenzyl, 3,4-dimethoxy-6-nitrobenzyl, phenyl(*o*-nitrophenyl)methyl); Urea-Type Derivatives (phenothiazinyl-(10)-carbonyl, N'-*p-*toluenesulfonylaminocarbonyl, *N'*-phenylaminothiocarbonyl);
- Miscellaneous Carbamates: (*t*-amyl, *S*-benzyl thiocarbamate, *p*-cyanobenzyl, cyclobutyl, cyclohexyl, cyclopentyl, cyclopropylmethyl, *p*-decyloxybenzyl, diisopropylmethyl, 2,2-dimethoxycarbonylvinyl, *o*-(*N*,*N-*dimethylcarboxamido)benzyl, 1,1-dimethyl-3-(*N*,*N-*dimethylcarboxamido)propyl, 1,1-dimethylpropynyl, di(2-pyridyl)methyl, 2-furanylmethyl, 2-Iodoethyl, Isobornyl, Isobutyl, Isonicotinyl, *p*-(*p'-*Methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p-*phenylazophenyl)ethyl, 1-methyl-1-phenylethyl, 1-methyl-1-(4-pyridyl)ethyl, phenyl, *p*-(phenylazo)benzyl, 2,4,6-tri-*t*-butylphenyl, 4-(trimethylammonium)benzyl, 2,4,6-trimethylbenzyl);
- Amides: (*N*-formyl, *N*-acetyl, *N*-choroacetyl, *N*-trichoroacetyl, *N*-trifluoroacetyl, *N*-phenylacetyl, *N*-3-phenylpropionyl, *N*-picolinoyl, *N*-3-pyridylcarboxamide, *N-*benzoylphenylalanyl, *N*-benzoyl, *N*-*p*-phenylbenzoyl);
- Amides With Assisted Cleavage: (*N*-*o*-nitrophenylacetyl, *N*-*o*-nitrophenoxyacetyl, *N*-acetoacetyl, (*N'*-dithiobenzyloxycarbonylamino)acetyl, *N*-3-(*p-*hydroxyphenyl)propionyl, *N*-3-(*o*-nitrophenyl)propionyl, *N*-2-methyl-2-(*o-*nitrophenoxy)propionyl, *N*-2-methyl-2-(*o*-phenylazophenoxy)propionyl, *N*-4-chlorobutyryl, *N*-3-methyl-3-nitrobutyryl, *N*-o-nitrocinnamoyl, *N-*acetylmethionine, *N*-*o*-nitrobenzoyl, *N*-*o*-(benzoyloxymethyl)benzoyl, 4,5-diphenyl-3-oxazolin-2-one);
- Cyclic Imide Derivatives: (*N*-phthalimide, *N*-dithiasuccinoyl, *N*-2,3-diphenylmaleoyl, *N*-2,5-dimethylpyrrolyl, *N*-1,1,4,4-tetramethyldisilylazacyclopentane adduct, 5-substituted 1,3-dimethyl-1,3,5-triazacyclohexan-2-one, 5-substituted 1,3-dibenzyl-1,3-5-triazacyclohexan-2-one, 1-substituted 3,5-dinitro-4-pyridonyl);
- *N*-Alkyl and *N*-Aryl Amines: (*N*-methyl, *N*-allyl, *N*-[2-(trimethylsilyl)ethoxy]methyl, *N*-3-acetoxypropyl, *N*-(1-isopropyl-4-nitro-2-oxo-3-pyrrolin-3-yl), Quaternary Ammonium Salts, *N*-benzyl, *N*-di(4-methoxyphenyl)methyl, *N*-5-dibenzosuberyl, *N*-triphenylmethyl, *N*-(4-methoxyphenyl)diphenylmethyl, *N*-9-phenylfluorenyl, *N*-2,7-dichloro-9-fluorenylmethylene, *N*-ferrocenylmethyl, *N*-2-picolylamine *N*'-oxide);
- Imine Derivatives: (*N*-1,1-dimethylthiomethylene, *N*-benzylidene, *N*-*p-*methoxybenylidene, *N*-diphenylmethylene, *N*-[(2-pyridyl)mesityl]methylene, *N*,(*N'*,*N'*-dimethylaminomethylene, *N*,*N*'-isopropylidene, *N*-*p*-nitrobenzylidene, *N*-salicylidene, *N*-5-chlorosalicylidene, *N*-(5-chloro-2-hydroxyphenyl)phenylmethylene, *N*-cyclohexylidene);
- Enamine Derivatives: (*N*-(5,5-dimethyl-3-oxo-1-cyclohexenyl));
- *N*-Metal Derivatives (*N*-borane derivatives, *N*-diphenylborinic acid derivatives, *N*-[phenyl(pentacarbonylchromium- or -tungsten)]carbenyl, *N*-copper or *N*-zinc chelate);
- N-N Derivatives: (*N*-nitro, *N*-nitroso, *N*-oxide);
- N-P Derivatives: (*N*-diphenylphosphinyl, *N*-dimethylthiophosphinyl, *N-*diphenylthiophosphinyl, *N*-dialkyl phosphoryl, *N*-dibenzyl phosphoryl, *N-*diphenyl phosphoryl);
- N-Si Derivatives, N-S Derivatives, and N-Sulfenyl Derivatives: (*N-*benzenesulfenyl, *N*-*o*-nitrobenzenesulfenyl, *N*-2,4-dinitrobenzenesulfenyl, *N-*pentachlorobenzenesulfenyl, *N*-2-nitro-4-methoxybenzenesulfenyl, *N-*triphenylmethylsulfenyl, *N*-3-nitropyridinesulfenyl); and *N*-sulfonyl Derivatives (*N*-*p*-toluenesulfonyl, *N*-benzenesulfonyl, *N*-2,3,6-trimethyl-4-methoxybenzenesulfonyl, *N*-2,4,6-trimethoxybenzenesulfonyl, *N*-2,6-dimethyl-4-methoxybenzenesulfonyl, *N*-pentamethylbenzenesulfonyl, *N*-2,3,5,6,-tetramethyl-4-methoxybenzenesulfonyl, *N*-4-methoxybenzenesulfonyl, *N*-2,4,6-trimethylbenzenesulfonyl, *N*-2,6-dimethoxy-4-methylbenzenesulfonyl, *N-*2,2,5,7,8-pentamethylchroman-6-sulfonyl, *N*-methanesulfonyl, *N*-β-trimethylsilyethanesulfonyl, *N*-9-anthracenesulfonyl, *N*-4-(4',8'-dimethoxynaphthylmethyl)benzenesulfonyl, *N*-benzylsulfonyl, *N-*trifluoromethylsulfonyl, *N*-phenacylsulfonyl).

More typically, protected amino groups include carbamates and amides, still more typically, -NHC(O)R¹ or -N=CR¹N(R¹)₂. Another protecting group, also useful as a prodrug for amino or -NH(R⁵), is: 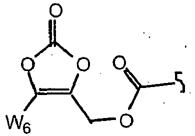 See for example Alexander, J. et al. (1996) J. Med. Chem. 39:480-486.

### Amino acid and polypeptide protecting group and conjugates

An amino acid or polypeptide protecting group of a compound of the invention has the structure R¹⁵NHCH(R¹⁶)C(O)-, where R¹⁵ is H, an amino acid or polypeptide residue, or R⁵, and R¹⁶ is defined below.

R¹⁶ is lower alkyl or lower alkyl (C₁-C₆) substituted with amino, carboxyl, amide, carboxyl ester, hydroxyl, C₆-C₇ aryl, guanidinyl, imidazolyl, indolyl, sulfhydryl, sulfoxide, and/or alkylphosphate. R¹⁰ also is taken together with the amino acid α 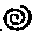 N to form a proline residue (R¹⁰ = -CH₂)₃-). However, R¹⁰ is generally the side group of a naturally-occurring amino acid such as H, -CH₃, -CH(CH₃)₂,-CH₂-CH(CH₃)₂, ₋CHCH₃-CH₂-CH₃, -CH₂-C₆H₅, -CH₂CH₂-S-CH₃, -CH₂OH,-CH(OH)-CH₃, -CH₂-SH, -CH₂-C₆H₄OH, -CH₂-CO-NH₂, -CH₂-CH₂-CO-NH₂, -CH₂-COOH, -CH₂-CH₂-COOH, -(CH₂)₄-NH₂ and -(CH₂)₃-NH-C(NH₂)-NH₂. R₁₀ also includes 1-guanidinoprop-3-yl, benzyl, 4-hydroxybenzyl, imidazol-4-yl, indol-3-yl, methoxyphenyl and ethoxyphenyl.

Another set of protecting groups include the residue of an amino-containing compound, in particular an amino acid, a polypeptide, a protecting group,-NHSO₂R, NHC(O)R, -N(R)₂, NH₂ or -NH(R)(H), whereby for example a carboxylic acid is reacted, *i.e*. coupled, with the amine to form an amide, as in C(O)NR₂. A phosphonic acid may be reacted with the amine to form a phosphonamidate, as in-P(O)(OR)(NR₂).

In general, amino acids have the structure R¹⁷C(O)CH(R¹⁶)NH-, where R¹⁷ is-OH, -OR, an amino acid or a polypeptide residue. Amino acids are low molecular weight compounds, on the order of less than about 1000 MW and which contain at least one amino or imino group and at least one carboxyl group. Generally the amino acids will be found in nature, *i.e*., can be detected in biological material such as bacteria or other microbes, plants, animals or man. Suitable amino acids typically are alpha amino acids, *i.e*. compounds characterized by one amino or imino nitrogen atom separated from the carbon atom of one carboxyl group by a single substituted or unsubstituted alpha carbon atom. Of particular interest are hydrophobic residues such as mono-or di-alkyl or aryl amino acids, cycloalkylamino acids and the like. These residues contribute to cell permeability by increasing the partition coefficient of the parental drug. Typically, the residue does not contain a sulfhydryl or guanidino substituent.

Naturally-occurring amino acid residues are those residues found naturally in plants, animals or microbes, especially proteins thereof. Polypeptides most typically will be substantially composed of such naturally-occurring amino acid residues. These amino acids are glycine, alanine, valine, leucine, isoleucine, serine, threonine, cysteine, methionine, glutamic acid, aspartic acid, lysine, hydroxylysine, arginine, histidine, phenylalanine, tyrosine, tryptophan, proline, asparagine, glutamine and hydroxyproline. Additionally, unnatural amino acids, for example, valanine, phenylglycine and homoarginine are also included. Commonly encountered amino acids that are not gene-encoded may also be used in the present invention. All of the amino acids used in the present invention may be either the D- or L- optical isomer. In addition, other peptidomimetics are also useful in the present invention. For a general review, see Spatola, A. F., in Chemistry and Biochemistry of Amino Acids, Peptides and Proteins, B. Weinstein, eds., Marcel Dekker, New York, p. 267 (1983).

When protecting groups are single amino acid residues or polypeptides they optionally are substituted at R³ of substituents A¹, A² or A³ in a compound of the invention. These conjugates are produced by forming an amide bond between a carboxyl group of the amino acid (or C-terminal amino acid of a polypeptide for example). Similarly, conjugates are formed between R³ and an amino group of an amino acid or polypeptide. Generally, only one of any site in the parental molecule is amidated with an amino acid as described herein, although it is within the scope of this invention to introduce amino acids at more than one permitted site. Usually, a carboxyl group of R³ is amidated with an amino acid. In general, the α-amino or α-carboxyl group of the amino acid or the terminal amino or carboxyl group of a polypeptide are bonded to the parental functionalities, *i.e*., carboxyl or amino groups in the amino acid side chains generally are not used to form the amide bonds with the parental compound (although these groups may need to be protected during synthesis of the conjugates as described further below).

With respect to the carboxyl-containing side chains of amino acids or polypeptides it will be understood that the carboxyl group optionally will be blocked, *e.g*., by R¹, esterified with R⁵ or amidated. Similarly, the amino side chains R¹⁶ optionally will be blocked with R¹ or substituted with R⁵.

Such ester or amide bonds with side chain amino or carboxyl groups, like the esters or amides with the parental molecule, optionally are hydrolyzable *in vivo* or *in vitro* under acidic (pH <3) or basic (pH >10) conditions. Alternatively, they are substantially stable in the gastrointestinal tract of humans but are hydrolyzed enzymatically in blood or in intracellular environments. The esters or amino acid or polypeptide amidates also are useful as intermediates for the preparation of the parental molecule containing free amino or carboxyl groups. The free acid or base of the parental compound, for example, is readily formed from the esters or amino acid or polypeptide conjugates of this invention by conventional hydrolysis procedures.

When an amino acid residue contains one or more chiral centers, any of the D, L, meso, threo or erythro (as appropriate) racemates, scalemates or mixtures thereof may be used. In general, if the intermediates are to be hydrolyzed non-enzymatically (as would be the case where the amides are used as chemical intermediates for the free acids or free amines), D isomers are useful. On the other hand, L isomers are more versatile since they can be susceptible to both non-enzymatic and enzymatic hydrolysis, and are more efficiently transported by amino acid or dipeptidyl transport systems in the gastrointestinal tract.

Examples of suitable amino acids whose residues are represented by R^{x} or R^{y} include the following:
Glycine;
Aminopolycarboxylic acids, *e.g*., aspartic acid, β-hydroxyaspartic acid, glutamic acid, β-hydroxyglutamic acid, β-methylaspartic acid, β-methylglutamic acid, β, β-dimethylaspartic acid, γ-hydroxyglutamic acid, β, γ-dihydroxyglutamic acid, β-phenylglutamic acid, γ-methyleneglutamic acid, 3-aminoadipic acid, 2-aminopimelic acid, 2-aminosuberic acid and 2-aminosebacic acid;
Amino acid amides such as glutamine and asparagine;
Polyamino- or polybasic-monocarboxylic acids such as arginine, lysine, β-aminoalanine, γ -aminobutyrine, ornithine, citruline, homoarginine, homocitrulline, hydroxylysine, allohydroxylsine and diaminobutyric acid;
Other basic amino acid residues such as histidine;
Diaminodicarboxylic acids such as α, α'-diaminosuccinic acid, α, α'-diaminoglutaric acid, α, α'-diaminoadipic acid, α, α'-diaminopimelic acid, α, α'-diamino- β-hydroxypimelic acid, α, α'-diaminosuberic acid, α, α'-diaminoazelaic acid, and α, α'-diaminosebacic acid;
Imino acids such as proline, hydroxyproline, allohydroxyproline, γ-methylproline, pipecolic acid, 5-hydroxypipecolic acid, and azetidine-2-carboxylic acid;
A mono- or di-alkyl (typically C₁-C₈ branched or normal) amino acid such as alanine, valine, leucine, allylglycine, butyrine, norvaline, norleucine, heptyline, α-methylserine, α-amino-α-methyl-γ-hydroxyvaleric acid, α-amino- α-methyl-δ-hydroxyvaleric acid, α-amino- α-methyl-ε-hydroxycaproic acid, isovaline, α-methylglutamic acid, α-aminoisobutyric acid, α-aminodiethylacetic acid, α-aminodiisopropylacetic acid, α-aminodi-n-propylacetic acid, α-aminodiisobutylacetic acid, α-aminodi-n-butylacetic acid, α-aminoethylisopropylacetic acid, α-amino-n-propylacetic acid, α-) aminodiisoamyacetic acid, α-methylaspartic acid, α-methylglutamic acid,1-aminocyclopropane-1-carboxylic acid, isoleucine, alloisoleucine, *tert-*leucine, β-methyltryptophan and α-amino- β-ethyl-β-phenylpropionic acid;
β-phenylserinyl;
Aliphatic α-amino-β-hydroxy acids such as serine, β-hydroxyleucine, β-hydroxynorleucine, β-hydroxynorvaline, and α-amino-β-hydroxystearic acid;
α-Amino, α-, γ-, δ- or ε-hydroxy acids such as homoserine, δ - hydroxynorvaline, γ-hydroxynorvaline and ε-hydroxynorleucine residues; canavine and canaline; γ -hydroxyornithine;
2-hexosaminic acids such as D-glucosaminic acid or D-galactosaminic acid;
α-Amino-β-thiols such as penicillamine, β-thiolnorvaline or β-thiolbutyrine;
Other sulfur containing amino acid residues including cysteine; homocystine, β-phenylmethionine, methionine, S-allyl-L-cysteine sulfoxide, 2-thiolhistidine, cystathionine, and thiol ethers of cysteine or homocysteine;
Phenylalanine, tryptophan and ring-substituted α-amino acids such as the phenyl- or cyclohexylamino acids α-aminophenylacetic acid, α-aminocyclohexylacetic acid and α-amino-β-cyclohexylpropionic acid; phenylalanine analogues and derivatives comprising aryl, lower alkyl, hydroxy, guanidino, oxyalkylether, nitro, sulfur or halo-substituted phenyl (*e.g*., tyrosine, methyltyrosine and o-chloro-, p-chloro-, 3,4-dichloro, *o*-, *m*- or *p*-methyl-, 2,4,6-trimethyl-, 2-ethoxy-5-nitro-, 2-hydroxy-5-nitro- and p-nitro-phenylalanine); furyl-, thienyl-, pyridyl-, pyrimidinyl-, purinyl- or naphthyl-alanines; and tryptophan analogues and derivatives including kynurenine, 3-hydroxykynurenine, 2-hydroxytryptophan and 4-carboxytryptophan;
α-Amino substituted amino acids including sarcosine (N-methylglycine), N-benzylglycine, N-methylalanine, N-benzylalanine, N-methylphenylalanine, N-benzylphenylalanine, N-methylvaline and N-benzylvaline; and
α-Hydroxy and substituted α -hydroxy amino acids including serine, threonine, allothreonine, phosphoserine and phosphothreonine.

Polypeptides are polymers of amino acids in which a carboxyl group of one amino acid monomer is bonded to an amino or imino group of the next amino acid monomer by an amide bond. Polypeptides include dipeptides, low molecular weight polypeptides (about 1500-5000 MW) and proteins. Proteins optionally contain 3, 5, 10, 50, 75, 100 or more residues, and suitably are substantially sequence-homologous with human, animal, plant or microbial proteins. They include enzymes (*e.g*., hydrogen peroxidase) as well as immunogens such as KLH, or antibodies or proteins of any type against which one wishes to raise an immune response. The nature and identity of the polypeptide may vary widely.

The polypeptide amidates are useful as immunogens in raising antibodies against either the polypeptide (if it is not immunogenic in the animal to which it is administered) or against the epitopes on the remainder of the compound of this invention.

Antibodies capable of binding to the parental non-peptidyl compound are used to separate the parental compound from mixtures, for example in diagnosis or manufacturing of the parental compound. The conjugates of parental compound and polypeptide generally are more immunogenic than the polypeptides in closely homologous animals, and therefore mal<e the polypeptide more immunogenic for facilitating raising antibodies against it. Accordingly, the polypeptide or protein may not need to be immunogenic in an animal typically used to raise antibodies, *e.g*., rabbit, mouse, horse, or rat, but the final product conjugate should be immunogenic in at least one of such animals. The polypeptide optionally contains a peptidolytic enzyme cleavage site at the peptide bond between the first and second residues adjacent to the acidic heteroatom. Such cleavage sites are flanged by enzymatic recognition structures, *e.g*., a particular sequence of residues recognized by a peptidolytic enzyme.

Peptidolytic enzymes for cleaving the polypeptide conjugates of this invention are well known, and in particular include carboxypeptidases. Carboxypeptidases digest polypeptides by removing C-terminal residues, and are specific in many instances for particular C-terminal sequences. Such enzymes and their substrate requirements in general are well known. For example, a dipeptide (having a given pair of residues and a free carboxyl terminus) is covalently bonded through its α-amino group to the phosphorus or carbon atoms of the compounds herein. In embodiments where W₁ is phosphonate it is expected that this peptide will be cleaved by the appropriate peptidolytic enzyme, leaving the carboxyl of the proximal amino acid residue to autocatalytically cleave the phosphonoamidate bond.

Suitable dipeptidyl groups (designated by their single letter code) are AA, AR, AN, AD, AC, AE, AQ, AG, AH, AI, AL, AK, AM, AF, AP, AS, AT, AW, AY, AV; RA, RR, RN, RD, RC, RE, RQ, RG, RH, RI, RL, RK, RM, RF, RP, RS, RT, RW, RY, RV, NA, NR, NN, ND, NC, NE, NQ, NG, NH, NI, NL, NK, NM, NF, NP, NS, NT, NW, NY, NV, DA, DR, DN, DD, DC, DE, DQ, DG, DH, DI, DL, DK, DM, DF, DP, DS, DT, DW, DY, DV, CA, CR, CN, CD, CC, CE, CQ, CG, CH, CI, CL, CK, CM, CF, CP, CS, CT, CW, CY, CV, EA, ER, EN, ED, EC, EE, EQ, EG, EH, EI, EL, EK, EM, EF, EP, ES, ET, EW, EY, EV, QA, QR, QN, QD, QC, QE, QQ, QG, QH, QI, QL, QK, QM, QF, QP, QS, QT, QW, QY, QV, GA,GR,GN,GD,GC,GE,GQ,GG,GH,GI,GL,GK,GM,GF, GP, GS, GT, GW, GY, GV, HA, HR, HN, HD, HC, HE, HQ HG, HH, HI, HL, HK, HM, HF, HP, HS, HT, HW, HY, HV, IA, IR, IN, ID, IC, IE, IQ, IG, IH, II, IL, IK, IM, IF, IP, IS,IT, IW, IY, IV, LA, LR, LN, LD, LC, LE, LQ, LG, LH, LI, LL, LK, LM, LF, LP, LS, LT, LW, LY, LV, KA, KR, KN, KD, KC, KE, KQ, KG, KH, KI, KL, KK, KM, KF, KP, KS, KT, KW, KY, KV, MA, MR, MN, MD, MC, ME, MQ MG, MH, MI, ML, MK, MM, MF, MP, MS, MT, MW, MY, MV, FA, FR, FN, FD, FC, FE, FQ, FG, FH, FI, FL, FK, FM, FF, FP, FS, FT, FW, FY, FV, PA, PR, PN, PD, PC, PE, PQ, PG, PH, PI, PL, PK, PM, PF, PP, PS, PT, PW, PY, PV, SA, SR, SN, SD, SC, SE, SQ, SG, SH, SI, SL, SK, SM, SF, SP, SS, ST, SW, SY, SV, TA, TR, TN, TD, TC, TE, TQ, TG, TH, TI, TL, TK, TM, TF, TP, TS, TT, TW, TY, TV, WA, WR, WN, WD, WC, WE, WQ WG, WH, WI, WL, WK, WM, WF, WP, WS, WT, WW, WY, WV, YA, YR, YN, YD, YC, YE, YQ, YG, YH, YI, YL, YK, YM, YF, YP, YS, YT, YW, YY, YV, VA, VR, VN, VD, VC, VE, VQ, VG, VH, VI, VL, VK, VM, VF, VP, VS, VT, VW, VY and VV.

Tripeptide residues are also useful as protecting groups. When a phosphonate is to be protected, the sequence -X⁴-pro-X⁵- (where X⁴ is any amino acid residue and X⁵ is an amino'acid residue, a carboxyl ester of proline, or hydrogen) will be cleaved by luminal carboxypeptidase to yield X⁴ with a free carboxyl, which in turn is expected to autocatalytically cleave the phosphonoamidate bond. The carboxy group of X⁵ optionally is esterified with benzyl.

Dipeptide or tripeptide species can be selected on the basis of known transport properties and/or susceptibility to peptidases that can affect transport to intestinal mucosal or other cell types. Dipeptides and tripeptides lacking an α-amino group are transport substrates for the peptide transporter found in brush border membrane of intestinal mucosal cells (Bai, J.P.F., (1992) Pharm Res. 9:969-978). Transport competent peptides can thus be used to enhance bioavailability of the amidate compounds. Di- or tripeptides having one or more amino acids in the D configuration are also compatible with peptide transport and can be utilized in the amidate compounds of this invention. Amino acids in the D configuration can be used to reduce the susceptibility of a di- or tripeptide to hydrolysis by proteases common to the brush border such as aminopeptidase N. In addition, di- or tripeptides alternatively are selected on the basis of their relative resistance to hydrolysis by proteases found in the lumen of the intestine. For example, tripeptides or polypeptides lacking asp and/or glu are poor substrates for aminopeptidase A, di- or tripeptides lacking amino acid residues on the N-terminal side of hydrophobic amino acids (leu, tyr, phe, val, trp) are poor substrates for endopeptidase, and peptides lacking a pro residue at the penultimate position at a free carboxyl terminus are poor substrates for carboxypeptidase P. Similar considerations can also be applied to the selection of peptides that are either relatively resistant or relatively susceptible to hydrolysis by cytosolic, renal, hepatic, serum or other peptidases. Such poorly cleaved polypeptide amidates are immunogens or are useful for bonding to proteins in order to prepare immunogens.

### Specific Embodiments of the Invention

Specific values described for radicals, substituents, and ranges, as well as specific embodiments of the invention described herein, are for illustration only; they do not exclude other defined values or other values within defined ranges.

In a specific embodiment of the invention A² is of the formula:

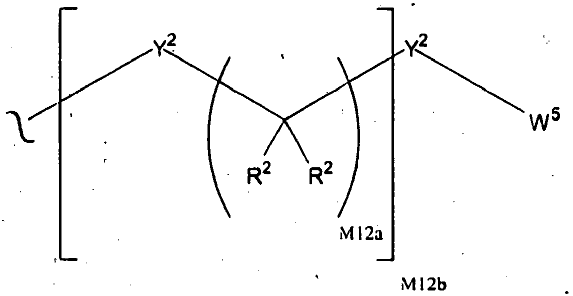

In another specific embodiment of the invention A² is of the formula:

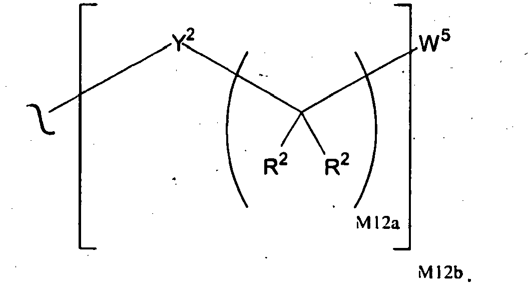

In another specific embodiment of the invention M12b is 1.

In another specific embodiment of the invention M12b is 0, Y² is a bond and W⁵ is a carbocycle or heterocycle where W⁵ is optionally and independently substituted with 1, 2, or 3 R² groups.

In another specific embodiment of the invention A² is of the formula: 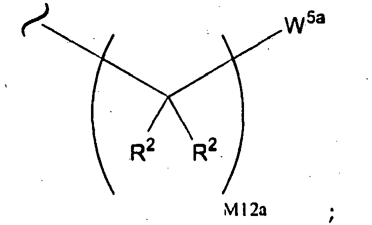 wherein W^{5a} is a carbocycle or heterocycle where W^{5a} is optionally and independently substituted with 1, 2, or 3 R² groups.

In another specific embodiment of the invention M12a is 1.

In another specific embodiment of the invention A² is selected from phenyl, substituted phenyl, benzyl, substituted benzyl, pyridyl and substituted pyridyl.

In another specific embodiment of the invention A² is of the formula:

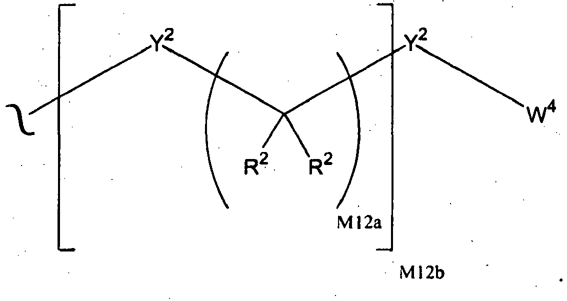

In another specific embodiment of the invention A² is of the formula:

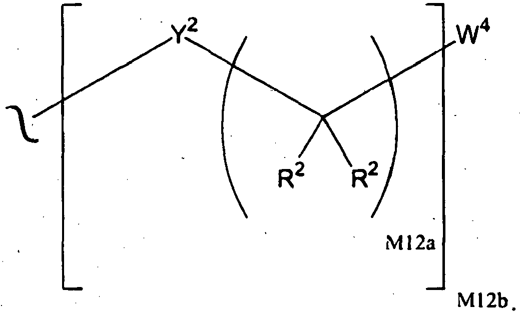

In another specific embodiment of the invention M12b is 1.

In a specific embodiment of the invention A³ is of the formula:

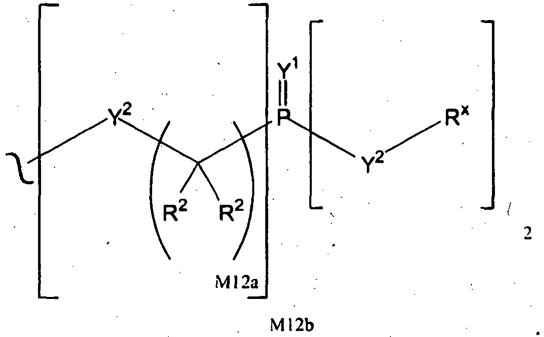

In another specific embodiment of the invention A³ is of the formula:

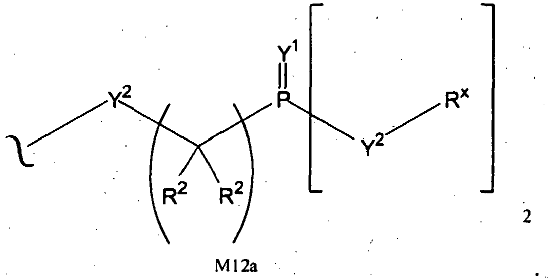

In another specific embodiment of the invention A³ is of the formula: 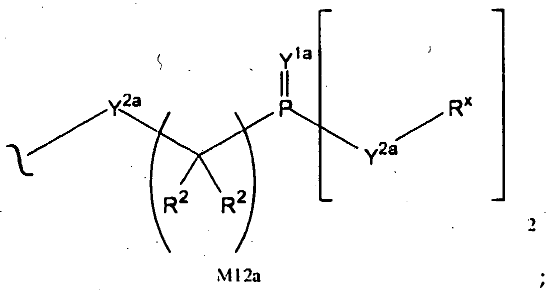 wherein Y^{1a} is O or S; and Y^{2a} is O, N(R^{x}) or S.

In another specific embodiment of the invention A³ is of the formula: 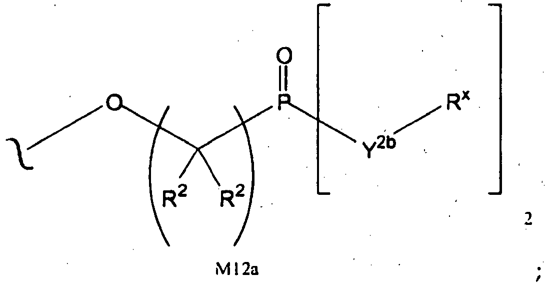 wherein Y^{2b} is O or N(R^{x}).

In another specific embodiment of the invention A³ is of the formula: 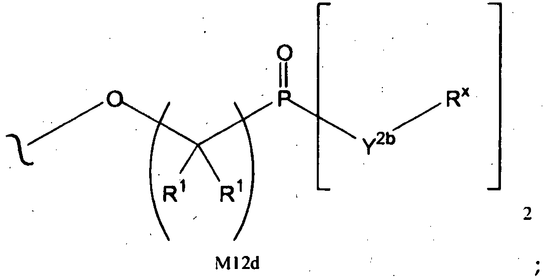 wherein Y^{2b} is O or N(R^{x}); and M12d is 1, 2, 3, 4, 5, 6, 7 or 8.

In another specific embodiment of the invention A³ is of the formula: 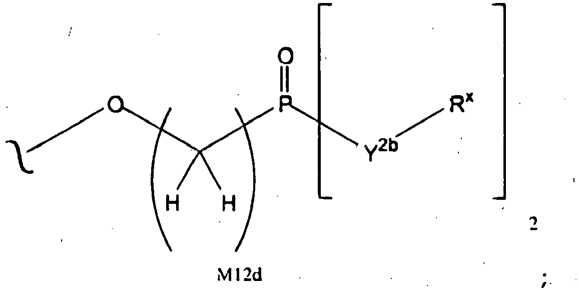 wherein Y^{2b} is 0 or N(R^{x}); and M12d is 1, 2, 3, 4, 5, 6, 7 or 8.

In another specific embodiment of the invention M12d is 1.

In another specific embodiment of the invention A³ is of the formula:

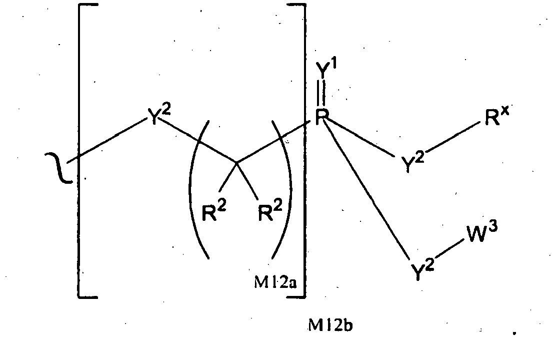

In another specific embodiment of the invention A³ is of the formula:

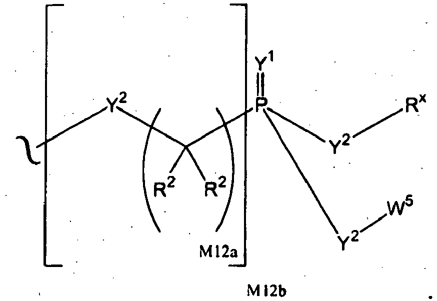

In another specific embodiment of the invention W⁵ is a carbocycle.

In another specific embodiment of the invention A³ is of the formula:

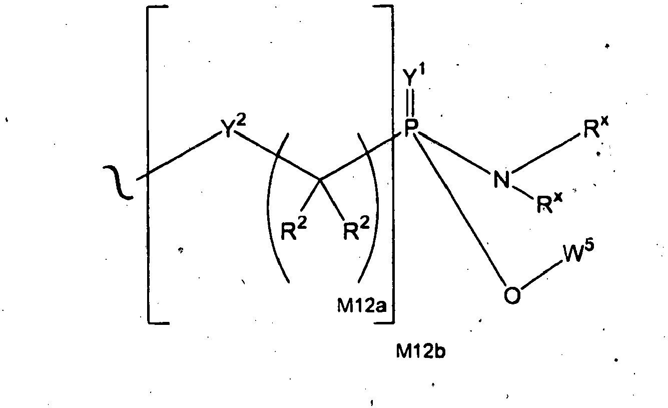

In another specific embodiment of the invention W⁵ is phenyl.

In another specific embodiment of the invention A³ is of the formula: 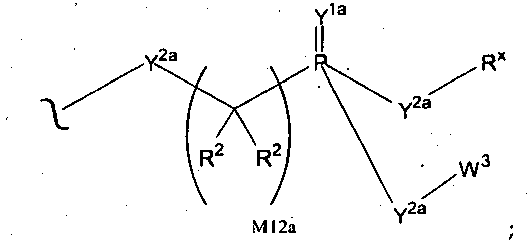 wherein Y^{1a} is O or S; and Y^{2a} is O, N(R^{x}) or S.

In another specific embodiment of the invention A³ is of the formula: 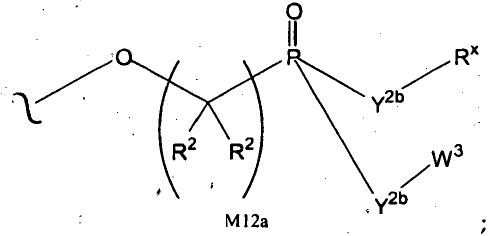 wherein Y^{2b} is O or N(R^{x}).

In another specific embodiment of the invention A³ is of the formula: 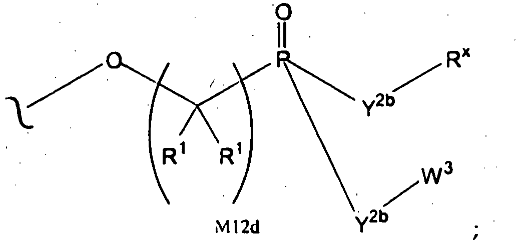 wherein Y^{2b} is O or N(R^{x}); and M12d is 1, 2,3,4, 5, 6, 7 or 8.

In another specific embodiment of the invention R¹ is H.

In another specific embodiment of the invention A³ is of the formula: 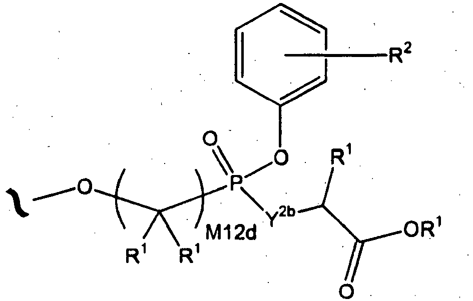 wherein the phenyl carbocycle is substituted with 0, 1, 2, or 3 R² groups.

In another specific embodiment of the invention A³ is of the formula:

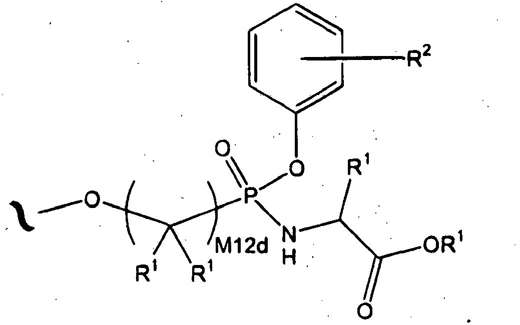

In another specific embodiment of the invention A³ is of the formula:

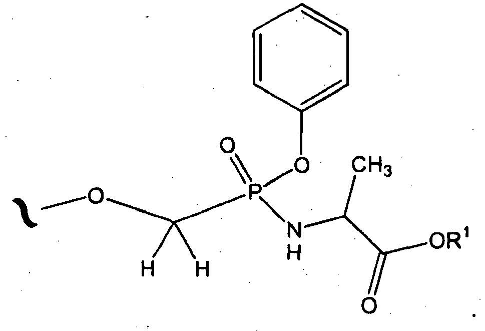

In another specific embodiment of the invention A³ is of the formula:

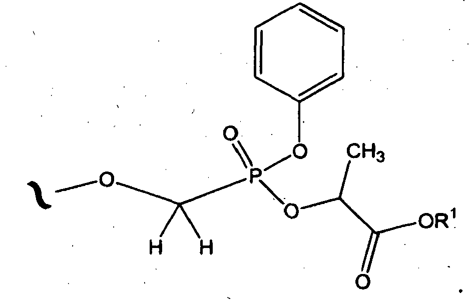

In another specific embodiment of the invention A³ is of the formula:

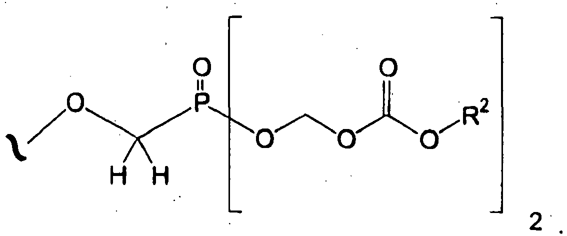

In another specific embodiment of the invention A³ is of the formula: 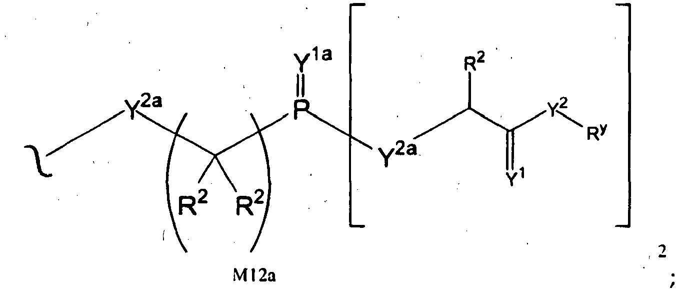 wherein Y^{1a} is O or S; and Y^{2a} is O, N(R²) or S.

In another specific embodiment of the invention A³ is of the formula: 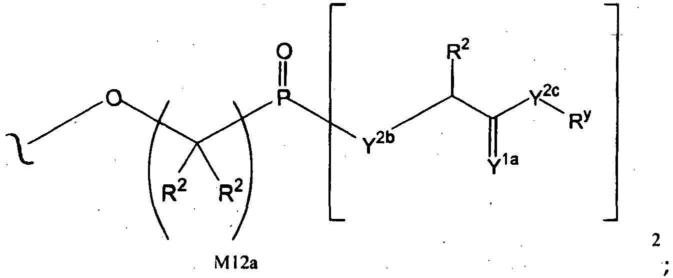 wherein Y^{1a} is O or S; Y^{2b} is O or N(R²); and Y^{2c} is O, N(R^{y}) or S.

In another specific embodiment of the invention A³ is of the formula: 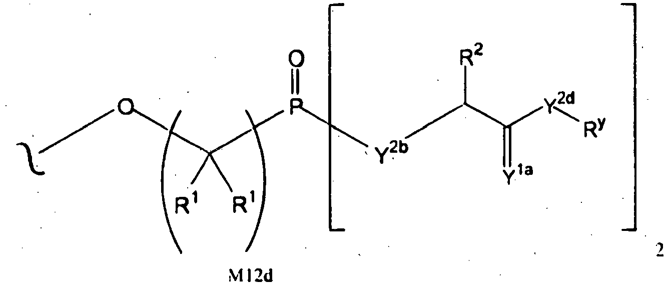 wheren Y^{1a} is O or S; Y^{2b} is O or N(R²); Y^{2d} is O or N(R^{y}); and M12d is 1, 2, 3, 4, 5, 6, 7 or 8.

In another specific embodiment of the invention A³ is of the formula: 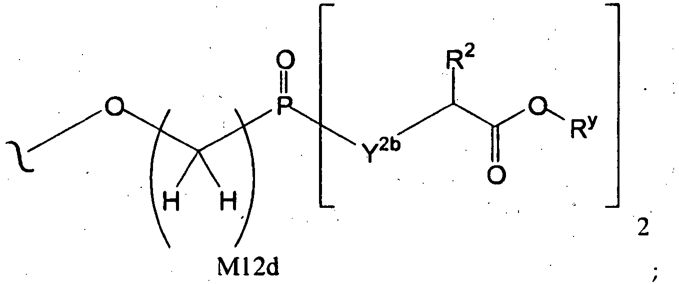 wherein Y^{2b} is O or N(R²); and M12d is 1, 2, 3, 4, 5, 6, 7 or 8.

In another specific embodiment of the invention A³ is of the formula: 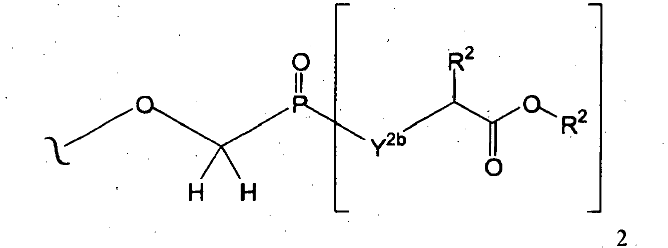 wherein Y^{2b} is O or N(R²).

In another specific embodiment of the invention A³ is of the formula:

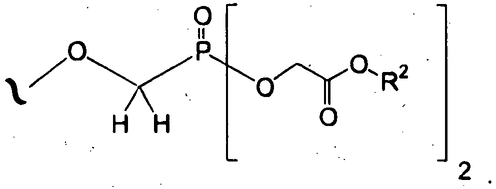

In another specific embodiment of the invention A³ is of the formula:

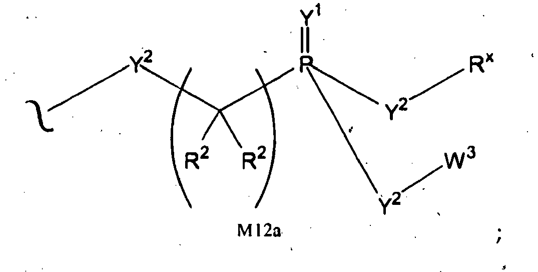

In another specific embodiment of the invention A³ is of the formula: 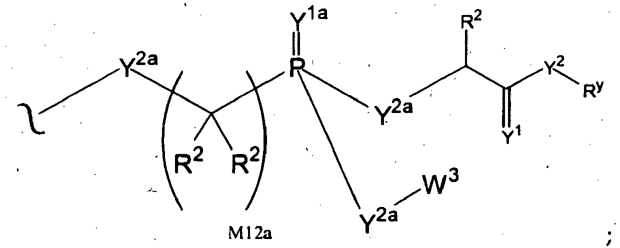 wherein Y^{1a} is O or S; and Y^{2a} is O, N(R²) or S.

In another specific embodiment of the invention A³ is of the formula: 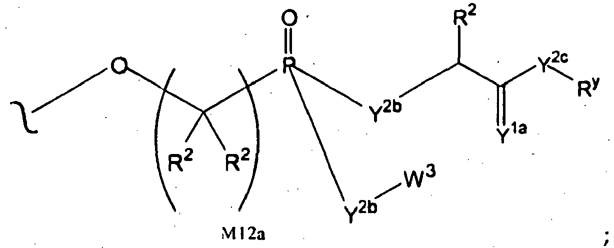 wherein Y^{1a} is O or S; Y^{2b} is O or N(R²); and Y^{2c} is O, N(R^{y}) or S.

In another specific embodiment of the invention A³ is of the formula: 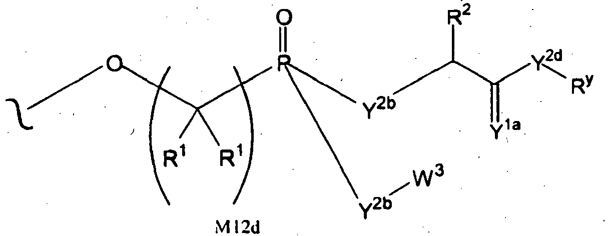 wherein Y^{1a} is O or S; Y^{2b} is O or N(R²); Y^{2d} is O or N(R^{y}); and M12d is 1, 2, 3, 4, 5, 6, 7 or 8.

In another specific embodiment of the invention A³ is of the formula: 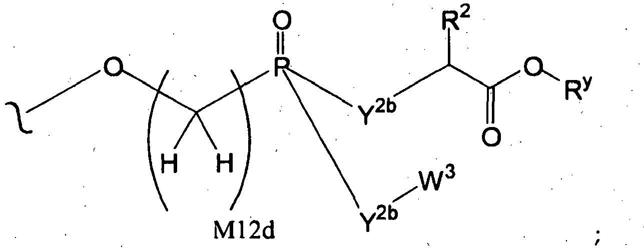 wherein Y^{2b} is O or N(R²); and M12d is 1, 2, 3, 4, 5, 6, 7 or 8.

In another specific embodiment of the invention A³ is of the formula: 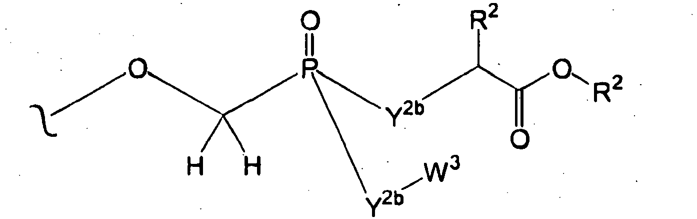 wherein Y^{2b} is O or N(R²).

In another specific embodiment of the invention A³ is of the formula: 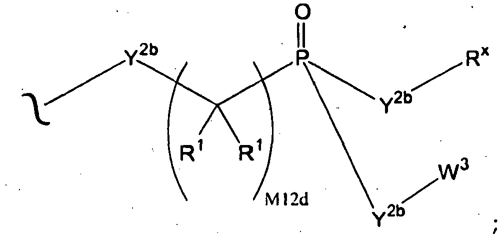 wherein: Y^{2b} is O or N(R^{x}); and M12d is 1, 2, 3, 4, 5, 6, 7 or 8.

In another specific embodiment of the invention A³ is of the formula: 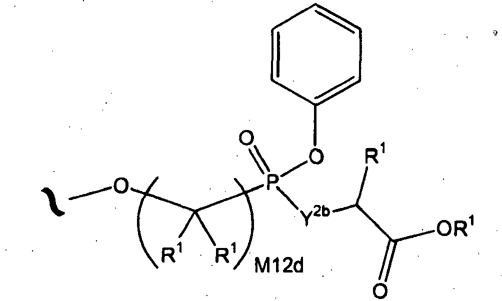 wherein the phenyl carbocycle is substituted with 0, 1, 2, or 3 R² groups.

In another specific embodiment of the invention A³ is of the formula: 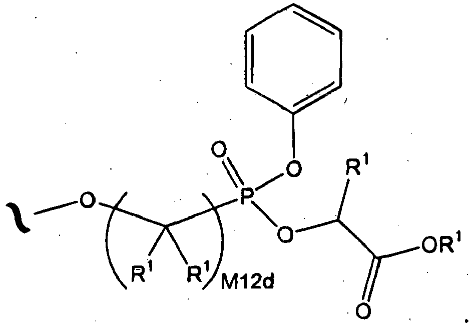 wherein the phenyl carbocycle is substituted with 0, 1, 2, or 3 R² groups.

In another specific embodiment of the invention A³ is of the formula:

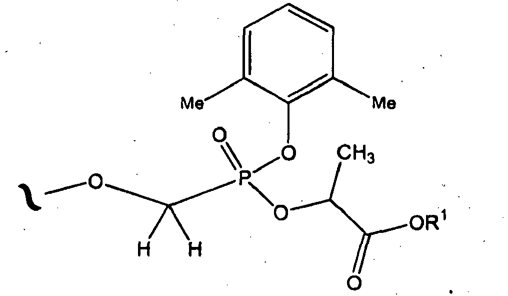

In a specific embodiment of the invention A⁰ is of the formula: 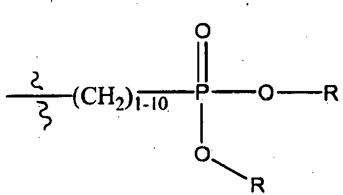 wherein each R is independently (C₁-C₆)alkyl.

In a specific embodiment of the invention R^{x} is independently H, R¹, W³, a protecting group, or the formula: 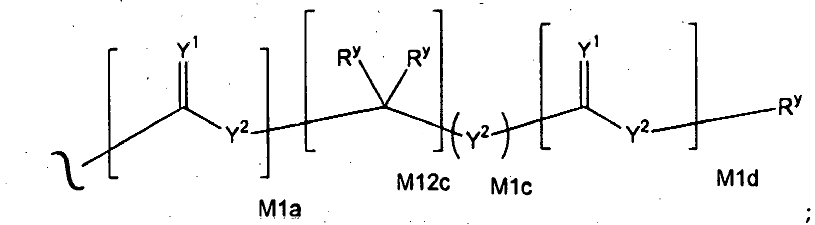 wherein:
R^{y} is independently H, W³, R² or a protecting group;
R¹ is independently H or alkyl of 1 to 18 carbon atoms;
R² is independently H, R¹, R³ or R⁴ wherein each R⁴ is independently substituted with 0 to 3 R³ groups or taken together at a carbon atom, two R² groups form a ring of 3 to 8 carbons and the ring may be substituted with 0 to 3 R³ groups;
wherein R³ is as defined herein.

In a specific embodiment of the invention R^{x} is of the formula: 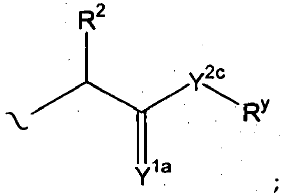 wherein Y^{1a} is O or S; and Y^{2c} is O, N(R^{y}) or S.

In a specific embodiment of the invention R^{x} is of the formula: 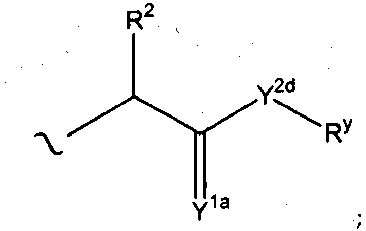 wherein Y^{1a} is O or S; and Y^{2d} is O or N(R^{y}).

In a specific embodiment of the invention R^{x} is of the formula:

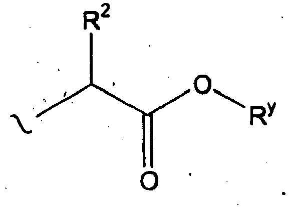

In a specific embodiment of the invention R^{y} is hydrogen or alkyl of 1 to 10 carbons.

In a specific embodiment of the invention R^{x} is of the formula:

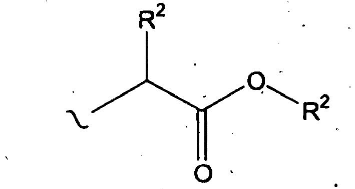

In a specific embodiment of the invention R^{x} is of the formula:

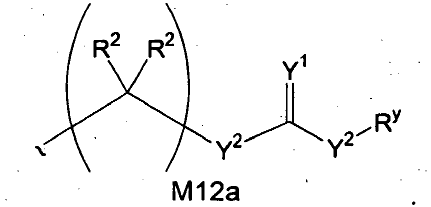

In a specific embodiment of the invention R^{x} is of the formula:

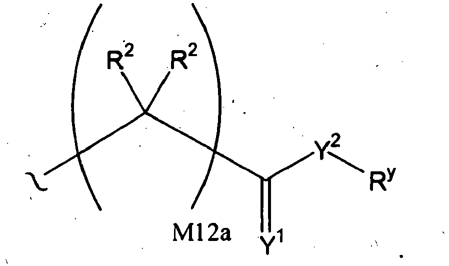

In a specific embodiment of the invention Y¹ is O or S

In a specific embodiment of the invention Y² is O, N(R^{y}) or S.

In one specific embodiment of the invention R^{x} is a group of the formula: 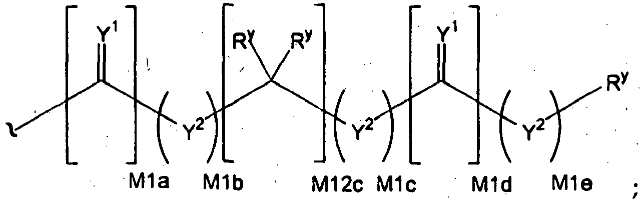 wherein:
m1a, mlb, m1c, m1d and mle are independently 0 or 1;
m12c is 0, 1, 2, 3, 4, 5, 6, 7, 8, 9, 10, 11 or 12;
R^{y} is H, W³, R² or a protecting group;
wherein W³ , R², Y¹ and Y² are as defined herein;
*provided that:*
if m1a, m12c, and m1d are 0, then mlb, m1c and mle are 0;
if m1a and m12c are 0 and m1d is not 0, then mlb and m1c are 0;
if m1a and m1d are 0 and m12c is not 0, then mlb and at least one of m1c and mle are 0;
if m1a is 0 and m12c and m1d are not 0, then m1b is 0;
if m12c and m1d are 0 and m1a is not 0, then at least two of mlb, m1c and m1e are 0;
if m12c is 0 and m1a and m1d are not 0, then at least one of mlb and m1c are 0; and
if m1d is 0 and m1a and m12c are not 0, then at least one of m1c and m1e are 0.

In compounds of the invention W⁵ carbocycles and W⁵ heterocycles may be independently substituted with 0 to 3 R² groups. W⁵ may be a saturated, unsaturated or aromatic ring comprising a mono- or bicyclic carbocycle or heterocycle. W⁵ may have 3 to 10 ring atoms, e.g., 3 to 7 ring atoms. The W⁵ rings are saturated when containing 3 ring atoms, saturated or mono-unsaturated when containing 4 ring atoms, saturated, or mono- or di-unsaturated when containing 5 ring atoms, and saturated, mono- or di-unsaturated, or aromatic when containing 6 ring atoms.

A W⁵ heterocycle may be a monocycle having 3 to 7 ring members (2 to 6 carbon atoms and 1 to 3 heteroatoms selected from' N, O, P, and S) or a bicycle having 7 to 10 ring members (4 to 9 carbon atoms and 1 to 3 heteroatoms selected from N, O, P, and S). W⁵ heterocyclic monocycles may have 3 to 6 ring atoms (2 to 5 carbon atoms and 1 to 2 heteroatoms selected from N, O, and S); or 5 or 6 ring atoms (3 to 5 carbon atoms and 1 to 2 heteroatoms selected from N and S). W⁵ heterocyclic bicycles have 7 to 10 ring atoms (6 to 9 carbon atoms and 1 to 2 heteroatoms selected from N, O, and S) arranged as a bicyclo [4,5], [5,5], [5,6], or [6,6] system; or 9 to 10 ring atoms (8 to 9 carbon atoms and 1 to 2 hetero atoms selected from N and S) arranged as a bicyclo [5,6] or [6,6] system. The W⁵ heterocycle may be bonded to Y² through a carbon, nitrogen, sulfur or other atom by a stable covalent bond.

W⁵ heterocycles include for example, pyridyl, dihydropyridyl isomers, piperidine, pyridazinyl, pyrimidinyl, pyrazinyl, s-triazinyl, oxazolyl, imidazolyl, thiazolyl, isoxazolyl, pyrazolyl, isothiazolyl, furanyl, thiofuranyl, thienyl, and pyrrolyl. W⁵ also includes, but is not limited to, examples such as:

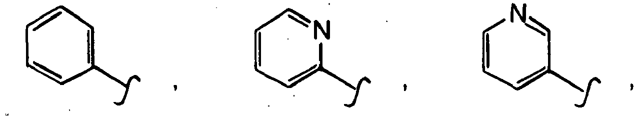

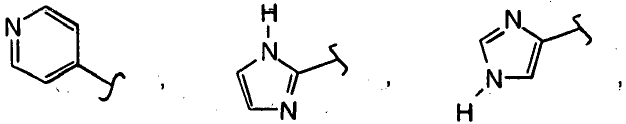

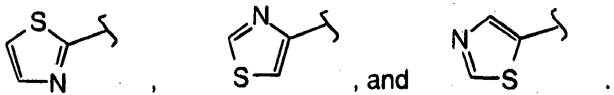

W⁵ carbocycles and heterocycles may be independently substituted with 0 to 3 R² groups, as defined above. For example, substituted W⁵ carbocycles include:

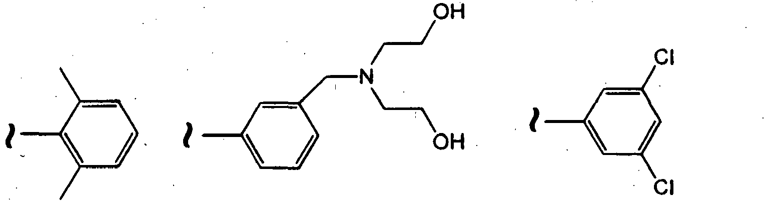

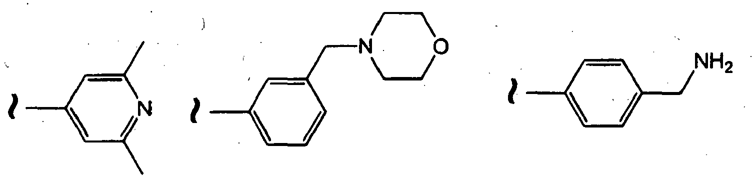

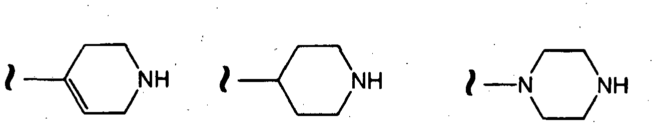

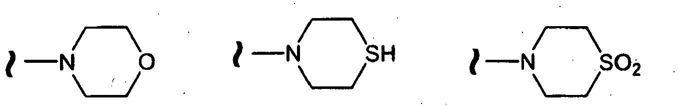

Examples of substituted phenyl carbocycles include:

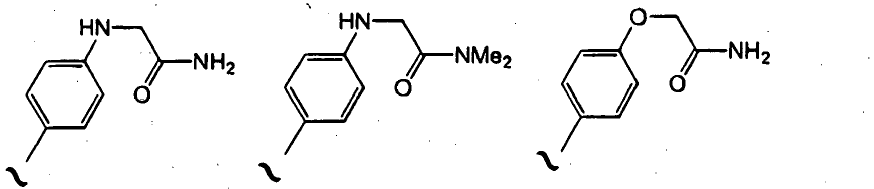

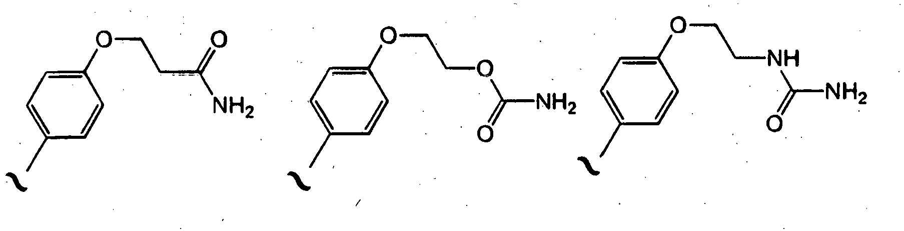

In another specific embodiment the invention provides a compound of formula II, 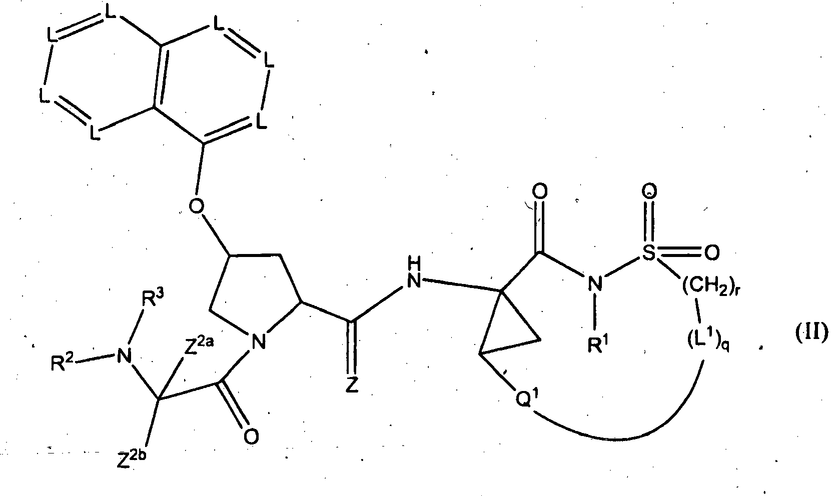 or a pharmaceutically acceptable salt, or solvate thereof,
wherein,
R¹ is independently selected from H, alkyl, alkenyl, alkynyl, aryl, cycloalkyl, heterocycle, halogen, haloalkyl, alkylsulfonamido, arylsulfonamido, - C(O)NHS(O)₂-, or -S(O)₂-, optionally substituted with one or more A³;
R² is (C2-10)alkyl, (C3-7)cycloalkyl or (C1-4)alkyl-(C3-7)cycloalkyl,
where said cycloalkyl and alkyl-cycloalkyl may be mono-, di- or tri-substituted with (C1-3)alkyl, or
where said alkyl, cycloalkyl and alkyl-cycloalkyl may be mono- or disubstituted with substituents selected from hydroxy and O-(C1-4)alkyl, or
where each of said alkyl-groups may be mono-, di- or tri-substituted with halogen, or
where each of said cycloalkyl groups being 5-, 6- or 7-membered, one or two -CH2-groups not being directly linked to each other may be replaced by -O- such that the O-atom is linked to the N atom to which R² is attached via at least two C-atoms, or
R² is phenyl, (C1-3)alkyl-phenyl, heteroaryl or (C1-3)alkyl-heteroaryl, wherein the heteroaryl-groups are 5- or 6-membered having from 1 to 3 heteroatoms selected from N, O and S; wherein said phenyl and heteroaryl groups may be mono-, di- or trisubstituted with substituents selected from halogen, -OH, (C1-4)alkyl, O-(C1-4)alkyl, S-(C1-4)alkyl,-NH₂, -NH((C1-4)alkyl) and -N((C1-4)alkyl)₂, -CONH₂ and -CONH-(C1-4)alkyl;
R³ is PRT, H or (C1-6)alkyl;
L is independently selected from C or N, providing there are no more than three consecutive N, each optionally substituted with one or more A³;
L¹ is independently selected from C, O, S, or N, providing there are no more than three consecutive N, each optionally substituted with one or more A³;
Z is O, S, C or N, optionally substituted with A³;
Z^{2a} is H, (C1-10)alkyl, (C2-10)alkenyl, (C2-10)alkynyl, wherein any carbon atom may be replaced with a heteroatom selected from O, S or N, or Z^{2a} optionally forms a carbocyle or heterocycle with R¹, R², Q¹, or any A³;
Z^{2b} is H, (C1-6)alkyl, (C2-8)alkenyl, (C2-8)alkynyl;
Q¹ is (C1)alkyl, (C2-8)alkenyl, or (C2-8)alkynyl;
A³ is independently selected from PRT, H, -C(O), -C(O)OH, -(CH₂)ᵣ-, -C(O)O-, -NH-, cyano, alkyl, alkenyl, alkynyl, amino, amido, imido, imino, halogen, CF₃, CH₂CF₃, cycloalkyl, nitro, aryl, aralkyl, alkoxy, aryloxy, heterocycle, heteroaryl, -C(A²)₂, -C(A²)₂-C(O)A², -C(O)OA², -O(A²),-N(A²)₂, -S(A²), -CH₂P(O)(A²)(OA²), -CH₂P(O)(A²)(N(A²)₂), -CH₂P(O)(OA²)(OA²), -OCH₂P(O)(OA²)(OA²), -OCH₂P(O)(A²)(OA²), -OCH₂P(O)(A²)(N(A²)₂), -C(O)OCH₂P(O)(OA²)(OA²), -C(O)OCH₂P(O)(A²)(OA²), -C(O)OCH₂P(O)(A²)(N(A²)₂), -CH₂P(O)(OA²)(N(A²)₂), -OCH₂P(O)(OA²)(N(A²)₂), -C(O)OCH₂P(O)(OA²)(N(A²)₂), -CH₂P(O)(N(A²)₂)(N(A²)₂), -C(O)OCH₂P(O)(N(A²)₂)(N(A²)₂), -OCH₂P(O)(N(A²)₂)(N(A²)₂), -(CH₂)ₘ-heterocycle, -(CH₂)ₘC(O)Oalkyl, -O-(CH₂)ₘ-O-C(O)-Oalkyl, -O-(CH₂)ᵣ-O-C(O)-(CH₂)ₘ -alkyl, -(CH₂)ₘO-C(O)-O-alkyl, -(CH₂)ₘO-C(O)-O-cycloalkyl, -N(H)C(Me)C(O)O-alkyl, or alkoxy arylsulfonamide, whereas each maybe optionally substituted with-R¹, -P(O)(OA²)(OA²), -P(O)(OA²)(N(A²)₂), -P(O)(A²)(OA²), -P(O)(A²)(N(A²)₂), or P(O)(N(A²)₂)(N(A²)₂), halogen, alkyl, alkenyl, alkynyl, aryl, carbocycle, heterocycle, aralkyl, aryl sulfonamide, aryl alkylsulfonamide, aryloxy sulfonamide, aryloxy alkylsulfonamide, aryloxy arylsulfonamide, alkyl sulfonamide, alkyloxy sulfonamide, alkyloxy alkylsulfonamide, -(CH₂)ₘheterocycle, -(CH₂)ₘ-C(O)O-alkyl, -O(CH₂)ₘOC(O)Oalkyl, -O-(CH₂)ₘ-O-C(O)-(CH₂)ₘ-alkyl, -(CH₂)ₘ-O-C(O)-O-alkyl, -(CH₂)ₘ-O-C(O)-O-cycloalkyl, -N(H)C(CH₃)C(O)O-alkyl, or alkoxy arylsulfonamide, optionally substituted with R¹; or
A³ forms a carbocyclic or heterocyclic ring with any other A³ or Q¹;
A² is independently selected from H, alkyl, alkenyl, alkynyl, amino, amino acid, alkoxy, aryloxy, cyano, haloalkyl, cycloalkyl, aryl, heteroaryl, alkylsulfonamide, or arylsulfonamide, optionally substituted with A³;
PRT is selected from -CH₂OC(=O)R⁹ and CH₂OC(=O)R⁹ where R⁹ is C₁-C₆ alkyl, C₁-C₆ substitued alkyl, C₆-C₂₀ aryl or C₆-C₂₀ substitued aryl;
m is 0 to 6;
r is 1 to 2; and
q is 1 to 10.

### Linking Groups and Linkers

The invention provides conjugates that comprise an HCV inhibiting compound that is optionally linked to one or more phosphonate groups either directly (e.g. through a covalent bond) or through a linking group (i.e. a linker). The nature of the linker is not critical provided it does not interfere with the ability of the phosphonate containing compound to function as a therapeutic agent. The phosphonate or the linker can be linked to the compound (*e.g*. a compound of formula A) at any synthetically feasible position on the compound by removing a hydrogen or any portion of the compound to provide an open valence for attachment of the phosphonate or the linker.

In one embodiment of the invention the linking group or linker (which can be designated "L") can include all or a portions of the group A⁰, A¹, A², or W³ described herein.

In another embodiment of the invention the linking group or linker has a molecular weight of from about 20 daltons to about 400 daltons.

In another embodiment of the invention the linking group or linker has a length of about 5 angstroms to about 300 angstroms.

In another embodiment of the invention the linking group or linker separates the DRUG and a P(=Y¹) residue by about 5 angstroms to about 200 angstroms, inclusive, in length.

In another embodiment of the invention the linking group or linker is a divalent, branched or unbranched, saturated or unsaturated, hydrocarbon chain, having from 2 to 25 carbon atoms, wherein one or more (*e.g*. 1, 2, 3, or 4) of the carbon atoms is optionally replaced by (-O-), and wherein the chain is optionally substituted on carbon with one or more (*e.g*. 1, 2, 3, or 4) substituents selected from (C₁-C₆)alkoxy, (C₃-C₆)cycloalkyl, (C₁-C₆)alkanoyl, (C₁-C₆)alkanoyloxy, (C₁-C₆)alkoxycarbonyl, (C₁-C₆)alkylthio, azido, cyano, nitro, halo, hydroxy, oxo (=O), carboxy, aryl, aryloxy, heteroaryl, and heteroaryloxy.

In another embodiment of the invention the linking group or linker is of the formula W-A wherein A is (C₁-C₂₄)alkyl, (C₂-C₂₄)alkenyl, (C₂-C₂₄)alkynyl, (C₃-C₈)cycloalkyl, (C₆-C₁₀)aryl or a combination thereof, wherein W is -N(R)C(=O)-,-C(=O)N(R)-; -OC(=O)-, -C(=O)O-, -O-, -S-, -S(O)-, -S(O)₂-, -N(R)-, -C(=O)-, or a direct bond; wherein each R is independently H or (C₁-C₆)alkyl.

In another.embodiment of the invention the linking group or linker is a divalent radical formed from a peptide.

In another embodiment of the invention the linking group or linker is a divalent radical formed from an amino acid.

In another embodiment of the invention the linking group or linker is a divalent radical formed from poly-L-glutamic acid, poly-L-aspartic acid, poly-L-histidine, poly-L-ornithine, poly-L-serine, poly-L-threonine, poly-L-tyrosine, poly-L-leucine, poly-L-lysine-L-phenylalanine, poly-L-lysine or poly-L-lysine-L-tyrosine.

In another embodiment of the invention the linking group or linker is of the formula W-(CH₂)ₙ wherein, n is between about 1 and about 10; and W is -N(R)C(=O)-, -C(=O)N(R)-, -OC(=O)-, -C(=O)O-, -O-, -S-, -S(O)-, -S(O)₂-, -C(=O)-, -N(R)-, or a direct bond; wherein each R is independently H or (C₁-C₆)alkyl.

In another embodiment of the invention the linking group or linker is methylene, ethylene, or propylene.

In another embodiment of the invention the linking group or linker is attached to the phosphonate group through a carbon atom of the linker.

### Intracellular Targeting

The phosphonate group of the compounds of the invention may cleave *in vivo* in stages after they have reached the desired site of action, *i.e*. inside a cell. One mechanism of action inside a cell may entail a first cleavage, *e.g*. by esterase, to provide a negatively-charged "locked-in" intermediate. Cleavage of a terminal ester grouping in a compound of the invention thus affords an unstable intermediate which releases a negatively charged "locked in" intermediate.

After passage inside a cell, intracellular enzymatic cleavage or modification of the phosphonate or prodrug compound may result in an intracellular accumulation of the cleaved or modified compound by a "trapping" mechanism. The cleaved or modified compound may then be "locked-in" the cell by a significant change in charge, polarity, or other physical property change which decreases the rate at which the cleaved or modified compound can exit the cell, relative to the rate at which it entered as the phosphonate prodrug. Other mechanisms by which a therapeutic effect are achieved may be operative as well. Enzymes which are capable of an enzymatic activation mechanism with the phosphonate prodrug compounds of the invention include, but are not limited to, amidases, esterases, microbial enzymes, phospholipases, cholinesterases, and phosphatases.

From the foregoing, it will be apparent that many different drugs can be derivatized in accord with the present invention. Numerous such drugs are specifically mentioned herein. However, it should be understood that the discussion of drug families and their specific members for derivatization according to this invention is not intended to be exhaustive, but merely illustrative.

### HCV-Inhibitory Compounds

The compounds of the invention include those with HCV-inhibitory activity. The compounds of the inventions optionally bear one or more (*e.g*. 1, 2, 3, or 4) phosphonate groups, which may be a prodrug moiety.

The term "HCV-inhibitory compound" includes those compounds that inhibit HCV.

Typically, compounds of the invention have a molecular weight of from about 400 amu to about 10,000 amu; in a specific embodiment of the invention, compounds have a molecular weight of less than about 5000 amu; in another specific embodiment of the invention, compounds have a molecular weight of less than about 2500 amu; in another specific embodiment of,the invention, compounds have a molecular weight of less than about 1000 amu; in another specific embodiment of the invention, compounds have a molecular weight of less than about 800 amu; in another specific embodiment of the invention, compounds have a molecular weight of less than about 600 amu; and in another specific embodiment of the invention, compounds have a molecular weight of less than about 600 amu and a molecular weight of greater than about 400 amu.

The compounds of the invention also typically have a logD(polarity) less than about 5. In one embodiment the invention provides compounds having a logD less than about 4; in another one embodiment the invention provides compounds having a logD less than about 3; in another one embodiment the invention provides compounds having a logD greater than about -5; in another one embodiment the invention provides compounds having a logD greater than about -3; and in another one embodiment the invention provides compounds having a logD greater than about 0 and less than about 3.

Selected substituents within the compounds of the invention are present to a recursive degree. In this context, "recursive substituent" means that a substituent may recite another instance of itself. Because of the recursive nature of such substituents, theoretically, a large number may be present in any given embodiment. For example, R^{x} contains a R^{y} substituent. R^{y} can be R², which in turn can be R³. If R³ is selected to be R^{3c}, then a second instance of R^{x} can be selected. One of ordinary skill in the art of medicinal chemistry understands that the total number of such substituents is reasonably limited by the desired properties of the compound intended. Such properties include, by of example and not limitation, physical properties such as molecular weight, solubility or log P, application properties such as activity against the intended target, and practical properties such as ease of synthesis.

By way of example and not limitation, W³, R^{y} and R³ are all recursive substituents in certain embodiments. Typically, each of these may independently occur 20, 19, 18, 17, 16, 15, 14, 13, 12, 11, 10, 9, 8, 7, 6, 5, 4, 3, 2, 1, or 0, times in a given embodiment. More typically, each of these may independently occur 12 or fewer times in a given embodiment. More typically yet, W³ will occur 0 to 8 times, R^{y} will occur 0 to 6 times and R³ will occur 0 to 10 times in a given embodiment. Even more typically, W³ will occur 0 to 6 times, R^{y} will occur 0 to 4 times and R³ will occur 0 to 8 times in a given embodiment.

Recursive substituents are an intended aspect of the invention. One of ordinary skill in the art of medicinal chemistry understands the versatility of such substituents. To the degree that recursive substituents are present in an embodiment of the invention, the total number will be determined as set forth above.

Whenever a compound described herein is substituted with more than one of the same designated group, *e.g*., "R¹" or "R^{6a}", then it will be understood that the groups may be the same or different, *i.e*., each group is independently selected. Wavy lines indicate the site of covalent bond attachments to the adjoining groups, moieties, or atoms.

In one embodiment of the invention, the compound is in an isolated and purified form. Generally, the term "isolated and purified" means that the compound is substantially free from biological materials (e.g. blood, tissue, cells, etc.). In one specific embodiment of the invention, the term means that the compound or conjugate of the invention is at least about 50 wt.% free from biological materials; in another specific embodiment, the term means that the compound or conjugate of the invention is at least about 75 wt.% free from biological materials; in another specific embodiment, the term means that the compound or conjugate of the invention is at least about 90 wt.% free from biological materials; in another specific embodiment, the term means that the compound or conjugate of the invention is at least about 98 wt.% free from biological materials; and in another embodiment, the term means that the compound or conjugate of the invention is at least about 99 wt.% free from biological materials. In another specific embodiment, the invention provides a compound or conjugate of the invention that has been synthetically prepared (e.g., *ex vivo*).

### Cellular Accumulation

In one embodiment, the invention is provides compounds capable of accumulating in human PBMC (peripheral blood mononuclear cells). PBMC refer to blood cells having round lymphocytes and monocytes. Physiologically, PBMC are critical components of the mechanism against infection. PBMC may be isolated from heparinized whole blood of normal healthy donors or buffy coats, by standard density gradient centrifugation and harvested from the interface, washed (*e.g*. phosphate-buffered saline) and stored in freezing medium. PBMC may be cultured in multi-well plates. At various times of culture, supernatant may be either removed for assessment, or cells may be harvested and analyzed (Smith R. etal (2003) Blood 102(7):2532-2540). The compounds of this embodiment may further comprise a phosphonate or phosphonate prodrug. More typically, the phosphonate or phosphonate prodrug can have the structure A³ as described herein.

Typically, compounds of the invention demonstrate improved intracellular half-life of the compounds or intracellular metabolites of the compounds in human PBMC when compared to analogs of the compounds not,having the phosphonate or phosphonate prodrug. Typically, the half-life is improved by at least about 50%, more typically at least in the range 50-100%, still more typically at least about 100%, more typically yet greater than about 100%.

In one embodiment of the invention the intracellular half-life of a metabolite of the compound in human PBMCs is improved when compared to an analog of the compound not having the phosphonate or phosphonate prodrug. In such embodiments, the metabolite may be generated intracellularly, *e.g*. generated within human PBMC. The metabolite may be a product of the cleavage of a phosphonate prodrug within human PBMCs. The phosphonate prodrug may be cleaved to form a metabolite having at least one negative charge at physiological pH. The phosphonate prodrug may be enzymatically cleaved within human PBMC to form a phosphonate having at least one active hydrogen atom of the form P-OH.

### Stereoisomers

The compounds of the invention may have chiral centers, *e.g*., chiral carbon or phosphorus atoms. The compounds of the invention thus include racemic mixtures of all stereoisomers, including enantiomers, diastereomers, and atropisomers. In addition, the compounds of the invention include enriched or resolved optical isomers at any or all asymmetric, chiral atoms. In other words, the chiral centers apparent from the depictions are provided as the chiral isomers or racemic mixtures. Both racemic and diastereomeric mixtures, as well as the individual optical isomers isolated or synthesized, substantially free of their enantiomeric or diastereomeric partners, are all within the scope of the invention. The racemic mixtures are separated into their individual, substantially optically pure isomers through well-known techniques such as, for example, the separation of diastereomeric salts formed with optically active adjuncts, *e.g*., acids or bases followed by conversion back to the optically active substances. In most instances, the desired optical isomer is synthesized by means of stereospecific reactions, beginning with the appropriate stereoisomer of the desired starting material.

The compounds of the invention can also exist as tautomeric isomers in certain cases. All though only one delocalized resonance structure may be depicted, all such forms are contemplated within the scope of the invention. For example, ene-amine tautomers can exist for purine, pyrimidine, imidazole, guanidine, amidine, and tetrazole systems and all their possible tautomeric forms are within the scope of the invention.

### Salts and Hydrates

The compositions of this invention optionally comprise salts of the compounds herein, especially pharmaceutically acceptable non-toxic salts containing, for example, Na⁺, Li⁺, K⁺, Ca⁺² and Mg⁺². Such salts may include those derived by combination of appropriate cations such as alkali and alkaline earth metal ions or ammonium and quaternary amino ions with an acid anion moiety, typically a carboxylic acid: Monovalent salts are preferred if a water soluble salt is desired.

Metal salts typically are prepared by reacting the metal hydroxide with a compound of this invention. Examples of metal salts which are prepared in this way are salts containing Li⁺, Na⁺, and K⁺. A less soluble metal salt can be precipitated from the solution of a more soluble salt by addition of the suitable metal compound.

In addition, salts may be formed from acid addition of certain organic and inorganic acids, *e.g*., HCl, HBr, H₂SO₄, H₃PO₄ or organic sulfonic acids, to basic centers, typically amines, or to acidic groups. Finally, it is to be understood that the compositions herein comprise compounds of the invention in their un-ionized, as well as zwitterionic form, and combinations with stoichiometric amounts of water as in hydrates.

Also included within the scope of this invention are the salts of the parental compounds with one or more amino acids. Any of the amino acids described above are suitable, especially the naturally-occurring amino acids found as protein components, although the amino acid typically is one bearing a side chain with a basic or acidic group, *e.g*., lysine, arginine or glutamic acid, or a neutral group such as glycine, serine, threonine, alanine, isoleucine, or leucine.

### Methods of Inhibition of HCV

Another aspect of the invention relates to methods of inhibiting the activity of HCV comprising the step of treating a sample suspected of containing HCV with a composition of the invention.

Compositions of the invention may act as inhibitors of HCV, as intermediates for such inhibitors or have other utilities as described below. The inhibitors will generally bind to locations on the surface or in a cavity of the liver. Compositions binding in the liver may bind with varying degrees of reversibility. Those compounds binding substantially irreversibly are ideal candidates for use in this method of the invention. Once labeled, the substantially irreversibly binding compositions are useful as probes for the detection of HCV. Accordingly, the invention relates to methods of detecting NS3 in a sample suspected of containing HCV comprising the steps of: treating a sample suspected of containing HCV with a composition comprising a compound of the invention bound to a label; and observing the effect of the sample on the activity of the label. Suitable labels are well known in the diagnostics field and include stable free radicals, fluorophores, radioisotopes, enzymes, chemiluminescent groups and chromogens. The compounds herein are labeled in conventional fashion using functional groups such as hydroxyl or amino.

Within the context of the invention samples suspected of containing HCV include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing HCV. Samples can be contained in any medium including water and organic solvent/water mixtures. Samples include living organisms such as humans, and man made materials such as cell cultures.

The treating step of the invention comprises adding the composition of the invention to the sample or it comprises adding a precursor of the composition to the sample. The addition step comprises any method of administration as described above.

If desired, the activity of HCV after application of the composition can be observed by any method including direct and indirect methods of detecting HCV activity. Quantitative, qualitative, and semiquantitative methods of determining HCV activity are all contemplated. Typically one of the screening methods described above are applied, however, any other method such as observation of the physiological properties of a living organism are also applicable.

Many organisms contain HCV. The compounds of this invention are useful in the treatment or prophylaxis of conditions associated with HCV activation in animals or in man.

However, in screening compounds capable of inhibiting HCV it should be kept in mind that the results of enzyme assays may not correlate with cell culture assays. Thus, a cell based assay should be the primary screening tool.

### Screens for HCV Inhibitors

Compositions of the invention are screened for inhibitory activity against HCV by any of the conventional techniques for evaluating enzyme activity. Within the context of the invention, typically compositions are first screened for inhibition of HCV *in vitro* and compositions showing inhibitory activity are then screened for activity *in vivo.* Compositions having *in vitro* Ki (inhibitory constants) of less then about 5 X 10⁻⁶ M, typically less than about 1 X 10⁻⁷ M and preferably less than about 5 X 10⁻⁸ M are preferred for *in vivo* use.

Useful *in vitro* screens have been described in detail.

### Pharmaceutical Formulations

The compounds of this invention are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as those set forth in the Handbook of Pharmaceutical Excipients (1986). Excipients include ascorbic acid and other antioxidants, chelating agents such as EDTA, carbohydrates such as dextrin, hydroxyalkylcellulose, hydroxyalkylmethylcellulose, stearic acid and the like. The pH of the formulations ranges from about 3 to about 11, but is ordinarily about 7 to 10.

While it is possible for the active ingredients to be administered alone it may be preferable to present them as pharmaceutical formulations. The formulations, both for veterinary and for human use, of the invention comprise at least one active ingredient, as above defined, together with one or more acceptable carriers therefor and optionally other therapeutic ingredients. The carrier(s) must be "acceptable" in the sense ot being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carner which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet is made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

For administration to the eye or other external tissues e.g., mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc.), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, *i.e.* an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the invention include Tween^{®} 60, Span^{®} 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils are used.

Pharmaceutical formulations according to the present invention comprise one or more compounds of the invention together with one or more pharmaceutically acceptable carriers or excipients and optionally other therapeutic agents. Pharmaceutical formulations containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, lactose monohydrate, croscarmellose sodium, povidone, calcium or sodium phosphate; granulating and disintegrating agents, such as maize starch, or alginic acid; binding agents, such as cellulose, microcrystalline cellulose, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 µg of the active ingredient per inilliliter of solution in order that infusion of a suitable volume at a rate of about 30 mL/hr can occur.

Formulations suitable for administration to the eye include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration may be prepared according to conventional methods and may be delivered with other therapeutic agents such as compounds heretofore used in the treatment or prophylaxis of conditions associated with HCV activity.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulations of this invention may include other agents conventional in the art having regard to the type of formulation in question, for example those suitable for oral administration may include flavoring agents.

The invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds of the invention can also be formulated to provide controlled release of the active ingredient to allow less frequent dosing or to improve the pharmacokinetic or toxicity profile of the active ingredient. Accordingly, the invention also provided compositions comprising one or more compounds of the invention formulated for sustained or controlled release.

Effective dose of active ingredient depends at least on the nature of the condition being treated, toxicity, whether the compound is being used prophylactically (lower doses), the method of delivery, and the pharmaceutical formulation, and will be determined by the clinician using conventional dose escalation studies. It can be expected to be from about 0.0001 to about 100 mg/kg body weight per day. Typically, from about 0.01 to about 10 mg/kg body weight per day. More typically, from about .01 to about 5 mg/kg body weight per day. More typically, from about .05 to about 0.5 mg/kg body weight per day. For example, the daily candidate dose for an adult human of approximately 70 kg body weight will range from 1 mg to 1000 mg, preferably between 5 mg and 500 mg, and may take the form of single or multiple doses.

### Routes of Administration

One or more compounds of the invention (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the recipient. An advantage of the compounds of this invention is that they are orally bioavailable and can be dosed orally.

### Combination Therapy

Active ingredients of the invention are also used in combination with other active ingredients. Such combinations are selected based on the condition to be treated, cross-reactivities of ingredients and pharmaco-properties of the combination.

It is also possible to combine any compound of the invention with one or more other active ingredients in a unitary dosage form for simultaneous or sequential administration to a patient. The combination therapy may be administered as a simultaneous or sequential regimen. When administered sequentially, the combination may be administered in two or more administrations.

The combination therapy may provide "synergy" and "synergistic effect", *i.e.* the effect achieved when the active ingredients used together is greater than the sum of the effects that results from using the compounds separately. A synergistic effect may be attained when the active ingredients are: (1) co-formulated and administered or delivered simultaneously in a combined formulation; (2) delivered by alternation or in parallel as separate formulations; or (3) by some other regimen. When delivered in alternation therapy, a synergistic effect may be attained when the compounds are administered or delivered sequentially, *e.g*., in separate tablets, pills or capsules, or by different injections in separate syringes. In general, during alternation therapy, an effective dosage of each active ingredient is administered sequentially, *i.e*. serially, whereas in combination therapy, effective dosages of two or more active ingredients are administered together.

### Metabolites of the Compounds of the Invention

Also falling within the scope of this invention are the *in vivo* metabolic products of the compounds described herein. Such products may result for example from the oxidation, reduction, hydrolysis, amidation, esterification and the like of the administered compound, primarily due to enzymatic processes. Accordingly, the invention includes compounds produced by a process comprising contacting a compound of this invention with a mammal for a period of time sufficient to yield a metabolic product thereof. Such products typically are identified by preparing a radiolabelled (*e.g*., C¹⁴ or H³) compound of the invention, administering it parenterally in a detectable dose (*e.g*., greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, *e.g*., by MS or NMR analysis. In general, analysis of metabolites is done in the same way as conventional drug metabolism studies well-known to those skilled in the art. The conversion products, so long as they are not otherwise found *in vivo,* are useful in diagnostic assays for therapeutic dosing of the compounds of the invention even if they possess no HCV - inhibitory activity of their own.

Recipes and methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37 °C. Simply because the compounds are stable to the gastrointestinal tract does not mean that they cannot be hydrolyzed *in vivo.* The phosphonate prodrugs of the invention typically will be stable in the digestive system but are substantially hydrolyzed to the parental drug in the digestive lumen, liver or other metabolic organ, or within cells in general.

### Exemplary Methods of Making the Compounds of the Invention.

The invention also relates to methods of making the compositions of the invention. The compositions are prepared by any of the applicable techniques of organic synthesis. Many such techniques are well known in the art. However, many of the known techniques are elaborated in Compendium of Organic Synthetic Methods (John Wiley & Sons, New York), Vol. 1, Ian T. Harrison and Shuyen Harrison, 1971; Vol. 2, Ian T. Harrison and Shuyen Harrison, 1974; Vol. 3, Louis S. Hegedus and Leroy Wade, 1977; Vol. 4, Leroy G. Wade, jr., 1980; Vol. 5, Leroy G. Wade, Jr., 1984; and Vol. 6, Michael B. Smith; as well as March, J., Advanced Organic Chemistry, Third Edition, (John Wiley & Sons, New York, 1985), Comprehensive Organic Synthesis. Selectivity, Strategy & Efficiency in Modern Organic Chemistry. In 9 Volumes, Barry M. Trost, Editor-in-Chief (Pergamon Press, New York, 1993 printing).

A number of exemplary methods for the preparation of the compositions of the invention are provided below. These methods are intended to illustrate the nature of such preparations and are not intended to limit the scope of applicable methods.

Generally, the reaction conditions such as temperature, reaction time, solvents, work-up procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20 °C), although for metal hydride reductions frequently the temperature is reduced to 0 °C to -100 °C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0 °C to -100 °C) are also common. Solvents can be either protic (common in equilibrating reactions) or aprotic (common in kinetically controlled reactions).

Standard synthetic techniques such as azeotropic removal of reaction byproducts and use of anhydrous reaction conditions (e.g., inert gas environments) are common in the art and will be applied when applicable.

### Schemes and Examples

General aspects of these exemplary methods are described below and in the Examples. Each of the products of the following processes is optionally separated, isolated, and/or purified prior to its use in subsequent processes.

Generally, the reaction conditions such as temperature, reaction time, solvents, work-up procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20 °C), although for metal hydride reductions frequently the temperature is reduced to 0 °C to - 100 °C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0 °C to -100 °C) are also common. Solvents can be either protic (common in equilibrating reactions) or aprotic (common in kinetically controlled reactions).

Standard synthetic techniques such as azeotropic removal of reaction byproducts and use of anhydrous reaction conditions (*e.g*., inert gas environments) are common in the art and will be applied when applicable.

The terms "treated", "treating", "treatment", and the like, when used in connection with a chemical synthetic operation, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two. For example, treating indicates the reasonable and usual manner in which organic chemicals are allowed to react. Normal concentrations (0.01M to 10M, typically 0.1M to 1M), temperatures (-100 °C to 250 °C, typically -78 °C to 150 °C, more typically -78 °C to 100 °C, still more typically 0 °C to 100 °C), reaction vessels (typically glass, plastic, metal), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic synthesis selects conditions and apparatus reasonably expected to successfully carry out the chemical reactions of the described processes based on the knowledge in the art.

Modifications of each of the exemplary schemes and in the examples (hereafter "exemplary schemes") leads to various analogs of the specific exemplary materials produce. The above-cited citations describing suitable methods of organic synthesis are applicable to such modifications.

In each of the exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

A single stereoisomer, *e.g.*, an enantiomer, substantially free of its stereoisomer may be obtained by resolution of the racemic mixture using a method such as formation of diastereomers using optically active resolving agents (Stereochemistry of Carbon Compounds, (1962) by E. L. Eliel, McGraw Hill; Lochmuller, C. H., (1975) J. Chromatogr., 113:(3) 283-302). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions.

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form,a diastereomeric pair (Eliel, E. and Wilen, S. (1994) Stereochemistry of Organic Compounds, John Wiley & Sons, Inc., p. 322). Diastereomeric compounds can be formed by reacting asymmetric compounds with enantiomerically pure chiral derivatizing reagents, such as menthyl derivatives, followed by separation of the diastereomers and hydrolysis to yield the free, enantiomerically enriched xanthene. A method of determining optical purity involves making chiral esters, such as a menthyl ester, *e.g*., (-) menthyl chloroformate in the presence of base, or Mosher ester, α-methoxy-α-(trifluoromethyl)phenyl acetate (Jacob III. (1982) J. Org. Chem. 47:4165), of the racemic mixture, and analyzing the NMR spectrum for the presence of the two atropisomeric diastereomers. Stable diastereomers of atropisomeric compounds can be separated and isolated by normal- and reverse-phase chromatography following methods for separation of atropisomeric naphthyl-isoquinolines (Hoye, T., WO 96/15111). By method (3), a racemic mixture of two enantiomers can be separated by chromatography using a chiral stationary phase (Chiral Liquid Chromatography (1989) W. J. Lough, Ed. Chapman and Hall, New York; Okamoto, (1990) J. of Chromatogr. 513:375-378). Enriched or purified enantiomers can be distinguished by methods used to distinguish other chiral molecules with asymmetric carbon atoms, such as optical rotation and circular dichroism.

### Examples General Section

A number of exemplary methods for the preparation of compounds of the invention are provided herein, for example, in the Examples hereinbelow. These methods are intended to illustrate the nature of such preparations are not intended to limit the scope of applicable methods. Certain compounds of the invention can be used as intermediates for the preparation of other compounds of the invention. For example, the interconversion of various phosphonate compounds of the invention is illustrated below.

### INTERCONVERSIONS OF THE PHOSPHONATES R-LINK-P(O)(OR¹)₂, R-LINK-P(O)(OR¹)(OH) AND R-LINK-P(O)(OH)₂.

The following schemes **32-38** describe the preparation of phosphonate esters of the general structure R-link-P(O)(OR¹)₂, in which the groups R¹ may be the same or different. The R¹ groups attached to a phosphonate ester, or to precursors thereto, may be changed using established chemical transformations. The interconversion reactions of phosphonates are illustrated in Scheme **S32.** The group R in Scheme **32** represents the substructure, *i.e*. the drug "scaffold, to which the substituent link-P(O)(OR¹)₂ is attached, either in the compounds of the invention, or in precursors thereto. At the point in the synthetic route of conducting a phosphonate interconversion, certain functional groups in R may be protected. The methods employed for a given phosphonate transformation depend on the nature of the substituent R¹, and of the substrate to which the phosphonate group is attached. The preparation and hydrolysis of phosphonate esters is described in Organic Phosphorus Compounds, G. M. Kosolapoff, L. Maeir, eds, Wiley, 1976, p. 9ff.

In general, synthesis of phosphonate esters is achieved by coupling a nucleophile amine or alcohol with the corresponding activated phosphonate electrophilic precursor. For example, chlorophosphonate addition on to 5'-hydroxy of nucleoside is a well known method for preparation of nucleoside phosphate monoesters. The activated precursor can be prepared by several well known methods. Chlorophosphonates useful for synthesis of the prodrugs are prepared from the substituted-1,3-propanediol (Wissner, et al, (1992) J. Med Chem. 35:1650). Chlorophosphonates are made by oxidation of the corresponding chlorophospholanes (Anderson, et al, (1984) J. Org. Chem. 49:1304) which are obtained by reaction of the substituted diol with phosphorus trichloride. Alternatively, the chlorophosphonate agent is made by treating substituted-1,3-diols with phosphorusoxychloride (Patois, et al, (1990) J. Chem. Soc. Perkin Trans. I, 1577). Chlorophosphonate species may also be generated in situ from corresponding cyclic phosphites (Silverburg, et al., (1996) Tetrahedron lett., 37:771-774), which in turn can be either made from chlorophospholane or phosphoramidate intermediate. Phosphoroflouridate intermediate prepared either from pyrophosphate or phosphoric acid may also act as precursor in preparation of cyclic prodrugs (Watanabe et al., (1988) Tetrahedron lett., 29:5763-66).

Phosphonate prodrugs of the present invention may also be prepared from the free acid by Mitsunobu reactions (Mitsunobu, (1981) Synthesis, 1; Campbell, (1992) J. Org. Chem. 57:6331), and other acid coupling reagents including, but not limited to, carbodiimides (Alexander, et al, (1994) Collect. Czech. Chem. Commun. 59:1853; Casara et al, (1992) Bioorg. Med. Chem. Lett. 2:145; Ohashi et al, (1988) Tetrahedron Lett., 29:1189), and benzotriazolyloxytris-(dimethylamino)phosphonium salts (Campagne et al (1993) Tetrahedron Lett. 34:6743).

Aryl halides undergo Ni⁺² catalyzed reaction with phosphite derivatives to give aryl phosphonate containing compounds (Balthazar, et al (1980) J. Org. Chem. 45:5425). Phosphonates may also be prepared from the chlorophosphonate in the presence of a palladium catalyst using aromatic triflates (Petrakis et al (1987) J. Am. Chem. Soc. 109:2831; Lu et al (1987) Synthesis 726). In another method, aryl phosphonate esters are prepared from aryl phosphates under anionic rearrangement conditions (Melvin (1981) Tetrahedron Lett. 22:3375; Casteel et al (1991) Synthesis, 691). N-Alkoxy aryl salts with alkali met al derivatives of cyclic alkyl phosphonate provide general synthesis for heteroaryl-2-phosphonate linkers (Redmore (1970) J. Org. Chem. 35:4114). These above mentioned methods can also be extended to compounds where the W⁵ group is a heterocycle. Cyclic-1,3-propanyl prodrugs of phosphonates are also synthesized from phosphonic diacids and substituted propane-1,3-diols using a coupling reagent such as 1,3-dicyclohexylcarbodiimide (DCC) in presence of a base (*e.g.*, pyridine). Other carbodiimide based coupling agents like 1,3-disopropylcarbodiimide or water soluble reagent, 1-(3-dimethylaminopropyl)-3-ethylcarbodiimide hydrochloride (EDCI) can also be utilized for the synthesis of cyclic phosphonate prodrugs.

The conversion of a phosphonate diester **S32.1** into the corresponding phosphonate monoester **S32.2** (Scheme **32,** Reaction **1**) is accomplished by a number of methods. For example, the ester **S32.1** in which R¹ is an aralkyl group such as benzyl, is converted into the monoester compound **S32.2** by reaction with a tertiary organic base such as diazabicyclooctane (DABCO) or quinuclidine, as described in J. Org. Chem. (1995) 60:2946. The reaction is performed in an inert hydrocarbon solvent such as toluene or xylene, at about 110 °C. The conversion of the diester **S32.1** in which R¹ is an aryl group such as phenyl, or an alkenyl group such as allyl, into the monoester **S32.2** is effected by treatment of the ester **S32.1** with a base such as aqueous sodium hydroxide in acetonitrile or lithium hydroxide in aqueous tetrahydrofuran. Phosphonate diesters **S32.1** in which one of the groups R¹ is aralkyl, such as benzyl, and the other is alkyl, is converted into the monoesters **S32.2** in which R¹ is alkyl by hydrogenation, for example using a palladium on carbon catalyst. Phosphonate diesters in which both of the groups R¹ are alkenyl, such as allyl, is converted into the monoester **S32.2** in which R¹ is alkenyl, by treatment with chlorotris(triphenylphosphine)rhodium (Wilkinson's catalyst) in aqueous ethanol at reflux, optionally in the presence of diazabicyclooctane, for example by using the procedure described in J. Org. Chem. (1973) 38:3224, for the cleavage of allyl carboxylates.

The conversion of a phosphonate diester **S32.1** or a phosphonate monoester **S32.2** into the corresponding phosphonic acid **S32.3** (Scheme **32,** Reactions **2** and **3**) can be effected by reaction of the diester or the monoester with trimethylsilyl bromide, as described in J. Chem. Soc., Chem. Comm., (1979) 739. The reaction is conducted in an inert solvent such as, for example, dichloromethane, optionally in the presence of a silylating agent such as bis(trimethylsilyl)trifluoroacetamide, at ambient temperature. A phosphonate monoester **S32.2** in which R¹ is aralkyl such as benzyl, is converted into the corresponding phosphonic acid **S32.3** by hydrogenation over a palladium catalyst, or by treatment with hydrogen chloride in an ethereal solvent such as dioxane. A phosphonate monoester **S32.2** in which R¹ is alkenyl such as, for example, allyl, is converted into the phosphonic acid **S32.3** by reaction with Wilkinson's catalyst in an aqueous organic solvent, for example in 15% aqueous acetonitrile, or in aqueous ethanol, for example using the procedure described in Helv. Chim. Acta. (1985) 68:618. Palladium catalyzed hydrogenolysis of phosphonate esters **S32.1** in which R¹ is benzyl is described in J. Org. Chem. (1959) 24:434. Platinum-catalyzed hydrogenolysis of phosphonate esters **S32.1** in which R¹ is phenyl is described in J. Am. Chem. Soc. (1956) 78:2336.

The conversion of a phosphonate monoester **S32.2** into a phosphonate diester **S32.1** (Scheme **32,** Reaction **4**) in which the newly introduced R¹ group is alkyl, aralkyl, haloalkyl such as chloroethyl, or aralkyl is effected by a number of reactions in which the substrate **S32.2** is reacted with a hydroxy compound R¹OH, in the presence of a coupling agent. Typically, the second phosphonate ester group is different than the first introduced phosphonate ester group, *i.e*. R¹ is followed by the introduction of R² where each of R¹ and R² is alkyl, aralkyl, haloalkyl such as chloroethyl, or aralkyl (Scheme **32,** Reaction **4a**) whereby **S32.2** is converted to **S32.1a.** Suitable coupling agents are those employed for the preparation of carboxylate esters, and include a carbodiimide such as dicyclohexylcarbodiimide, in which case the reaction is preferably conducted in a basic organic solvent such as pyridine, or (benzotriazol-1-yloxy)tripyrrolidinophosphonium hexafluorophosphate (PYBOP, Sigma), in which case the reaction is performed in a polar solvent such as dimethylformamide, in the presence of a tertiary organic base such as diisopropylethylamine, or Aldrithiol-2 (Aldrich) in which case the reaction is conducted in a basic solvent such as pyridine, in the presence of a triaryl phosphine such as triphenylphosphine. Alternatively, the conversion of the phosphonate monoester **S32.2** to the diester **S32.1** is effected by the use of the Mitsunobu reaction, as described above. The substrate is reacted with the hydroxy compound R¹OH, in the presence of diethyl azodicarboxylate and a triarylphosphine such as triphenyl phosphine. Alternatively, the phosphonate monoester **S32.2** is transformed into the phosphonate diester **S32.1,** in which the introduced R¹ group is alkenyl or aralkyl, by reaction of the monoester with the halide R¹Br, in which R¹ is as alkenyl or aralkyl. The alkylation reaction is conducted in a polar organic solvent such as dimethylformamide or acetonitrile, in the presence of a base such as cesium carbonate. Alternatively, the phosphonate monoester is transformed into the phosphonate diester in a two step procedure. In the first step, the phosphonate monoester **S32.2** is transformed into the chloro analog RP(O)(OR¹)Cl by reaction with thionyl chloride or oxalyl chloride and the like, as described in Organic Phosphorus Compounds, G. M. Kosolapoff, L. Maeir, eds, Wiley, 1976, p. 17, and the thus-obtained product RP(O)(OR¹)Cl is then reacted with the hydroxy compound R¹OH, in the presence of a base such as triethylamine, to afford the phosphonate diester **S32.1.**

A phosphonic acid R-link-P(O)(OH)₂ is transformed into a phosphonate monoester RP(O)(OR¹)(OH) (Scheme **32,** Reaction 5) by means of the methods described above of for the preparation of the phosphonate diester R-link-P(O)(OR¹)₂ **S32.1,** except that only one molar proportion of the component R¹OH or R¹Br is employed. Dialkyl phosphonates may be prepared according to the methods of: Quast et al (1974) Synthesis 490; Stowell et al (1990) Tetrahedron Lett. 3261; US 5663159.

A phosphonic acid R-link-P(O)(OH)₂ **S32.3** is transformed into a phosphonate diester R-link-P(O)(OR¹)₂ **S32.1** (Scheme **32,** Reaction **6**) by a coupling reaction with the hydroxy compound R¹OH, in the presence of a coupling agent such as Aldrithiol-2 (Aldrich) and triphenylphosphine. The reaction is conducted in a basic solvent such as pyridine. Alternatively, phosphonic acids **S32.3** are transformed into phosphonic esters **S32.1** in which R¹ is aryl, by means of a coupling reaction employing for example, dicyclohexylcarbodiimide in pyridine at ca 70 °C. Alternatively, phosphonic acids **S32.3** are transformed into phosphonic esters **S32.1** in which R¹ is alkenyl, by means of an alkylation reaction. The phosphonic acid is reacted with the alkenyl bromide R¹Br in a polar organic solvent such as acetonitrile solution at reflux temperature, the presence of a base such as cesium carbonate, to afford the phosphonic ester **S32.1.**

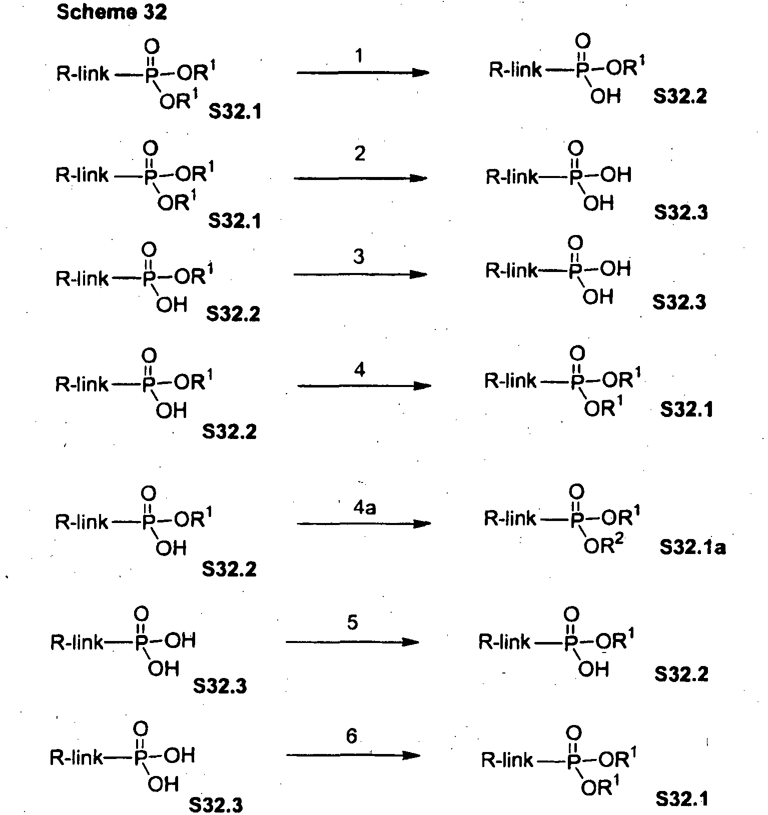

### Preparation of phosphonate carbamates.

Phosphonate esters may contain a carbamate linkage. The preparation of carbamates is described in Comprehensive Organic Functional Group Transformations, A. R. Katritzky, ed., Pergamon, 1995, Vol. 6, p. 416ff, and in Organic Functional Group Preparations, by S. R. Sandler and W. Karo, Academic Press, 1986, p. 260ff. The carbamoyl group may be formed by reaction of a hydroxy group according to the methods known in the art, including the teachings of Ellis, US 2002/0103378 A1 and Hajima, US 6018049.

Scheme **33** illustrates various methods by which the carbamate linkage is synthesized. As shown in Scheme **33,** in the general reaction generating carbamates, an alcohol **S33.1,** is converted into the activated derivative **S33.2** in which Lv is a leaving group such as halo, imidazolyl, benztriazolyl and the like, as described herein. The activated derivative **S33.2** is then reacted with an amine **S33.3,** to afford the carbamate product **S33.4.** Examples 1-7 in Scheme **33** depict methods by which the general reaction is effected. Examples **8-10** illustrate alternative methods for the preparation of carbamates.

Scheme **33,** Example **1** illustrates the preparation of carbamates employing a chloroformyl derivative of the alcohol **S33.5.** In this procedure, the alcohol **S33.5** is reacted with phosgene, in an inert solvent such as toluene, at about 0 °C, as described in Org. Syn. Coll. Vol. 3, 167, 1965, or with an equivalent reagent such as trichloromethoxy chloroformate, as described in Org. Syn. Coll. Vol. 6, 715, 1988, to afford the chloroformate **S33.6.** The latter compound is then reacted with the amine component **S33.3,** in the presence of an organic or inorganic base, to afford the carbamate **S33.7.** For example, the chloroformyl compound **S33.6** is reacted with the amine **S33.3** in a water-miscible solvent such as tetrahydrofuran, in the presence of aqueous sodium hydroxide, as described in Org. Syn. Coll. Vol. 3,167, 1965, to yield the carbamate **S33.7.** Alternatively, the reaction is performed in dichloromethane in the presence of an organic base such as diisopropylethylamine or dimethylaminopyridine.

Scheme **33,** Example **2** depicts the reaction of the chloroformate compound **S33.6** with imidazole to produce the imidazolide **S33.8.** The imidazolide product is then reacted with the amine **S33.3** to yield the carbamate **S33.7.** The preparation of the imidazolide is performed in an aprotic solvent such as dichloromethane at 0°, and the preparation of the carbamate is conducted in a similar solvent at ambient temperature, optionally in the presence of a base such as dimethylaminopyridine, as described in J. Med. Chem., 1989, 32, 357.

Scheme **33** Example **3,** depicts the reaction of the chloroformate **S33.6** with an activated hydroxyl compound R"OH, to yield the mixed carbonate ester **S33.10.** The reaction is conducted in an inert organic solvent such as ether or dichloromethane, in the presence of a base such as dicyclohexylamine or triethylamine. The hydroxyl component R"OH is selected from the group of compounds **S33.19 - S33.24** shown in Scheme **33,** and similar compounds. For example, if the component R"OH is hydroxybenztriazole **S33.19,** N-hydroxysuccinimide **S33.20,** or pentachlorophenol, **S33.21,** the mixed carbonate **S33.10** is obtained by the reaction of the chloroformate with the hydroxyl compound in an ethereal solvent in the presence of dicyclohexylamine, as described in Can. J. Chem., 1982, 60, 976. A similar reaction in which the component R"OH is pentafluorophenol **S33.22** or 2-hydroxypyridine **S33.23** is performed in an ethereal solvent in the presence of triethylamine, as described in Syn., 1986, 303, and Chem. Ber. 118, 468, 1985.

Scheme **33** Example **4** illustrates the preparation of carbamates in which an alkyloxycarbonylimidazole **S33.8** is employed. In this procedure, an alcohol **S33.5** is reacted with an equimolar amount of carbonyl diimidazole **S33.11** to prepare the intermediate **S33.8.** The reaction is conducted in an aprotic organic solvent such as dichloromethane or tetrahydrofuran. The acyloxyimidazole **S33.8** is then reacted with an equimolar amount of the amine R'NH₂ to afford the carbamate **S33.7.** The reaction is performed in an aprotic organic solvent such as dichloromethane, as described in Tet. Lett., 42, 2001, 5227, to afford the carbamate **S33.7.**

Scheme **33,** Example **5** illustrates the preparation of carbamates by means of an intermediate alkoxycarbonylbenztriazole **S33.13.** In this procedure, an alcohol ROH is reacted at ambient temperature with an equimolar amount of benztriazole carbonyl chloride **S33.12,** to afford the alkoxycarbonyl **product S33.13.** The reaction is performed in an organic solvent such as benzene or toluene, in the presence of a tertiary organic amine such as triethylamine, as described in Synthesis., 1977, 704. The product is then reacted with the amine R'NH₂ to afford the carbamate **S33.7.** The reaction is conducted in toluene or ethanol, at from ambient temperature to about 80 C as described in Synthesis., 1977, 704.

Scheme **33,** Example **6** illustrates the preparation of carbamates in which a carbonate (R"O)₂CO, **S33.14,** is reacted with an alcohol **S33.5** to afford the intermediate alkyloxycarbonyl intermediate **S33.15.** The latter reagent is then reacted with the amine R'NH₂ to afford the carbamate **S33.7.** The procedure in which the reagent **S33.15** is derived from hydroxybenztriazole **S33.19** is described in Synthesis, 1993, 908; the procedure in which the reagent **S33.15** is derived from N-hydroxysuccinimide **S33.20** is described in Tet. Lett., 1992, 2781; the procedure in which the reagent **S33.15** is derived from 2-hydroxypyridine **S33.23** is described in Tet. Lett., 1991, 4251; the procedure in which the reagent **S33.15** is derived from 4-nitrophenol **S33.24** is described in Synthesis. 1993, 103. The reaction between equimolar amounts of the alcohol ROH and the carbonate **S33.14** is conducted in an inert organic solvent at ambient temperature.

Scheme **33,** Example **7** illustrates the preparation of carbamates from alkoxycarbonyl azides **S33.16.** In this procedure, an alkyl chloroformate **S33.6** is reacted with an azide, for example sodium azide, to afford the alkoxycarbonyl azide **S33.16.** The latter compound is then reacted with an equimolar amount of the amine R'NH₂ to afford the carbamate **S33.7.** The reaction is conducted at ambient temperature in a polar aprotic solvent such as dimethylsulfoxide, for example as described in Synthesis., 1982, 404.

Scheme **33,** Example **8** illustrates the preparation of carbamates by means of the reaction between an alcohol ROH and the chloroformyl derivative of an amine **S33.17.** In this procedure, which is described in Synthetic Organic Chemistry R. B. Wagner, H. D. Zook, Wiley, 1953, p. 647, the reactants are combined at ambient temperature in an aprotic solvent such as acetonitrile, in the presence of a base such as triethylamine, to afford the carbamate **S33.7.**

Scheme **33,** Example **9** illustrates the preparation of carbamates by means of the reaction between an alcohol ROH and an isocyanate **S33.18.** In this procedure, which is described in Synthetic Organic Chemistry, R. B. Wagner, H. D. Zook, Wiley, 1953, p. 645, the reactants are combined at ambient temperature in an aprotic solvent such as ether or dichloromethane and the like, to afford the carbamate **S33.7.**

Scheme **33,** Example **10** illustrates the preparation of carbamates by means of the reaction between an alcohol ROH and an amine R'NH₂. In this procedure, which is described in Chem. Lett. 1972, 373, the reactants are combined at ambient temperature in an aprotic organic solvent such as tetrahydrofuran, in the presence of a tertiary base such as triethylamine, and selenium. Carbon monoxide is passed through the solution and the reaction proceeds to afford the carbamate **S33.7.**

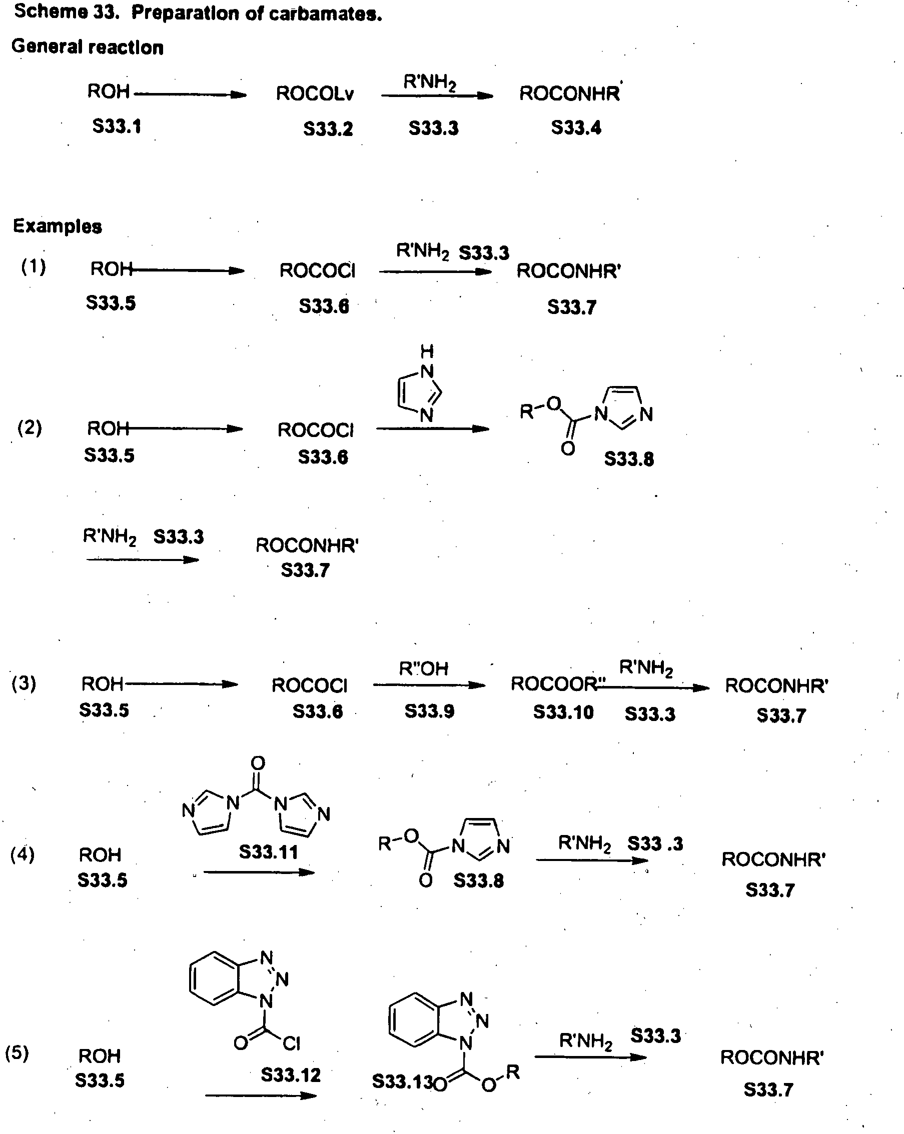

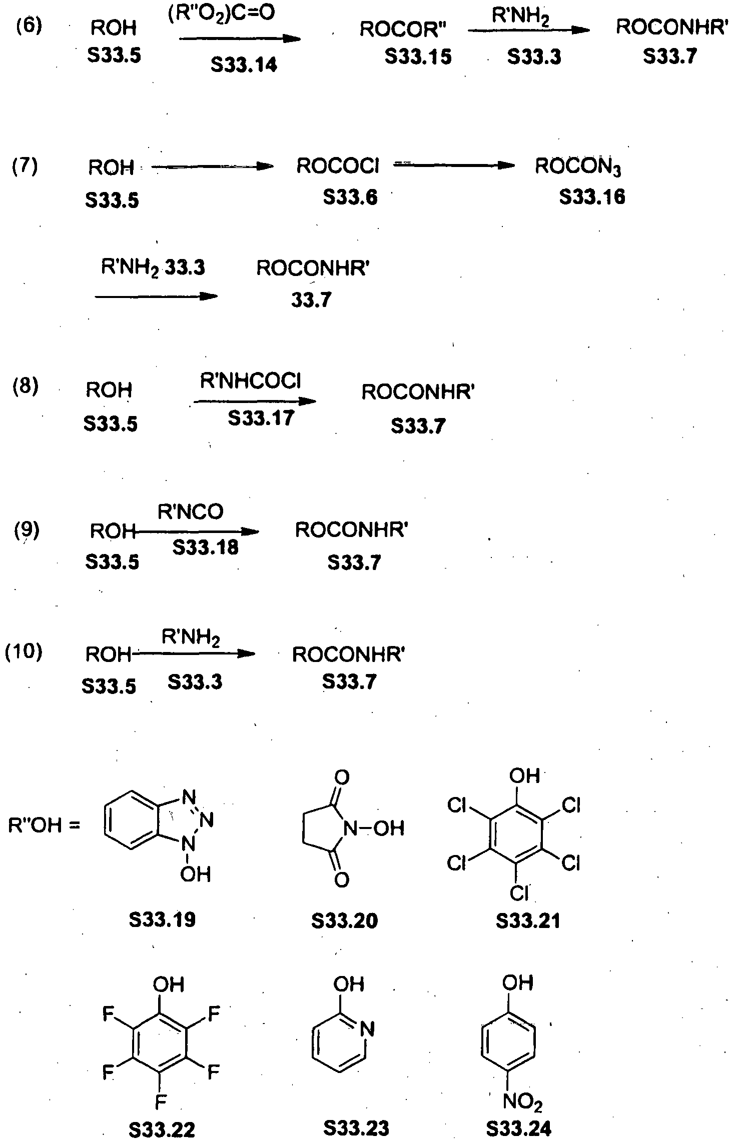

### PREPARATION OF CARBOALKOXY-SUBSTITUTED PHOSPHONATE BISAMIDATES, MONOAMIDATES, DIESTERS AND MONOESTERS.

A number of methods are available for the conversion of phosphonic acids into amidates and esters. In one group of methods, the phosphonic acid is either converted into an isolated activated intermediate such as a phosphoryl chloride, or the phosphonic acid is activated in situ for reaction with an amine or a hydroxy compound.

The conversion of phosphonic acids into phosphoryl chlorides is accomplished by reaction with thionyl chloride, for example as described in J. Gen. Chem. USSR, 1983, 53, 480, Zh. Obschei Khim., 1958, 28, 1063, or J. Org. Chem., 1994, 59, 6144, or by reaction with oxalyl chloride, as described in J. Am. Chem. Soc., 1994, 116, 3251, or J. Org. Chem., 1994, 59, 6144, or by reaction with phosphorus pentachloride, as described in J. Org. Chem., 2001, 66, 329, or in J. Med. Chem., 1995, 38, 1372. The resultant phosphoryl chlorides are then reacted with amines or hydroxy compounds in the presence of a base to afford the amidate or ester products.

Phosphonic acids are converted into activated imidazolyl derivatives by reaction with carbonyl diimidazole, as described in J. Chem. Soc., Chem. Comm. (1991) 312, or Nucleosides & Nucleotides (2000) 19:1885. Activated sulfonyloxy derivatives are obtained by the reaction of phosphonic acids with trichloromethylsulfonyl chloride or with triisopropylbenzenesulfonyl chloride, as described in Tet. Lett. (1996) 7857, or Bioorg. Med. Chem. Lett. (1998) 8:663. The activated sulfonyloxy derivatives are then reacted with amines or hydroxy compounds to afford amidates or esters.

Alternatively, the phosphonic acid and the amine or hydroxy reactant are combined in the presence of a diimide coupling agent. The preparation of phosphonic amidates and esters by means of coupling reactions in the presence of dicyclohexyl carbodiimide is described, for example, in J. Chem. Soc., Chem. Comm. (1991) 312 or Coll. Czech. Chem. Comm. (1987) 52:2792. The use of ethyl dimethylaminopropyl carbodiimide for activation and coupling of phosphonic acids is described in Tet. Lett., (2001) 42:8841, or Nucleosides & Nucleotides (2000) 19:1885.

A number of additional coupling reagents have been described for the preparation of amidates and esters from phosphonic acids. The agents include Aldrithiol-2, and PYBOP and BOP, as described in J. Org. Chem., 1995, 60, 5214, and J. Med. Chem. (1997) 40:3842, mesitylene-2-sulfonyl-3-nitro-1,2,4-triazole (MSNT), as described in J. Med. Chem. (1996) 39:4958, diphenylphosphoryl azide, as described in J. Org. Chem. (1984) 49:1158, 1-(2,4,6-triisopropylbenzenesulfonyl-3-nitro-1,2,4-triazole (TPSNT) as described in Bioorg. Med. Chem. Lett. (1998) 8:1013, bromotris(dimethylamino)phosphonium hexafluorophosphate (BroP), as described in Tet. Lett., (1996) 37:3997, 2-chloro-5,5-dimethyl-2-oxo-1,3,2-dioxaphosphinane, as described in Nucleosides Nucleotides 1995,14, 871, and diphenyl chlorophosphate, as described in J. Med. Chem., 1988, 31, 1305.

Phosphonic acids are converted into amidates and esters by means of the Mitsunobu reaction, in which the phosphonic acid and the amine or hydroxy reactant are combined in the presence of a triaryl phosphine and a dialkyl azodicarboxylate. The procedure is described in Org. Lett., 2001, 3, 643, or J. Med. Chem., 1997, 40, 3842.

Phosphonic esters are also obtained by the reaction between phosphonic acids and halo compounds, in the presence of a suitable base. The method is described, for example, in Anal. Chem., 1987, 59, 1056, or J. Chem. Soc. Perkin Trans., I, 1993, 19, 2303, or J. Med. Chem., 1995, 38, 1372, or Tet. Lett., 2002, 43, 1161.

Schemes **34-37** illustrate the conversion of phosphonate esters and phosphonic acids into carboalkoxy-substituted phosphonbisamidates (Scheme **34**), phosphonamidates (Scheme **35**), phosphonate monoesters (Scheme **36**) and phosphonate diesters, (Scheme **37**). Scheme 38 illustrates synthesis of gem-dialkyl amino phosphonate reagents.

Scheme **34** illustrates various methods for the conversion of phosphonate diesters **S34.1** into phosphonbisamidates **S34.5.** The diester **S34.1,** prepared as described previously, is hydrolyzed, either to the monoester **S34.2** or to the phosphonic acid **S34.6.** The methods employed for these transformations are described above. The monoester **S34.2** is converted into the monoamidate **S34.3** by reaction with an aminoester **S34.9,** in which the group R² is H or alkyl; the group R^{4b} is a divalent alkylene moiety such as, for example, CHCH₃, CHCH₂CH₃, CH(CH(CH₃)₂), CH(CH₂Ph), and the like, or a side chain group present in natural or modified aminoacids; and the group R^{5b} is C₁-C₁₂ alkyl, such as methyl, ethyl, propyl, isopropyl, or isobutyl; C₆-C₂₀ aryl, such as phenyl or substituted phenyl; or C₆-C₂₀ arylalkyl, such as benzyl or benzyhydryl. The reactants are combined in the presence of a coupling agent such as a carbodiimide, for example dicyclohexyl carbodiimide, as described in J. Am. Chem. Soc., (1957) 79:3575, optionally in the presence of an activating agent such as hydroxybenztriazole, to yield the amidate product **S34.3.** The amidate-forming reaction is also effected in the presence of coupling agents such as BOP, as described in J. Org. Chem. (1995) 60:5214, Aldrithiol, PYBOP and similar coupling agents used for the preparation of amides and esters. Alternatively, the reactants **S34.2** and **S34.9** are transformed into the monoamidate **S34.3** by means of a Mitsunobu reaction. The preparation of amidates by means of the Mitsunobu reaction is described in J. Med. Chem. (1995) 38:2742. Equimolar amounts of the reactants are combined in an inert solvent such as tetrahydrofuran in the presence of a triaryl phosphine and a dialkyl azodicarboxylate. The thus-obtained monoamidate ester **S34.3** is then transformed into amidate phosphonic acid **S34.4.** The conditions used for the hydrolysis reaction depend on the nature of the R¹ group, as described previously. The phosphonic acid amidate **S34.4** is then reacted with an aminoester **S34.9,** as described above, to yield the bisamidate product **S34.5,** in which the amino substituents are the same or different. Alternatively, the phosphonic acid **S34.6** may be treated with two different amino ester reagents simulataneously, *i.e.* **S34.9** where R², R^{4b} or R^{5b} are different. The resulting mixture of bisamidate products **S34.5** may then be separable, *e.g*. by chromatography.

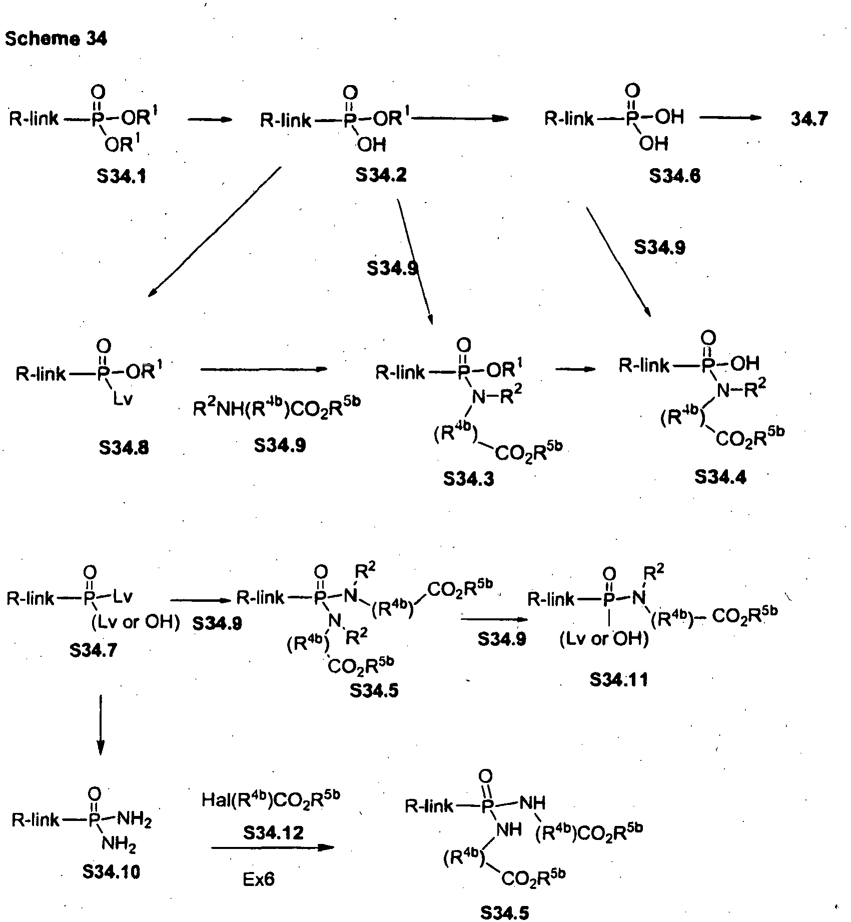

An example of this procedure is shown in Scheme **34,** Example **1.** In this procedure, a dibenzyl phosphonate **S34.14** is reacted with diazabicyclooctane (DABCO) in toluene at reflux, as described in J. Org. Chem., 1995, 60, 2946, to afford the monobenzyl phosphonate **S34.15.** The product is then reacted with equimolar amounts of ethyl alaninate **S34.16** and dicyclohexyl carbodiimide in pyridine, to yield the amidate product **S34.17.** The benzyl group is then removed, for example by hydrogenolysis over a palladium catalyst, to give the monoacid product **S34.18** which may be unstable according to J. Med. Chem. (1997) 40(23):3842. This compound **S34.18** is then reacted in a Mitsunobu reaction with ethyl leucinate **S34.19,** triphenyl phosphine and diethylazodicarboxylate, as described in J. Med. Chem., 1995, 38, 2742, to produce the bisamidate product **S34.20.**

Using the above procedures, but employing in place of ethyl leucinate **S34.19** or ethyl alaninate **S34.16,** different aminoesters **S34.9,** the corresponding products **S34.5** are obtained.

Alternatively, the phosphonic acid **S34.6** is converted into the bisamidate **S34.5** by use of the coupling reactions described above. The reaction is performed in one step, in which case the nitrogen-related substituents present in the product **S34.5** are the same, or in two steps, in which case the nitrogen-related substituents can be different.

An example of the method is shown in Scheme 34, Example 2. In this procedure, a phosphonic acid **S34.6** is reacted in pyridine solution with excess ethyl phenylalaninate **S34.21** and dicyclohexylcarbodiimide, for example as described in J. Chem. Soc., Chem. Comm., 1991, 1063, to give the bisamidate product **S34.22.**

Using the above procedures, but employing, in place of ethyl phenylalaninate, different aminoesters **S34.9,** the corresponding products **S34.5** are obtained.

As a further alternative, the phosphonic acid **S34.6** is converted into the mono or bis-activated derivative **S34.7,** in which Lv is a leaving group such as chloro, imidazolyl, triisopropylbenzenesulfonyloxy etc. The conversion of phosphonic acids into chlorides **S34.7** (Lv = Cl) is effected by reaction with thionyl chloride or oxalyl chloride and the like, as described in Organic Phosphorus Compounds, G. M. Kosolapoff, L. Maeir, eds, Wiley, 1976, p. 17. The conversion of phosphonic acids into monoimidazolides **S34.7** (Lv = imidazolyl) is described in J. Med. Chem., 2002, 45, 1284 and in J. Chem. Soc. Chem. Comm., 1991, 312. Alternatively, the phosphonic acid is activated by reaction with triisopropylbenzenesulfonyl chloride, as described in Nucleosides and Nucleotides, 2000, 10, 1885. The activated product is then reacted with the aminoester **S34.9,** in the presence of a base, to give the bisamidate **S34.5.** The reaction is performed in one step, in which case the nitrogen substituents present in the product S34.5 are the same, or in two steps, via the intermediate S34.11, in which case the nitrogen substituents can be different.

Examples of these methods are shown in Scheme 34, Examples 3 and 5. In the procedure illustrated in Scheme 34, Example 3, a phosphonic acid S34.6 is reacted with ten molar equivalents of thionyl chloride, as described in Zh. Obschei Khim., 1958, 28, 1063, to give the dichloro compound S34.23. The product is then reacted at reflux temperature in a polar aprotic solvent such as acetonitrile, and in the presence of a base such as triethylamine, with butyl serinate S34.24 to afford the bisamidate product S34.25.

Using the above procedures, but employing, in place of butyl serinate S34.24, different aminoesters **S34.9,** the corresponding products **S34.5** are obtained.

In the procedure illustrated in Scheme 34, Example 5, the phosphonic acid **S34.6** is reacted, as described in J. Chem. Soc. Chem. Comm., 1991, 312, with carbonyl diimidazole to give the imidazolide **S34.S32.** The product is then reacted in acetonitrile solution at ambient temperature, with one molar equivalent of ethyl alaninate **S34.33** to yield the monodisplacement product **S34.S34.** The latter compound is then reacted with carbonyl diimidazole to produce the activated intermediate **S34.35,** and the product is then reacted, under the same conditions, with ethyl N-methylalaninate **S34.33a** to give the bisamidate product **S34.36.**

Using the above procedures, but employing, in place of ethyl alaninate **S34.33** or ethyl N-methylalaninate **S34.33a,** different aminoesters **S34.9,** the corresponding products **S34.5** are obtained.

The intermediate monoamidate **S34.3** is also prepared from the monoester **S34.2** by first converting the monoester into the activated derivative **S34.8** in which Lv is a leaving group such as halo, imidazolyl etc, using the procedures described above. The product **S34.8** is then reacted with an aminoester **S34.9** in the presence of a base such as pyridine, to give an intermediate monoamidate product **S34.3.** The latter compound is then converted, by removal of the R¹ group and coupling of the product with the aminoester **S34.9,** as described above, into the bisamidate **S34.5.**

An example of this procedure, in which the phosphonic acid is activated by conversion to the chloro derivative **S34.26,** is shown in Scheme 34, Example 4. In this procedure, the phosphonic monobenzyl ester **S34.15** is reacted, in dichloromethane, with thionyl chloride, as described in Tet. Letters., 1994, 35, 4097, to afford the phosphoryl chloride **S34.26.** The product is then reacted in acetonitrile solution at ambient temperature with one molar equivalent of ethyl 3-amino-2-methylpropionate **S34.27** to yield the monoamidate product **S34.28.** The latter compound is hydrogenated in ethylacetate over a 5% palladium on carbon catalyst to produce the monoacid product **S34.29.** The product is subjected to a Mitsunobu coupling procedure, with equimolar amounts of butyl alaninate **S34.30,** triphenyl phosphine, diethylazodicarboxylate and triethylamine in tetrahydrofuran, to give the bisamidate product **S34.31.**

Using the above procedures, but employing, in place of ethyl 3-amino-2-methylpropionate **S34.27** or butyl alaninate **S34.30,** different aminoesters **S34.9,** the corresponding products **S34.5** are obtained.

The activated phosphonic acid derivative **S34.7** is also converted into the bisamidate **S34.5** via the diamino compound **S34.10**. The conversion of activated phosphonic acid derivatives such as phosphoryl chlorides into the corresponding amino analogs **S34.10,** by reaction with ammonia, is described in Organic Phosphorus Compounds, G. M. Kosolapoff, L. Maeir, eds, Wiley, 1976. The bisamino compound **S34.10** is then reacted at elevated temperature with a haloester **S34.12** (Hal = halogen, *i.e.* F, Cl, Br, I), in a polar organic solvent such as dimethylformamide, in the presence of a base such as 4, 4-dimethylaminopyridine (DMAP) or potassium carbonate, to yield the bisamidate **S34.5.** Alternatively, **S34.6** may be treated with two different amino ester reagents simulataneously, *i.e.* **S34.12** where R^{4b} or R^{5b} are different. The resulting mixture of bisamidate products **S34.5** may then be separable, *e.g.* by chromatography.

An example of this procedure is shown in Scheme 34, Example 6. In this method, a dichlorophosphonate **S34.23** is reacted with ammonia to afford the diamide **S34.37.** The reaction is performed in aqueous, aqueous alcoholic or alcoholic solution, at reflux temperature. The resulting diamino compound is then reacted with two molar equivalents of ethyl 2-bromo-3-methylbutyrate **S34.38,** in a polar organic solvent such as N-methylpyrrolidinone at ca. 150 °C, in the presence of a base such as potassium carbonate, and optionally in the presence of a catalytic amount of potassium iodide, to afford the bisamidate product **S34.39.**

Using the above procedures, but employing, in place of ethyl 2-bromo-3-methylbutyrate **S34.38,** different haloesters **S34.12** the corresponding products **S34.5** are obtained.

The procedures shown in Scheme 34 are also applicable to the preparation of bisamidates in which the aminoester moiety incorporates different functional groups. Scheme 34, Example 7 illustrates the preparation of bisamidates derived from tyrosine. In this procedure, the monoimidazolide **S34.32** is reacted with propyl tyrosinate **S34.40,** as described in Example 5, to yield the monoamidate **S34.41.** The product is reacted with carbonyl diimidazole to give the imidazolide **S34.42,** and this material is reacted with a further molar equivalent of propyl tyrosinate to produce the bisamidate product **S34.43.**

Using the above procedures, but employing, in place of propyl tyrosinate **S34.40,** different aminoesters **S34.9,** the corresponding products **S34.5** are obtained. The aminoesters employed in the two stages of the above procedure can be the same or different, so that bisamidates with the same or different amino substituents are prepared.

Scheme 35 illustrates methods for the preparation of phosphonate monoamidates.

In one procedure, a phosphonate monoester **S34.1** is converted, as described in Scheme 34, into the activated derivative **S34.8.** This compound is then reacted, as described above, with an aminoester **S34.9,** in the presence of a base, to afford the monoamidate product **S35.1.**

The procedure is illustrated in Scheme 35, Example 1. In this method, a monophenyl phosphonate **S35.7** is reacted with, for example, thionyl chloride, as described in J. Gen. Chem. USSR., 1983, 32, 367, to give the chloro product **S35.8**. The product is then reacted, as described in Scheme 34, with ethyl alaninate**S3**, to yield the amidate **S35.10.**

Using the above procedures, but employing, in place of ethyl alaninate **S35.9,** different aminoesters **S34.9,** the corresponding products **S35.1** are obtained.

Alternatively, the phosphonate monoester **S34.1** is coupled, as described in Scheme 34, with an aminoester **S34.9** to produce the amidate**S335.1**. If necessary, the R¹ substituent is then altered, by initial cleavage to afford the phosphonic acid **S35.2.** The procedures for this transformation depend on the nature of the R¹ group, and are described above. The phosphonic acid is then transformed into the ester amidate product **S35.3,** by reaction with the hydroxy compound R³OH, in which the group R³ is aryl, heterocycle, alkyl, cycloalkyl, haloalkyl etc, using the same coupling procedures (carbodiimide, Aldrithiol-2, PYBOP, Mitsunobu reaction etc) described in Scheme 34 for the coupling of amines and phosphonic acids.

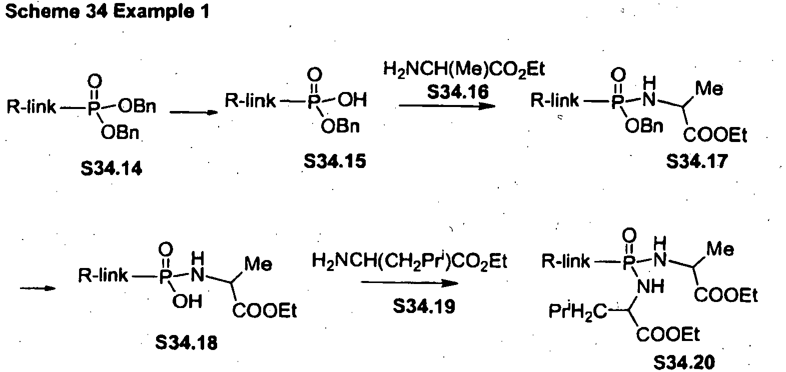

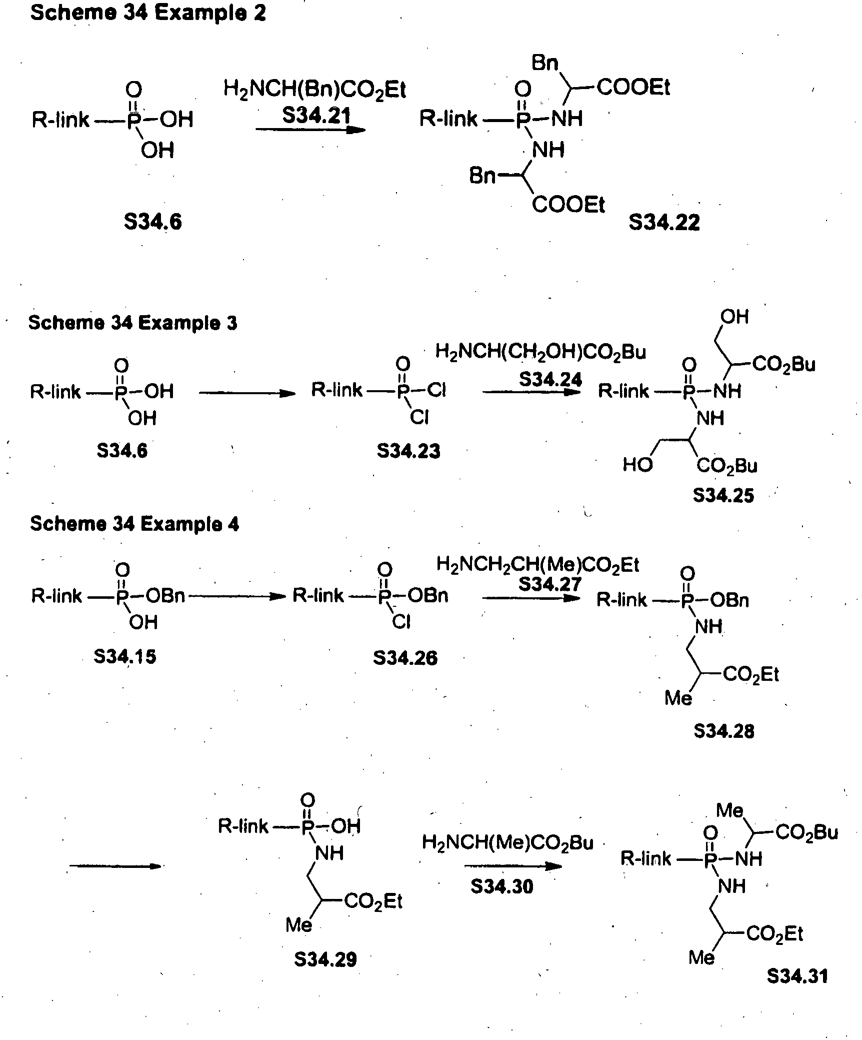

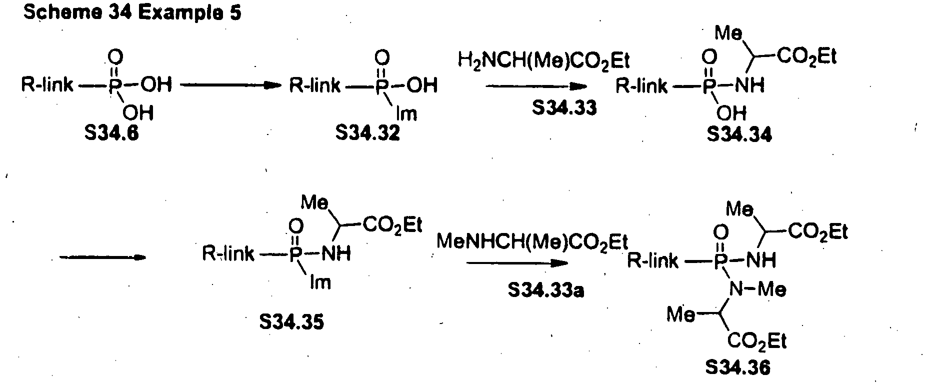

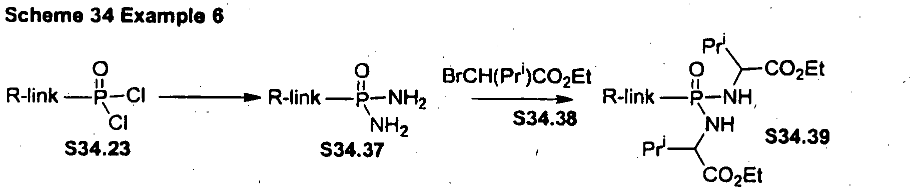

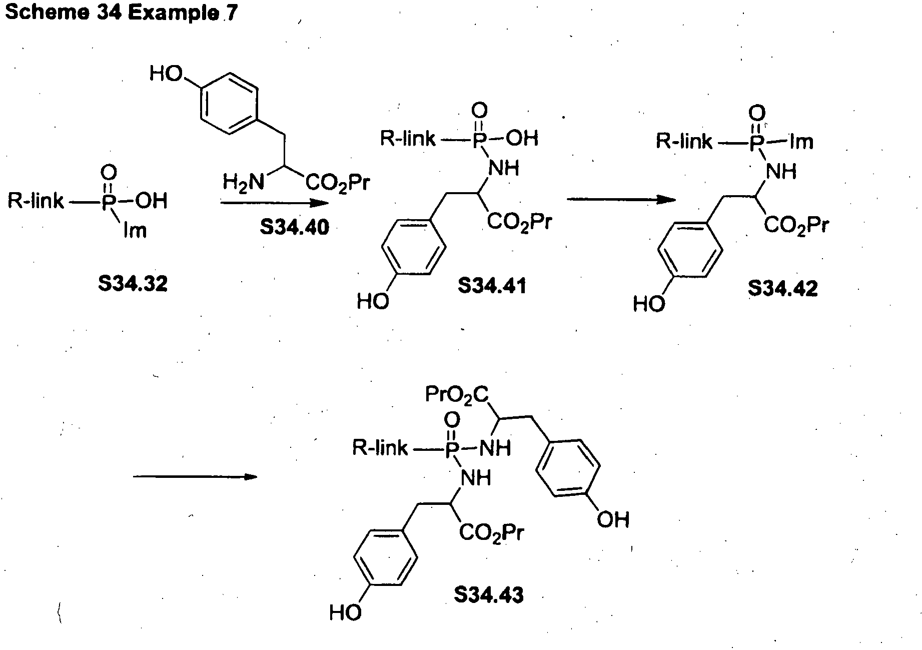

Examples of this method are shown in Scheme 35, Examples 2 and 3. In the sequence shown in Example 2, a monobenzyl phosphonate **S35.11** is transformed by reaction with ethyl alaninate, using one of the methods described above, into the monoamidate **S35.12.** The benzyl group is then removed by catalytic hydrogenation in ethylacetate solution over a 5% palladium on carbon catalyst, to afford the phosphonic acid amidate **S35.13.** The product is then reacted in dichloromethane solution at ambient temperature with equimolar amounts of 1-(dimethylaminopropyl)-3-ethylcarbodiimide and trifluoroethanol **S35.14,** for example as described in Tet. Lett., 2001, 42, 8841, to yield the amidate ester **S35.15.**

In the sequence shown in Scheme 35, Example 3, the monoamidate **S35.13** is coupled, in tetrahydrofuran solution at ambient temperature, with equimolar amounts of dicyclohexyl carbodiimide and 4-hydroxy-N-methylpiperidine **S35.16,** to produce the amidate ester product **S35.17.**

Using the above procedures, but employing, in place of the ethyl alaninate product **S35.12** different monoacids **S35.2,** and in place of trifluoroethanol **S35.14** or 4-hydroxy-N-methylpiperidine **S35.16,** different hydroxy compounds R³OH, the corresponding products **S35.3** are obtained.

Alternatively, the activated phosphonate ester **S34.8** is reacted with ammonia to yield the amidate **S35.4.** The product is then reacted, as described in Scheme 34, with a.haloester **S35.5,** in the presence of a base, to produce the amidate product **S35.6.** If appropriate, the nature of the R¹ group is changed, using the procedures described above, to give the product **S35.3.** The method is illustrated in Scheme 35, Example **4.** In this sequence, the monophenyl phosphoryl chloride **S35.18** is reacted, as described in Scheme **34,** with ammonia, to yield the amino product **S35.19.** This material is then reacted in N-methylpyrrolidinone solution at 170° with butyl 2-bromo-3-phenylpropionate **S35.20** and potassium carbonate, to afford the amidate product **S35.21.**

Using these procedures, but employing, in place of butyl 2-bromo-3-phenylpropionate **S35.20,** different haloesters **S35.5,** the corresponding products **S35.6** are obtained.

The monoamidate products **S35.3** are also prepared from the doubly activated phosphonate derivatives **S34.7.** In this procedure, examples of which are described in Synlett., 1998, 1, 73, the intermediate **S34.7** is reacted with a limited amount of the aminoester **S34.9** to give the mono-displacement product **S34.11.** The latter compound is then reacted with the hydroxy compound R³OH in a polar organic solvent such as dimethylformamide, in the presence of a base such as diisopropylethylamine, to yield the monoamidate ester **S35.3.**

The method is illustrated in Scheme 35, Example 5. In this method, the phosphoryl dichloride **S35.22** is reacted in dichloromethane solution with one molar equivalent of ethyl N-methyl tyrosinate **S35.23** and dimethylaminopyridine, to generate the monoamidate **S35.24.** The product is then reacted with phenol **S35.25** in dimethylformarmide containing potassium carbonate, to yield the ester amidate product **S35.26.**

Using these procedures, but employing, in place of ethyl N-methyl tyrosinate **S35.23** or phenol **S35.25,** the aminoesters **34.9** and/or the hydroxy compounds R³OH, the corresponding products **S35.3** are obtained.

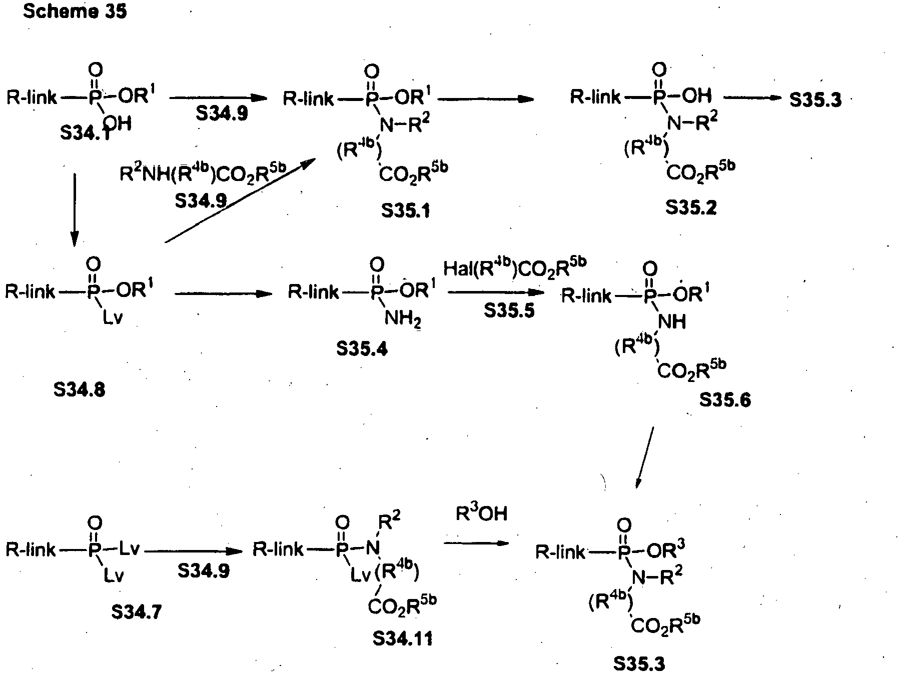

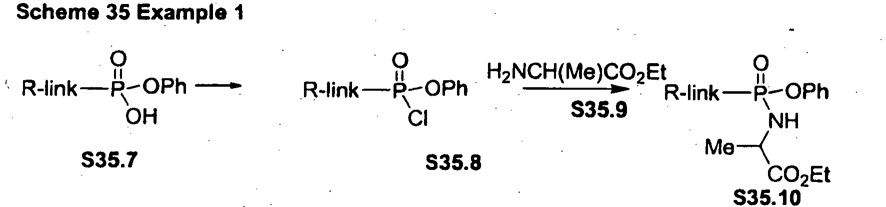

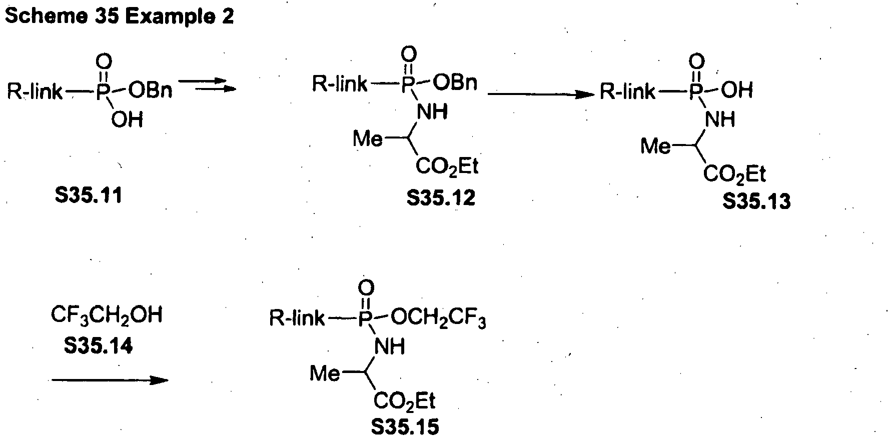

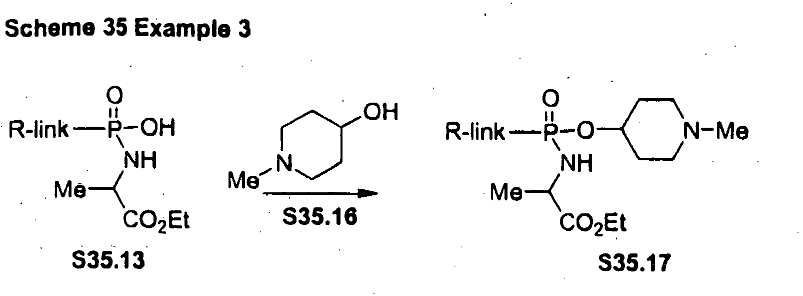

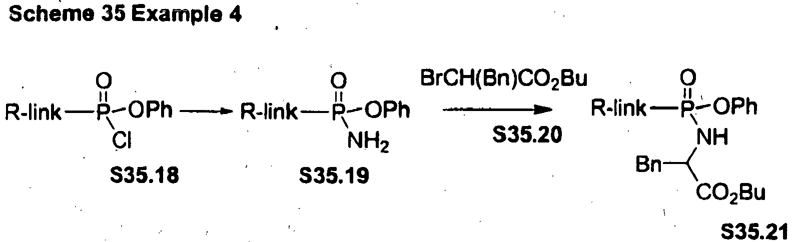

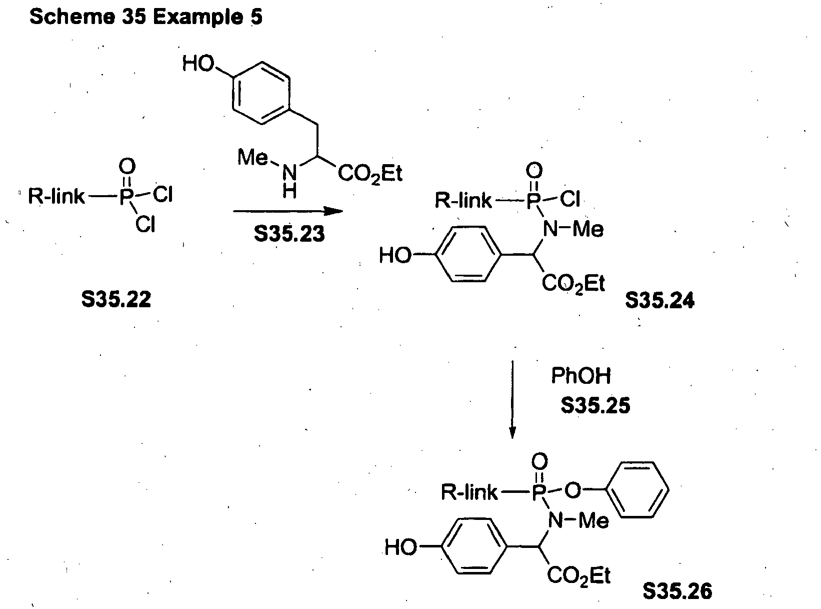

Scheme 36 illustrates methods for the preparation of carboalkoxy-substituted phosphonate diesters in which one of the ester groups incorporates a carboalkoxy substituent.

In one procedure, a phosphonate monoester **S34.1,** prepared as described above, is coupled, using one of the methods described above, with a hydroxyester **S36.1,** in which the groups R^{4b} and R^{5b} are as described in Scheme 34. For example, equimolar amounts of the reactants are coupled in the presence of a carbodiimide such as dicyclohexyl carbodiimide, as described in Aust. J. Chem., 1963, 609, optionally in the presence of dimethylaminopyridine, as described in Tet., 1999, 55, 12997. The reaction is conducted in an inert solvent at ambient temperature.

The procedure is illustrated in Scheme 36, Example 1. In this method, a monophenyl phosphonate **S36.9** is coupled, in dichloromethane solution in the presence of dicyclohexyl carbodiimide, with ethyl 3-hydroxy-2-methylpropionate **S36.10** to yield the phosphonate mixed diester **S36.11.**

Using this procedure, but employing, in place of ethyl 3-hydroxy-2-methylpropionate **S36.10,** different hydroxyesters **S33.1,** the corresponding products **S33.2** are obtained.

The conversion of a phosphonate monoester **S34.1** into a mixed diester **S36.2** is also accomplished by means of a Mitsunobu coupling reaction with the hydroxyester **S36.1,** as described in Org. Lett., 2001, 643. In this method, the reactants **34.1** and **S36.1** are combined in a polar solvent such as tetrahydrofuran, in the presence of a triarylphosphine and a dialkyl azodicarboxylate, to give the mixed diester **S36.2.** The R¹ substituent is varied by cleavage, using the methods described previously, to afford the monoacid product **S36.3.** The product is then coupled, for example using methods described above, with the hydroxy compound R³OH, to give the diester product **S36.4.**

The procedure is illustrated in Scheme 36, Example 2. In this method, a monoallyl phosphonate **S36.12** is coupled in tetrahydrofuran solution, in the presence of triphenylphosphine and diethylazodicarboxylate, with ethyl lactate **S36.13** to give the mixed diester **S36.14.** The product is reacted with tris(triphenylphosphine) rhodium chloride (Wilkinson catalyst) in acetonitrile, as described previously, to remove the allyl group and produce the monoacid product **S36.15.** The latter compound is then coupled, in pyridine solution at ambient temperature, in the presence of dicyclohexyl carbodiimide, with one molar equivalent of 3-hydroxypyridine **S36.16** to yield the mixed diester **S36.17.**

Using the above procedures, but employing, in place of the ethyl lactate **S36.13** or 3-hydroxypyridine, a different hydroxyester **S36.1** and/or a different hydroxy compound R³OH, the corresponding products **S36.4** are obtained.

The mixed diesters **S36.2** are also obtained from the monoesters **S34.1** via the intermediacy of the activated monoesters **S36.5.** In this procedure, the monoester **S34.1** is converted into the activated compound **S36.5** by reaction with, for example, phosphorus pentachloride, as described in J. Org. Chem., 2001, 66, 329, or with thionyl chloride or oxalyl chloride (Lv = Cl), or with triisopropylbenzenesulfonyl chloride in pyridine, as described in Nucleosides and Nucleotides, 2000, 19, 1885, or with carbonyl diimidazole, as described in J. Med. Chem., 2002, 45, 1284. The resultant activated monoester is then reacted with the hydroxyester **S36.1,** as described above, to yield the mixed diester **S36.2.**

The procedure is illustrated in Scheme 36, Example 3. In this sequence, a monophenyl phosphonate **S36.9** is reacted, in acetonitrile solution at 70 °C, with ten equivalents of thionyl chloride, so as to produce the phosphoryl chloride **S36.19.** The product is then reacted with ethyl 4-carbamoyl-2-hydroxybutyrate **S36.20** in dichloromethane containing triethylamine, to give the mixed diester **S36.21.**

Using the above procedures, but employing, in place of ethyl 4-carbamoyl-2-hydroxybutyrate **S36.20,** different hydroxyesters **S36.1,** the corresponding products **S36.2** are obtained.

The mixed phosphonate diesters are also obtained by an alternative route for incorporation of the R³O group into intermediates **S36.3** in which the hydroxyester moiety is already incorporated. In this procedure, the monoacid intermediate **S36.3** is converted into the activated derivative **S36.6** in which Lv is a leaving group such as chloro, imidazole, and the like, as previously described. The activated intermediate is then reacted with the hydroxy compound R³OH, in the presence of a base, to yield the mixed diester product **S36.4.**

The method is illustrated in Scheme 36, Example 4. In this sequence, the phosphonate monoacid **S36.22** is reacted with trichloromethanesulfonyl chloride in tetrahydrofuran containing collidine, as described in J. Med. Chem., 1995, 38, 4648, to produce the trichloromethanesulfonyloxy product **S36.23.** This compound is reacted with 3-(morpholinomethyl)phenol **S36.24** in dichloromethane containing triethylamine, to yield the mixed diester product **S36.25.**

Using the above procedures, but employing, in place of with 3-(morpholinomethyl)phenol **S36.24,** different alcohols R³OH, the corresponding products **S36.4** are obtained.

The phosphonate esters **S36.4** are also obtained by means of alkylation reactions performed on the monoesters **S34.1.** The reaction between the monoacid **S34.1** and the haloester **S36.7** is performed in a polar solvent in the presence of a base such as diisopropylethylamine, as described in Anal. Chem., 1987, 59, 1056, or triethylamine, as described in J. Med. Chem., 1995, 38,1372, or in a non-polar solvent such as benzene, in the presence of 18-crown-6, as described in Syn. Comm., 1995, 25, 3565.

The method is illustrated in Scheme 36, Example 5. In this procedure, the monoacid **S36.26** is reacted with ethyl 2-bromo-3-phenylpropionate **S36.27** and diisopropylethylamine in dimethylformamide at 80 °C to afford the mixed diester product **S36.28.**

Using the above procedure, but employing, in place of ethyl 2-bromo-3-phenylpropionate **S36.27,** different haloesters **S36.7,** the corresponding products **S36.4** are obtained.

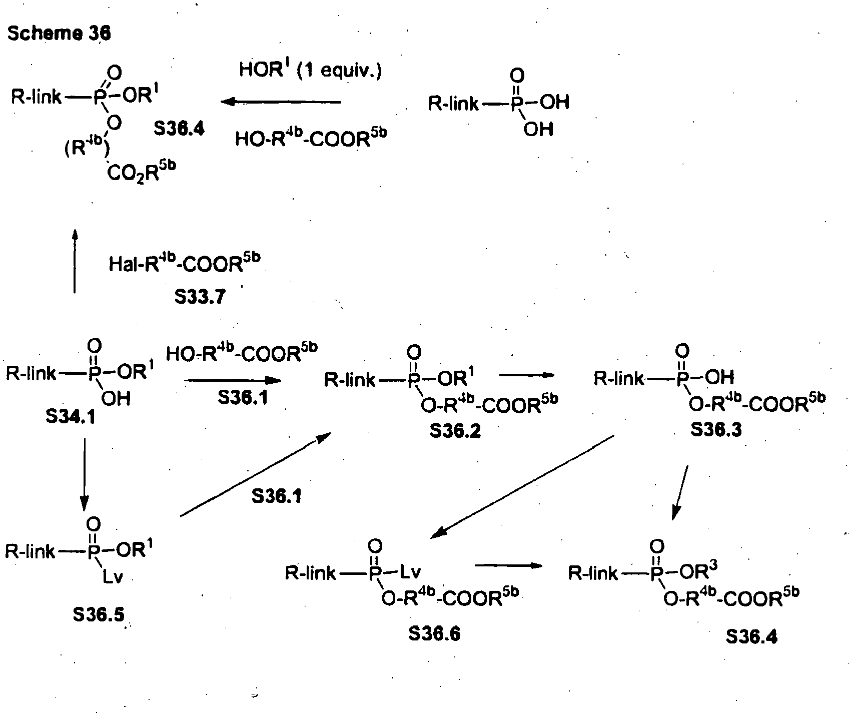

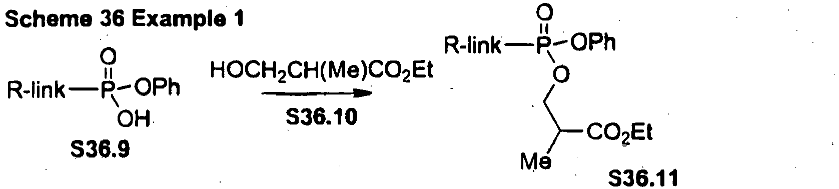

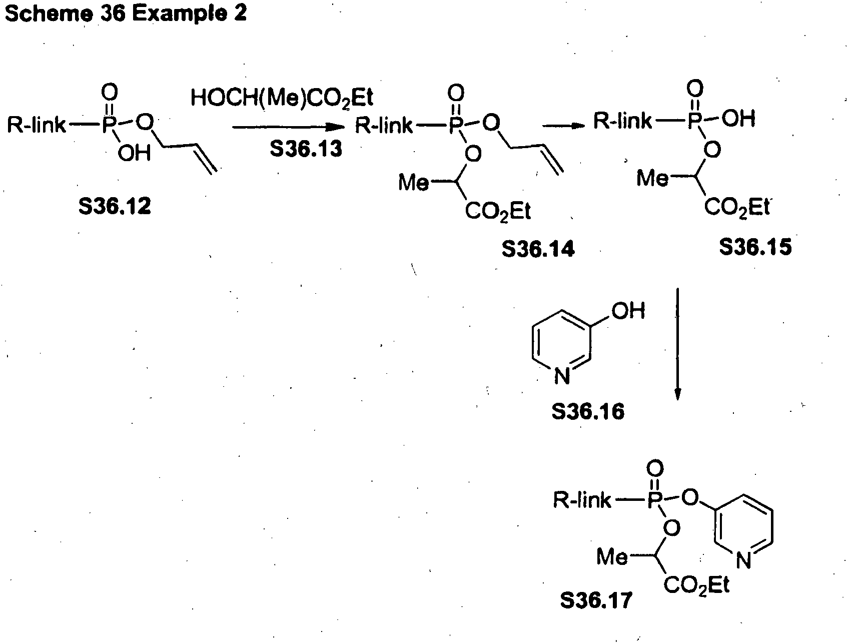

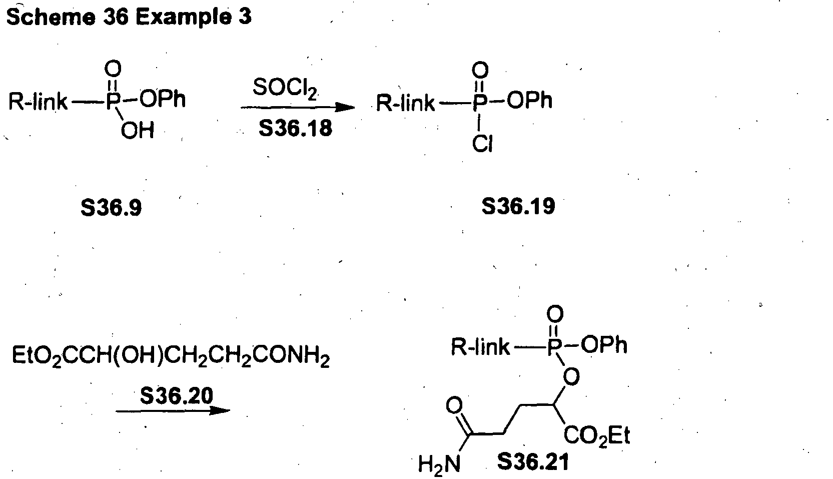

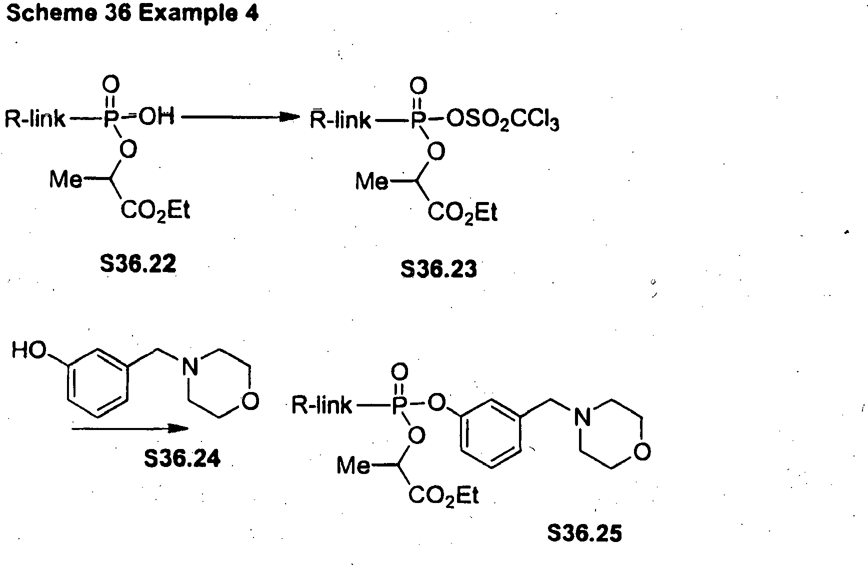

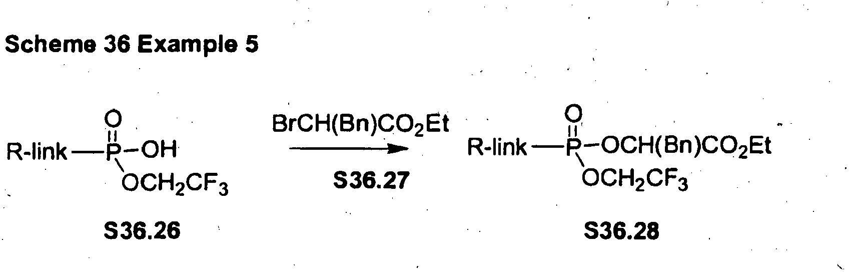

Scheme 37 illustrates methods for the preparation of phosphonate diesters in which both the ester substituents incorporate carboalkoxy groups.

The compounds are prepared directly or indirectly from the phosphonic acids **S34.6.** In one alternative, the phosphonic acid is coupled with the hydroxyester **S37.2,** using the conditions described previously in Schemes 34-36, such as coupling reactions using dicyclohexyl carbodiimide or similar reagents, or under the conditions of the Mitsunobu reaction, to afford the diester product **S37.3** in which the ester substituents are identical.

This method is illustrated in Scheme 37, Example 1. In this procedure, the phosphonic acid **S34.6** is reacted with three molar equivalents of butyl lactate **S37.5** in the presence of Aldrithiol-2 and triphenyl phosphine in pyridine at ca. 70 °C, to afford the diester **S37.6.**

Using the above procedure, but employing, in place of butyl lactate **S37.5,** different hydroxyesters **S37.2,** the corresponding products **S37.3** are obtained.

Alternatively, the diesters **S37.3** are obtained by alkylation of the phosphonic acid **S34.6** with a haloester **S37.1.** The alkylation reaction is performed as described in Scheme 36 for the preparation of the esters **S36.4.**

This method is illustrated in Scheme 37, Example 2. In this procedure, the phosphonic acid **S34.6** is reacted with excess ethyl 3-bromo-2-methylpropionate **S37.7** and diisopropylethylamine in dimethylformamide at ca. 80 °C, as described in Anal. Chem., 1987, 59, 1056, to produce the diester **S37.8.**

Using the above procedure, but employing, in place of ethyl 3-bromo-2-methylpropionate **S37.7,** different haloesters **S37.1,** the corresponding products **S37.3** are obtained.

The diesters **S37.3** are also obtained by displacement reactions of activated derivatives **S34.7** of the phosphonic acid with the hydroxyesters **S37.2.** The displacement reaction is performed in a polar solvent in the presence of a suitable base, as described in Scheme 36. The displacement reaction is performed in the presence of an excess of the hydroxyester, to afford the diester product **S37.3 in** which the ester substituents are identical, or sequentially with limited amounts of different hydroxyesters, to prepare diesters **S37.3** in which the ester substituents are different.

The methods are illustrated in Scheme 37, Examples 3 and 4. As shown in Example 3, the phosphoryl dichloride **S35.22** is reacted with three molar equivalents of ethyl 3-hydroxy-2-(hydroxymethyl)propionate **S37.9** in tetrahydrofuran containing potassium carbonate, to obtain the diester product **S37.10.**

Using the above procedure, but employing, in place of ethyl 3-hydroxy-2-(hydroxymethyl)propionate **S37.9,** different hydroxyesters **S37.2,** the corresponding products **S37.3** are obtained.

Scheme 37, Example 4 depicts the displacement reaction between equimolar amounts of the phosphoryl dichloride **S35.22** and ethyl 2-methyl-3-hydroxypropionate **S37.11,** to yield the monoester product **S37.12.** The reaction is conducted in acetonitrile at 70° in the presence of diisopropylethylamine. The product **S37.12** is then reacted, under the same conditions, with one molar equivalent of ethyl lactate **S37.13,** to give the diester product **S37.14.**

Using the above procedures, but employing, in place of ethyl 2-methyl-3-hydroxypropionate **S37.11** and ethyl lactate **S37.13,** sequential reactions with different hydroxyesters **S37.2,** the corresponding products **S37.3** are obtained.

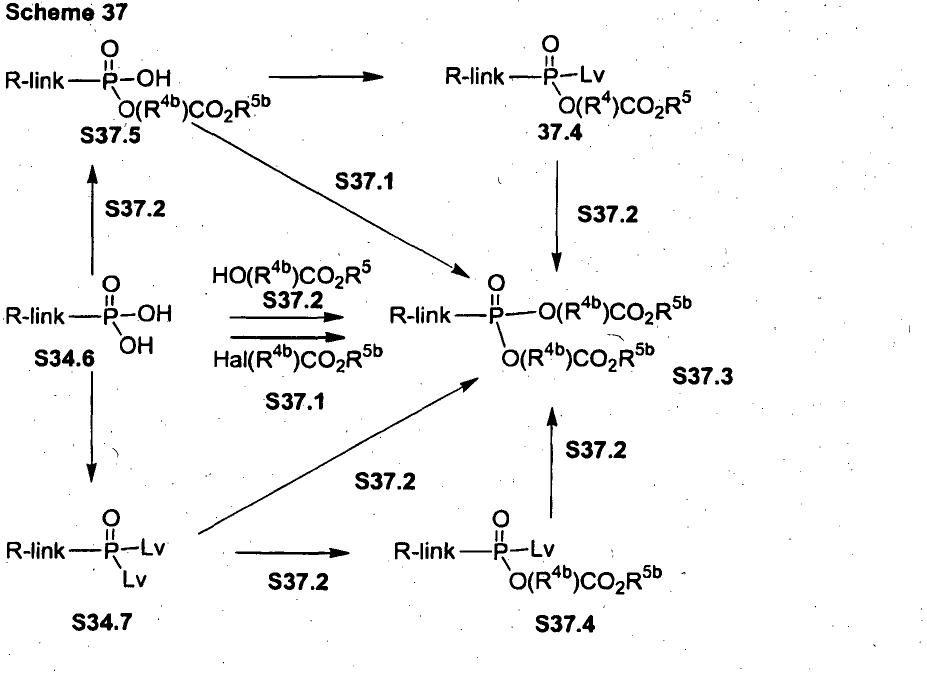

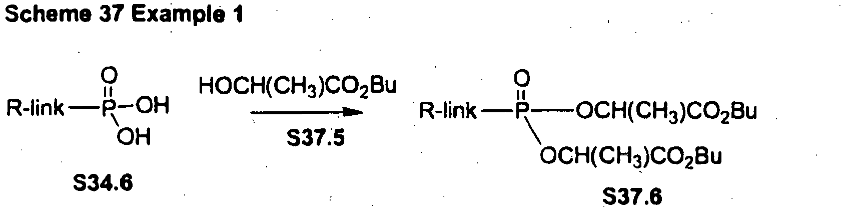

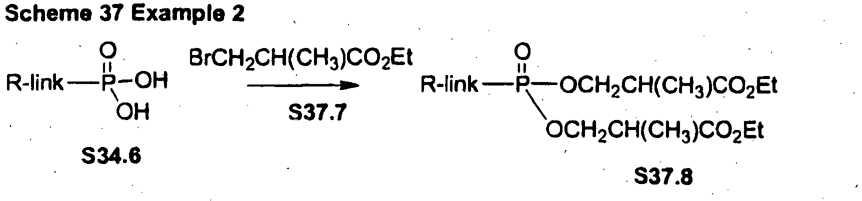

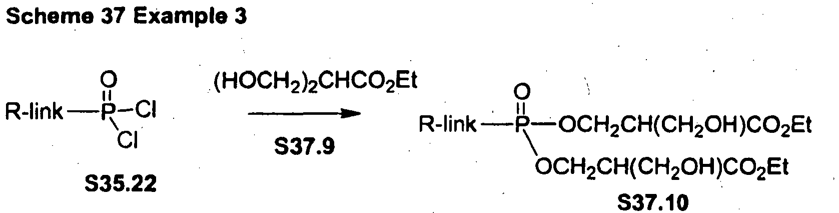

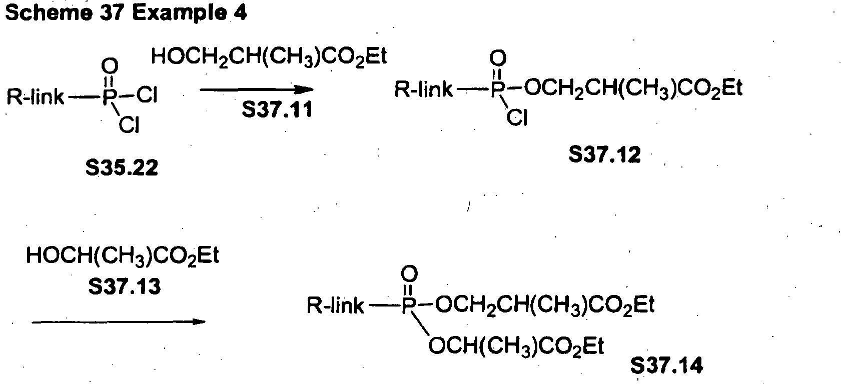

2,2-Dimethyl-2-aminoethylphosphonic acid intermediates can be prepared by the route in Scheme 5. Condensation of 2-methyl-2-propanesulfinamide with acetone give sulfinyl imine **S38.11** (J. Org. Chem. 1999, 64, 12). Addition of dimethyl methylphosphonate lithium to **S38.11** afford **S38.12.** Acidic methanolysis of **S38.12** provide amine **S38.13.** Protection of amine with Cbz group and removal of methyl groups yield phosphonic acid **S38.14**, which can be converted to desired **S38.15** (Scheme 38a) using methods reported earlier on. An alternative synthesis of compound **S38.14** is also shown in Scheme 38b. Commercially available 2-amino-2-methyl-1-propanol is converted to aziridines **S38.16** according to literature methods (J. Org. Chem. 1992, 57, 5813; Syn. Lett. 1997, 8, 893). Aziridine opening with phosphite give **S38.17** (Tetrahedron Lett. 1980, 21, 1623). Reprotection) of **S38.17** affords **S38.14.**

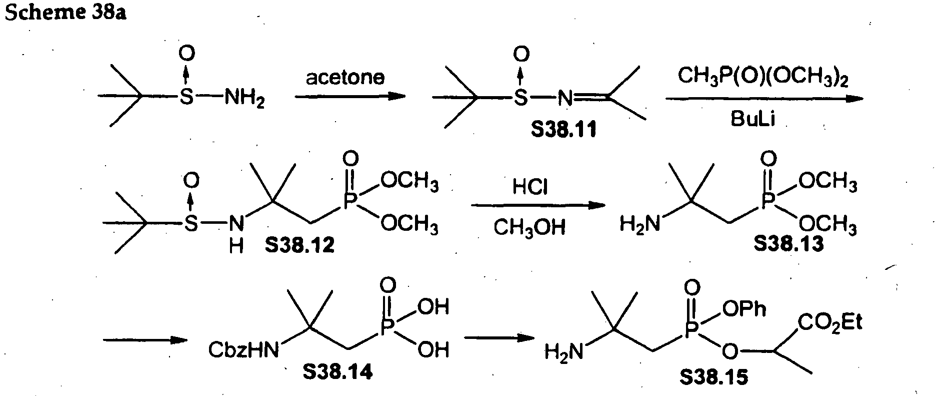

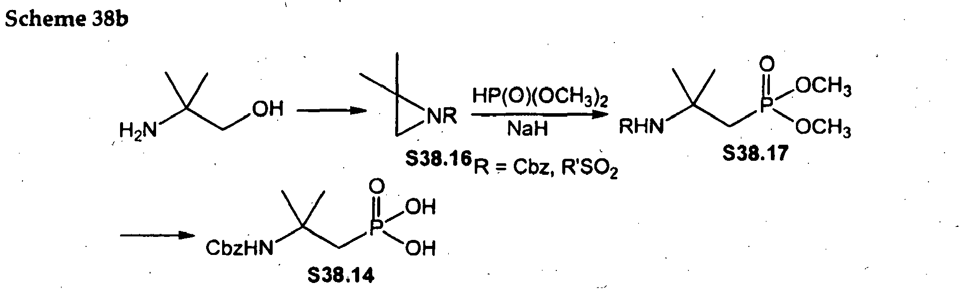

In another embodiment, the invention provides a conjugate, or a pharmaceutically acceptable salt or solvate thereof, that is a compound of the formulae I, II, III, or IV:

### EXEMPLARY EMBODIMENTS

The invention also provides a compound selected from the group consisting of: 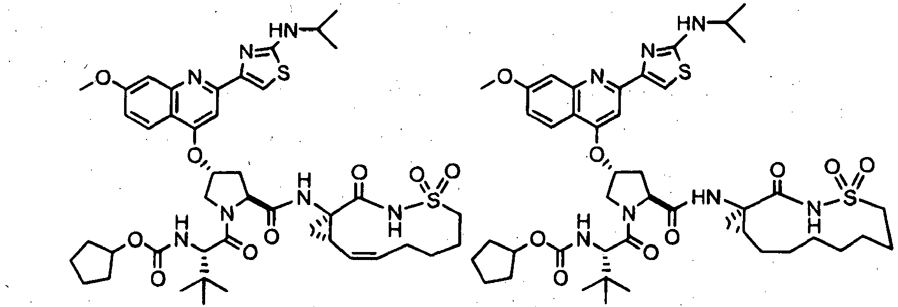 and

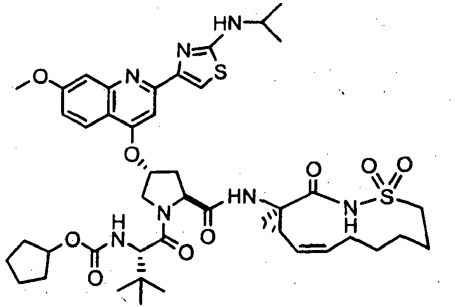

The invention also provides a pharmaceutical composition comprising an effective amount of a compound or conjugate of the invention, or a pharmaceutically acceptable salt or solvate thereof, in combination with a pharmaceutically acceptable excipient.

This invention also pertains to increasing cellular accumulation and retention of a drug compound, thus improving their therapeutic and diagnostic value, comprising linking the compound to one or more phosphonate groups.

The invention also provides compounds for use in a method of inhibiting HCV; comprising administering to a mammal afflicted with a condition associated with HCV activity, an amount of a compound of the invention, effective to inhibit HCV.

The invention also provides compounds of the invention for use in a medical therapy (preferably for use in inhibiting HCV or treating a condition associated with HCV activity), as well as the use of a compound of the invention for the manufacture of a medicament useful for inhibiting HCV or the treatment of a condition associated with HCV activity in a mammal.

The invention also provides processes and novel intermediates disclosed herein which are useful for preparing compounds of the invention. Some of the compounds of the invention are useful to prepare other compounds of the invention.

In another aspect the invention provides a method of inhibiting HCV activity in a sample comprising treating the sample with a compound or conjugate of the invention.

### DETAILED DESCRIPTION OF EXEMPLARY EMBODIMENTS

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the embodiments.

The invention will now be illustrated by the following non-limiting Examples:

### EXAMPLES

### Example 108: Preparation of Compound 108.

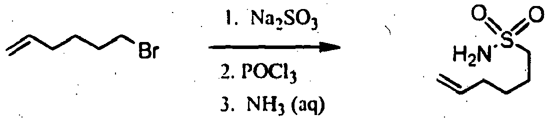

Step 1. To a solution of Na₂SO₃(6 g, 48 mmol) in H₂O(28 mL) was added 6-bromo-1-hexene (5.4 mL, 40 mmol). The reaction mixture was heated to reflux for 4 hr. The reaction mixture was cooled to rt, and extracted with Et₂O (20 mL). The aqueous phase was evaporated to a white solid, and dried at 130°C under vacuum for 2 hr. The resulting white solid was treated with POCl₃ (40 mL) for 4 hr at 130 °C. Solvent was evaporated to dryness. The residue was taken up in CH₃CN (50 mL) and cooled to 0°C. To this solution was added aqueous NH₃(100 mL, 28%) in CH₃CN (40 mL) dropwise. After the addition, CH₂Cl₂(100 mL) was added, and the two phases were separated. The organic phase was washed with H₂0 (50 mL), brine (50 mL) and dried over Na₂SO₄. The crude product was collected after evaporation of the solvent.

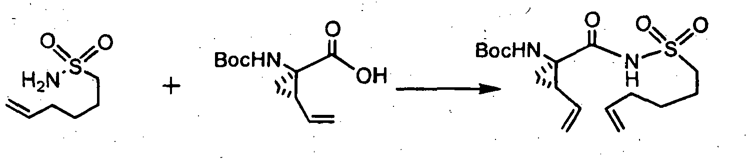

Step 2. To a solution of acid (2.0 g, 8.8 mmol) in THF (30 (mL) stirred at rt was added CDI (1.6 g, 9.7 mmol). The reaction mixture was heated to 65°C for 2 hr. A solution of sulfonamide (2.6 g, 15.3 mmol) in THF (5.0 mL) was added, followed by DBU (2.0 mL). After the addition, the reaction mixture was heated for 14 hr at 65 °C. The reaction mixture was cooled to rt and diluted with EtOAc, washed with saturated NH₄Cl, brine and dried over Na₂SO₄. The drying agent was filtered off and the solvent was evaporated. The residue was purified by SiO₂ column (10-20-35% EtOAc in hexanes) to give the desired product (1.1 g). HNMR (300 MHz, CDCl₃): δ 5.44-5.76 (m, 2H), 5.21 (d, 1H), 5.06 (d, 1H), 4.96-4.86 (m, 2H), 3.4-3.34 (m, 2H), 2.14-1.92 (m, 2H), 1.86-1.66 (m, 2H), 1.38 (s, 9H).

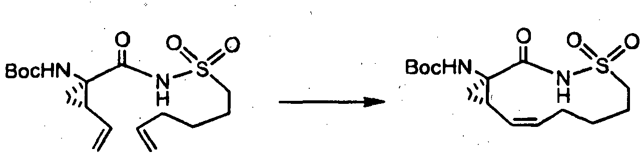

Step 3. A solution of starting material (210 mg, 0.57 mmol) in CH₂Cl₂(130 mL) was degassed with a gentle stream of N₂ for 40 min. Grubbs catalyst (G1, 93 mg, 0.11 mmol) was added and degassed for 30 min. The reaction mixture was then heated at 65°C for 6 hr. The reaction mixture was cooled to rt and solvent was evaporated off. The residue was purified by SiO₂ column (20-35-45% EtOAc in hexanes) to give the desired product (40 mg, 19%). HNMR (300 MHz, CDCl₃): δ 10.2 (bs, 1H), 5.51-5.22 (m, 1H), 5.25 (s, 1H), 3.33-3.23 (t, 1H), 3.01-2.88 (m, 1H), 2.28-1.7 (m, 6H), 1.43 (s, 9H), 1.4-1.15 (m, 2H).

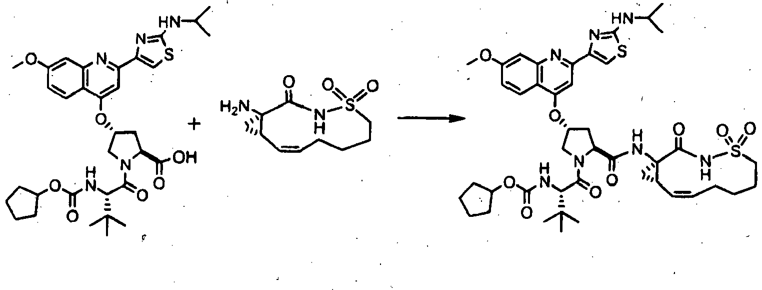

Step 4. To a solution of cyclic acylsulfonamide (100 mg) in CH₂Cl₂(5 mL) was added TFA (2.0 (mL). The reaction mixture was stirred at rt for 3 hr. Solvent was removed under vacuum. The residue was azeotroped with PhMe three times. The crude TFA salt was diluted with DMF (2.0 mL), to this solution was added acid (100 mg, 0.15 mmol), HATU (87 mg, 0.23 mmol) and NMM (62 mg, 0.61 mmol). The resulting reaction mixture was stirred at rt for 14 hr. Diluted with EtOAc and washed with saturated NH₄Cl, brine and dried over Na₂SO₄. The drying agent was filtered off and the solvent was evaporated. The crude product was purified by HPLC to give compound **108** as a yellow solid (15 mg, 11%). HNMR (300 MHz, CDCl₃): δ 10.02 (bs, 1H), 8.76 (s, 1H), 7.82-7.52 (m, 5H), 5.74 (s, 1H), 5.6-5.5 (m, 1H), 5.24-5.02 (m, 2H), 4.73 (t, J = 8.2 Hz, 1H), 4.61 (d, J = 12.3 Hz, 1H), 4.38 (s, 1H), 3.95 (s, 3H), 3.6-3.5 (m, 1H), 3.2 (t, J = 12 Hz, 1H), 2.9-2.6 (m, 3H), 2.3-1.5 (m, 6H), 1.42 (d, J = 6.6 Hz, 6H), 0.95 (s, 9H).

### Example 109: Preparation of Compound 109.

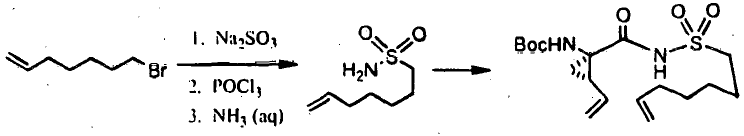

Step 1. See example **108.**

HNMR (300 MHz, CDCl₃): δ 5.8-5.48 (m, 2H), 5.3-4.9 (m, 5H), 3.4-3.2 (m, 2H), 2.18-1.58 (m, 7H), 1.44 (s, 9H).

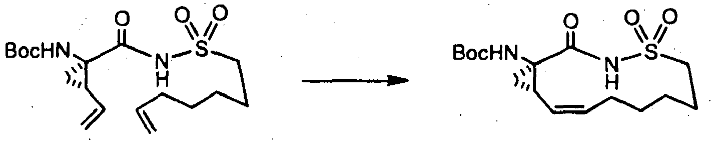

Step 2. A solution of starting material (982 mg, 2.54 mmol) in CH₂Cl₂ (100 mL) was degassed with a gentle stream of N₂ for 40 min. Grubbs catalyst (312 mg, 0.38 mmol) was added and degassed for 30 min. The reaction mixture was then heated at 65 °C for 24 hr. The reaction mixture was cooled to rt and solvent was evaporated off. The residue was purified by SiO₂ column (20-35-45% EtOAc in hexanes) to give the desired product (510mg, 56%). HNMR (300 MHz, CDCl₃): δ 9.9 (s, 1H), 5.72-5.6 (m, 1H), 5.44-5.28 (m, 2H), 3.7-3.6 (m, 1H), 3.04-2.9 (m, 1H)2.2-1.6 (m, 4H), 1.42 9s, 9H), 1.22-1.14 (m, 2H).

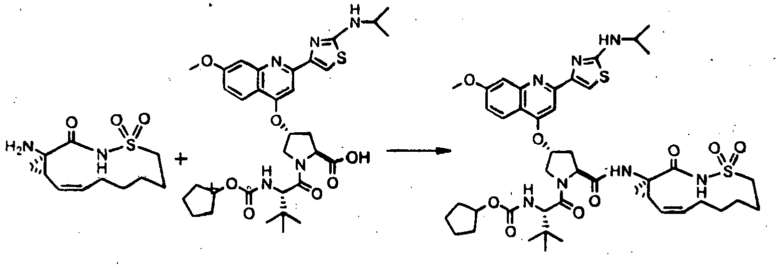

Step 3. To a solution of cyclic acylsulfonamide (92 mg) in CH₂Cl₂(4.0 mL) was added TFA (2.0 (mL). The reaction mixture was stirred at rt for 3 hr. Solvent was removed under vacuum. The residue was azeotroped with PhMe three times. The crude TFA salt was diluted with DMF (2.0 mL), to this solution was added acid (100 mg, 0.15 mmol), HATU (87 mg, 0.23 mmol) and NMM (62 mg, 0.61 mmol). The resulting reaction mixture was stirred at rt for 14 hr. Diluted with EtOAc and washed with saturated NH₄Cl, brine and dried over Na₂SO₄. The drying agent was filtered off and the solvent was evaporated. The crude product was purified by HPLC to give compound **109** as a yellow solid (38 mg, 16%). HNMR (300 MHz, CDCl₃): δ 9.8 (s, 1H), 8.66 (s, 1H), 8.13 (d, J = 9.4 Hz, 1H), 7.76-7.7 (m, 2H), 5.76 (s, 1H), 5.7-5.62 (m, 1H), 5.31 (dd, J = 16.5, 7.3 Hz, 1H), 5.18 (s, 1H), 5.04 (s, 1H), 4.75-4.63 (m, 2H), 4.12-4.04 (m, 1H), 3.95.(s, 3H), 3.56-3.54 (m, 1H), 2.9-2.67 (m, 2H), 2.05-1.21 (m, 8H), 0.93 (s, 9H).

### Example 110: Preparation of Compound 110.

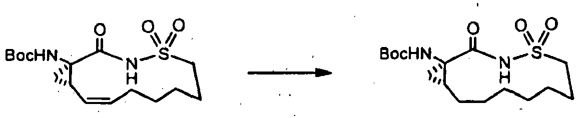

Step 1. To a solution of cyclic acylsulfonamide (230 mg, 0.64 mmol) in THF (2.0 mL) was added 2,4,6-triiospropylbenzenesulphonyl hydrazide (1.1 g, 3.85 mmol). The reaction flask was then placed in a preheated 65 °C oil bath. Et₃N (388 mg, 3.85 mmol) was added slowly. After the addition, the reaction mixture was cooled to rt, diluted with EtOAc, and washed with NH₄Cl, NaHCO₃, brine. The organic phase was dried over Na₂SO₄. The residue was purified by SiO₂ column (20-35-45% EtOAc in hexanes) to give the desired product (162mg, 70%). HNMR (300 MHz, CDCl₃): δ 9.8 (s, 1H), 4.1-3.84 (m, 2H), 3.14-3.02 (m, 1H), 2.86-2.74 (m, 1H)1.75-1.22 (m, 8H), 1.21 (9s, 9H).

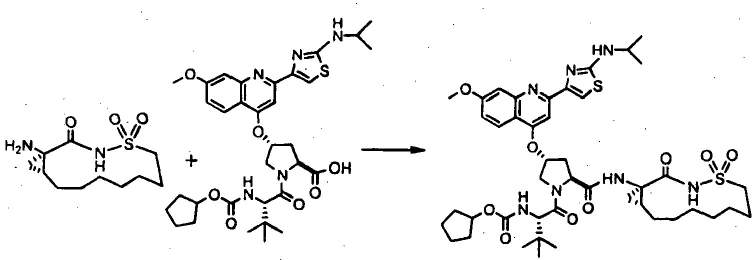

Step 2. To a solution of cyclic acylsulfonamide (80 mg, 0.22 mmol) in CH₂Cl₂(4.0 mL) was added TFA (2.0 mL). The reaction mixture was stirred at rt for 3 hr. Solvent was removed under vacuum. The residue was azeotroped with PhMe three times. The crude TFA salt was diluted with DMF (2.0 mL), to this solution was added acid (217 mg, 0.33 mmol), HATU (117 mg, 0.31 mmol) and NMM (89 mg, 0.88 mmol). The resulting reaction mixture was stirred at rt for 14 hr. Diluted with EtOAc and washed with saturated NH₄Cl, brine and dried over Na₂SO₄. The drying agent was filtered off and the solvent was evaporated. The crude product was purified by HPLC to give a yellow solid (39 mg, 16%). HNMR (300 MHz, CDCl₃): δ 9.82 (bs, 1H), 8.66 (s, 1H), 8.13 (d, J = 9.4, 1H), 7.76 (d, J = 3.2 Hz, 1H), 7.76 (s, 1H), 7.4 (s, 1H), 5.73 (s, 1H), 5.25 (d, J = 8.8 Hz, 1H), 4.64-4.59 (m, 2H), 4.45 (s, 1H), 4.09-3.93 (m, 2H), 3.91 (s, 3H), 3.56-3.45 (m, 1H), 3.02-2.63 (m, 3H), 2.04-1.96 (m, 2H), 1.71-1.08 (m, 8H), 0.97 (s, 9H).

### BIOLOGICAL ASSAY DESCRIPTION

### Evaluation of Protease Inhibitors:

### NS3 Enzymatic Potency:

Purified NS3 protease is complexed with NS4A peptide and then incubated with serial dilutions of compound (DMSO used as solvent). Reactions are started by addition of dual-labeled peptide substrate and the resulting kinetic increase in fluorescence is measured. Non-linear regression of velocity data is performed to calculate IC_{50S}. Activity is initially be tested against genotype 1b protease. Depending on the potency obtained against genotype 1b, additional genotypes (1a, 2a, 3) and or protease inhibitor resistant enzymes (D168Y, D168V, or A156T mutants) may be tested. BILN-2061 is used as a control during all assays. Representative compounds were evaluated in this assay and were found to have an IC₅₀ of less than about 1 µm.

### Replicon Potency and Cytotoxicity:

Huh-luc cells (stably replicating Bartenschlager's I389luc-ubi-neo/NS3-3'/ET genotype 1b replicon) is treated with serial dilutions of compound (DMSO is used as solvent) for 72 hours. Replicon copy number is measured by bioluminescence and non-linear regression is performed to calculate EC_{50S}. Parallel plates treated with the same drug dilutions are assayed for cytotoxicity using the Promega CellTiter-Glocell, viability assay. Depending on the potency achieved against the 1b replicon, compounds may be tested against a genotype 1a replicon and/or inhibitor resistant replicons encoding D168Y or A156T mutations. BILN-2061 is used as a control during all assays.

### Effect of serum proteins on replicon potency:

Replicon assays are conducted in normal cell culture medium (DMEM + 10%FBS) supplemented with physiologic concentrations of human serum albumin (40 mg/mL) or -acid glycoprotein (1 mg/mL). EC_{50S} in the presence of human serum proteins are compared to the EC₅₀ in normal medium to determine the fold shift in potency.

### Enyzmatic Selectivity:

The inhibition of mammalian proteases including Porcine Pancreatic Elastase, Human Leukocyte Elastase, Protease 3, and Cathepsin D are measured at Kₘ for the respective substrates for each enzyme. IC₅₀ for each enzyme is compared to the IC₅₀ obtained with NS3 1b protease to calculate selectivity. Representative compounds of the invention have shown activity.

### MT-4 Cell Cytotoxicity:

MT4 cells are treated with serial dilutions of compounds for a five day period. Cell viability is measured at the end of the treatment period using the Promega CellTiter-Glo assay and non-linear regression is performed to calculate CC₅₀.

### Compound Concentration Associated with Cells at EC₅₀:

Huh-luc cultures are incubated with compound at concentrations equal to EC₅₀. At multiple time points (0 - 72 hours), cells are washed 2X with cold medium and extracted with 85% acetonitrile; a sample of the media at each time-point will also be extracted. Cell and media extracts are analyzed by LC/MS/MS to determine the Molar concentration of compounds in each fraction. Representative compounds of the invention have shown activity.

### Solubility and Stability:

Solubility is determined by taking an aliquot of 10 mM DMSO stock solution and preparing the compound at a final concentration of 100 µM in the test media solutions (PBS, pH 7.4 and 0.1 N HCl, pH 1.5) with a total DMSO concentration of 1%. The test media solutions are incubated at room temperature with shaking for 1 hr. The solutions will then be centrifuged and the recovered supernatants are assayed on the HPLC/UV. Solubility will be calculated by comparing the amount of compound detected in the defined test solution compared to the amount detected in DMSO at the same concentration. Stability of compounds after an 1 hour incubation with PBS at 37°C will also be determined.

### Stability in Cryopreserved Human, Dog, and Rat Hepatocytes:

Each compound is incubated for up to 1 hour in hepatocyte suspensions (100 µl, 80,000 cells per well) at 37°C. Cryopreserved hepatocytes are reconstituted in the serum-free incubation medium. The suspension is transferred into 96-well plates (50 µL/well). The compounds are diluted to 2 µM in incubation medium and then are added to hepatocyte suspensions to start the incubation. Samples are taken at 0, 10, 30 and 60 minutes after the start of incubation and reaction will be quenched with a mixture consisting of 0.3% formic acid in 90% acetonitrile/10% water. The concentration of the compound in each sample is analyzed using LC/MS/MS. The disappearance half-life of the compound in hepatocyte suspension is determined by fitting the concentration-time data with a monophasic exponential equation. The data will also be scaled up to represent intrinsic hepatic clearance and/or total hepatic clearance.

### Stability in Hepatic S9 Fraction from Human, Dog, and Rat:

Each compound is incubated for up to 1 hour in S9 suspension (500 µl, 3 mg protein/mL) at 37°C (n = 3). The compounds are added to the S9 suspension to start the incubation. Samples are taken at 0, 10, 30, and 60 minutes after the start of incubation. The concentration of the compound in each sample is analyzed using LC/MS/MS. The disappearance half-life of the compound in S9 suspension is determined by fitting the concentration-time data with a monophasic exponential equation.

### Caco-2 Permeability:

Compounds are assayed via a contract service (Absorption Systems, Exton, PA). Compounds are provided to the contractor in a blinded manner. Both forward (A-to-B) and reverse (B-to-A) permeability will be measured. Caco-2 monolayers are grown to confluence on collagen-coated, microporous, polycarbonate membranes in 12-well Costar Transwell® plates. The compounds are dosed on the apical side for forward permeability (A-to-B), and are dosed on the basolateral side for reverse permeability (B-to-A). The cells are incubated at 37°C with 5% CO2 in a humidified incubator. At the beginning of incubation and at 1 hr and 2 hr after incubation, a 200-µL aliquot is taken from the receiver chamber and replaced with fresh assay buffer. The concentration of the compound in each sample is determined with LC/MS/MS. The apparent permeability, Papp, is calculated.

### Plasma Protein Blinding:

Plasma protein binding is measured by equilibrium dialysis. Each compound is spiked into blank plasma at a final concentration of 2 µM. The spiked plasma and phosphate buffer is placed into opposite sides of the assembled dialysis cells, which will then be rotated slowly in a 37°C water bath. At the end of the incubation, the concentration of the compound in plasma and phosphate buffer is determined. The percent unbound is calculated using the following equation: 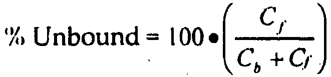 Where C_{f} and C_{b} are free and bound concentrations determined as the post-dialysis buffer and plasma concentrations, respectively

### CYP450 Profiling:

Each compound is incubated with each of 5 recombinant human CYP450 enzymes, including CYP1A2, CYP2C9, CYP3A4, CYP2D6 and CYP2C19 in the presence and absence of NADPH. Serial samples will be taken from the incubation mixture at the beginning of the incubation and at 5, 15, 30, 45 and 60 min after the start of the incubation. The concentration of the compound in the incubation mixture is determined by LC/MS/MS. The percentage of the compound remaining after incubation at each time point is calculated by comparing with the sampling at the start of incubation.

### Stability in Rat, Dog, Monkey and Human Plasma:

Compounds will be incubated for up to 2 hour in plasma (rat, dog, monkey, or human) at 37°C. Compounds are added to the plasma at final concentrations of 1 and 10 ug/mL. Aliquots are taken at 0, 5, 15, 30, 60, and 120 min after adding the compound. Concentration of compounds and major metabolites at each timepoint are measured by LC/MS/MS.

## Claims

1. A compound, including enantiomers thereof, of formula II: 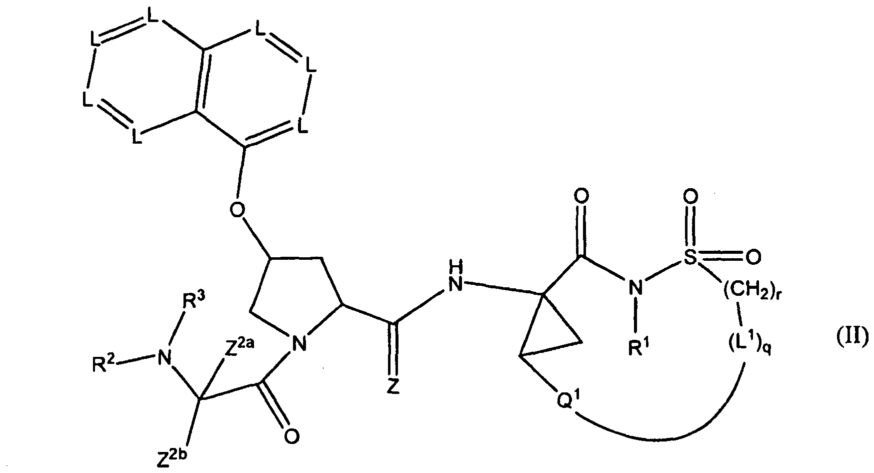 or a pharmaceutically acceptable salt, or solvate thereof,
wherein:
R¹ is independently selected from H, alkyl, alkenyl, alkynyl, aryl, cycloalkyl, heterocycle, halogen, haloalkyl, alkylsulfonamido, arylsulfonamido,-C(O)NHS(O)₂-, or -S(O)₂-, optionally substituted with one or more A³;
R² is selected from
a) -C(Y¹)(A³)
b) (C2-10)alkyl, (C3-7)cycloalkyl or (C1-4)alkyl-(C3-7)cycloalkyl,
where said cycloalkyl and alkyl-cycloalkyl may be mono-, di- or tri-substituted with (C1-3)alkyl, or
where said alkyl, cycloalkyl and alkyl-cycloalkyl may be mono- or disubstituted with substituents selected from hydroxy and O-(C1-4)alkyl, or
where each of said alkyl-groups may be mono-, di- or tri-substituted
with halogen, or
where each of said cycloalkyl groups being 5-, 6- or 7-membered, one
or two -CH2-groups not being directly linked to each other may be replaced by -O- such that the O-atom is linked to the N atom to which R² is attached via at least two C-atoms, or
c) phenyl, (C1-3)alkyl-phenyl, heteroaryl or (C1-3)alkyl-heteroaryl, wherein the heteroaryl-groups are 5- or 6-membered having from 1 to 3 heteroatoms selected from N, O and S, wherein said phenyl and heteroaryl groups may be mono-, di- or trisubstituted with substituents selected from halogen, -OH, (C1-4)alkyl, O-(C1-4)alkyl, S-(C1-4)alkyl,-NH2, -NH((C1-4)alkyl) and -N((C1-4)alkyl)2, -CONH2 and -CONH-(C1-4)alkyl;
R³ is PRT, H or (C1-6)alkyl;
L is independently selected from C or N, providing there are no more than three consecutive N, eachC being substituted with one or more A³;
L¹ is independently selected from C or N, providing there are no more than three consecutive N, each substituted with one or more A³;
Z is O, S, C or N, substituted with A³;
Z^{2a} is H, (C1-10)alkyl, (C2-10)alkenyl, (C2-10)alkynyl, wherein any carbon atom may be replaced with a heteroatom selected from O, S or N, or Z^{2a} optionally forms a carbocyle or heterocycle with Q¹, or any A³;
Z^{2b} is H, (C1-6)alkyl, (C2-8)alkenyl, (C2-8)alkynyl;
Q¹ is (C1)alkylene, (C2-8)alkenylene, or (C2-8)alkynylene;
A³ is independently selected from PRT, H, -OH, -C(O)OH, -(CH₂)ₘ-, -C(O)O-, -NH-, cyano, alkyl, alkenyl, alkynyl, amino, amido, imido, imino, halogen, CF₃, CH₂CF₃, cycloalkyl, nitro, aryl, aralkyl, alkoxy, aryloxy, heterocycle, heteroaryl, -C(A²)₃, -C(A²)₂-C(O)A², -C(O)A², -C(O)OA², -O(A²), -N(A²)₂, -S(A²), -CH₂P(O)(A²)(OA²), -CH₂P(O)(A²)(N(A²)₂), -CH₂P(O)(OA²)(OA²), -OCH₂P(O)(OA²)(OA²), -OCH₂P(O)(A²)(OA²), -OCH₂P(O)(A²)(N(A²)₂), -C(O)OCH₂P(O)(OA²)(OA²), -C(O)OCH₂P(O)(A²)(OA²), -C(O)OCH₂P(O)(A²)(N(A²)₂), -CH₂P(O)(OA²)(N(A²)₂), -OCH₂P(O)(OA²)(N(A²)₂), -C(O)OCH₂P(O)(OA²)(N(A²)₂), -CH₂P(O)(N(A²)₂)(N(A²)₂), -C(O)OCH₂P(O)(N(A²)₂)(N(A²)₂), -OCH₂P(O)(N(A²)₂)(N(A²)₂), -(CH₂)ₘ-heterocycle, -(CH₂)ₘC(O)Oalkyl, -O-(CH₂)ₘ-O-C(O)-Oalkyl, -O-(CH₂)ᵣ-O-C(O)-(CH₂)ₘ -alkyl, -(CH₂)ₘO-C(O)-O-alkyl, -(CH₂)ₘO-C(O)-O-cycloalkyl, -N(H)C(Me)C(O)O-alkyl, or alkoxy arylsulfonamide,
wherein each A³ may be optionally substituted with 1 to 4 -R¹, -P(O)(OA²)(OA²), -P(O)(OA²)(N(A²)₂), -P(O)(A²)(OA²), -P(O)(A²)(N(A²)₂), or -P(O)(N(A²)₂)(N(A²)₂), halogen, alkyl, alkenyl, alkynyl, aryl, carbocycle, heterocycle, aralkyl, aryl sulfonamide, aryl alkylsulfonamide, aryloxy sulfonamide, aryloxy alkylsulfonamide, aryloxy arylsulfonamide, alkyl sulfonamide, alkyloxy sulfonamide, alkyloxy alkylsulfonamide, -(CH₂)ₘheterocycle, -(CH₂)ₘ-C(O)O-alkyl, -O(CH₂)ₘOC(O)Oalkyl, -O-(CH₂)ₘ-O-C(O) -(CH₂)ₘ-alkyl, -(CH₂)ₘ-O-C(O)-O-alkyl, -(CH₂)ₘ-O-C(O)-O-cycloalkyl, -N(H)C(CH₃)C(O)O-alkyl, alkoxy, -N(R³)(R³) or alkoxy arylsulfonamide, optionally substituted with -R¹, or
A³ forms a carbocyclic or heterocyclic ring with any other A³ or Q¹;
Y₁ is O, S, N(R₂), N(OR₂) or N(N(R₂))₂;
A² is independently selected from H, alkyl, alkenyl, alkynyl, amino, amino acid, alkoxy, aryloxy, cyano, haloalkyl, cycloalkyl, aryl, heteroaryl, alkylsulfonamide, or arylsulfonamide, optionally substituted with A³;
PRT is selected from -CH₂OC(=O)R⁹ and -CH₂OC(=O)OR⁹ where R⁹ is C₁-C₆ alkyl, C₁-C₆ substituted alkyl, C₆-C₂₀ aryl or C₆-C₂₀ substitued aryl;
m is 0 to 6;
r is 1 to 2; and
q is 1 to 10.

2. A compound of claim 1 selected from the group consisting of: 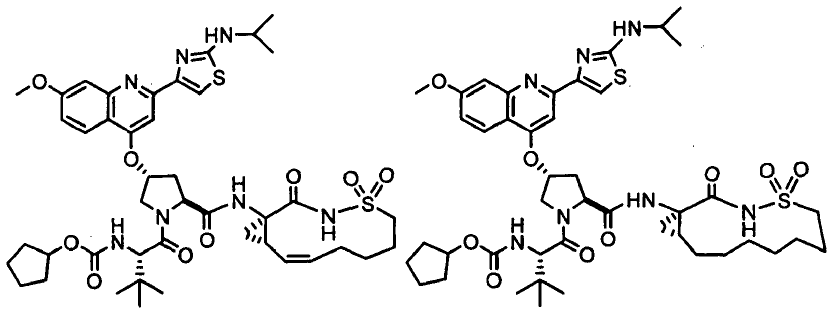 and

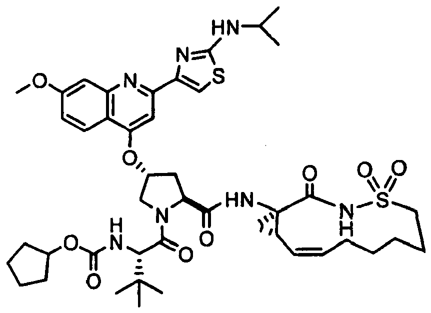

3. A compound of formula II according to claim 1, wherein R2 is selected from meanings (b) and (c) as defined in claim 1.

4. A pharmaceutical composition comprising the compound as described in any one of claims 1 to 3 and at least one pharmaceutically acceptable carrier.

5. The pharmaceutical composition of claim 4 for use in treating disorders associated with HCV.

6. The pharmaceutical composition of claim 5 further comprising a nucleoside analogue.

7. The pharmaceutical composition of claim 6 further comprising an interferon or pegylated interferon.

8. The pharmaceutical composition of claim 7, wherein said nucleoside analogue is selected from ribavirin, viramidine levovirin, a L-nucleoside, and isatoribine and said interferon is α-interferon or pegylated interferon.

9. A compound as described in any one of claims 1 to 3 for use in a method of treating disorders associated with hepatitis C.

10. A compound as described in any one of claims 1 to 3 for use in medical therapy.

11. The use of a compound as described in any one of claims 1 to 3 to prepare a medicament for treating a disorder associated with hepatitis C in a mammal.

## Patentansprüche

1. Verbindung der Formel II einschließlich ihrer Enantiomeren: 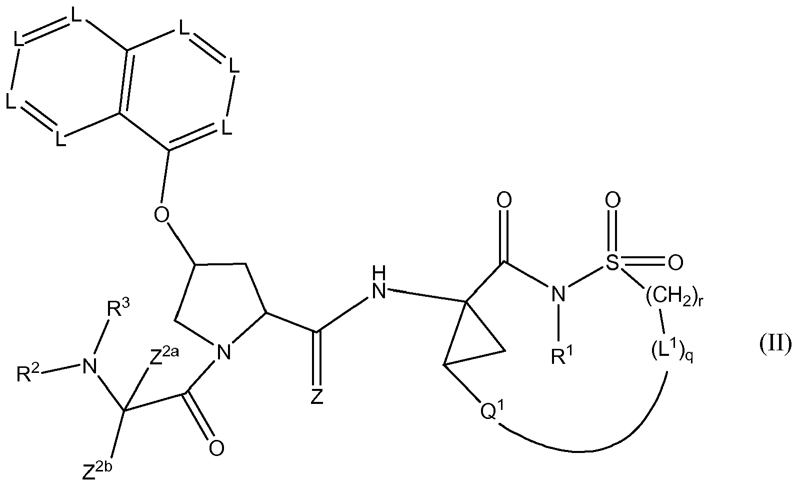 oder ein pharmazeutisch akzeptables Salz oder Solvat davon,
wobei:
R¹ unabhängig ausgewählt ist unter H, Alkyl, Alkenyl, Alkinyl, Aryl, Cycloalkyl, Heterocyclus, Halogen, Halogenalkyl, Alkylsulfonamido, Arylsulfonamido, -C(O)NHS(O)₂- oder -S(O)₂-, gegebenenfalls substituiert mit einem oder mehreren A³;
R² ausgewählt ist unter
a) -C(Y¹)(A³)
b) (C2-10)-Alkyl, (C3-7)-Cycloalkyl oder (C1-4)-Alkyl-(C3-7)-cycloalkyl,
wobei Cycloalkyl und Alkyl-cycloalkyl mono-, di- oder tri-substituiert mit (C1-3)-Alkyl sein können, oder
wobei Alkyl, Cycloalkyl und Alkyl-cycloalkyl mono- oder di-substituiert mit Substituenten sein können, die ausgewählt sind unter Hydroxy und O-(C1-4)-Alkyl, oder
wobei jede der Alkylgruppen mono-, di- oder tri-substituiert mit
Halogen sein kann, oder
wobei jede der Cycloalkylgruppen 5-, 6- oder 7-gliedrig ist, eine oder
zwei -CH2-Gruppen, welche nicht direkt miteinander verknüpft sind, so durch -O- ersetzt sein können, dass das O-Atom mit dem N-Atom verknüpft ist, an das R² über wenigstens zwei C-Atome gebunden ist, oder
c) Phenyl, (C1-3)-Alkyl-phenyl, Heteroaryl oder (C1-3)-Alkyl-heteroaryl, wobei die Heteroarylgruppen 5- oder 6-gliedrig mit 1 bis 3 Heteroatomen sind, welche ausgewählt sind unter N, O und S, wobei die Phenyl- und Heteroarylgruppen mono-, di- oder tri-substituiert mit Substituenten sein können, welche ausgewählt sind unter Halogen,-OH, (C1-4)-Alkyl, O-(C1-4)-Alkyl, S-(C1-4)-Alkyl, -NH2, -NH((C1-4)-Alkyl) und -N((C1-4)-Alkyl)2, -CONH2 und -CONH-(C1-4)-Alkyl;
R³ PRT, H oder (C1-6)-Alkyl ist;
L unabhängig ausgewählt ist unter C oder N, vorausgesetzt, dass nicht mehr als drei N aufeinander folgen, wobei jedes C mit einem oder mehreren A³ substituiert ist;
L¹ unabhängig ausgewählt ist unter C oder N, vorausgesetzt, dass nicht mehr als drei N aufeinander folgen, die jeweils mit einem oder mehreren A³ substituiert sind;
Z O, S, C oder N ist, substituiert mit A³;
Z^{2a} H, (C1-10)-Alkyl, (C2-10)-Alkenyl, (C2-10)-Alkinyl ist, wobei jedes Kohlenstoffatom durch ein Heteroatom ersetzt sein kann, ausgewählt unter O, S oder N, oder Z^{2a} bildet gegebenenfalls einen Carbocyclus oder Heterocyclus mit Q¹ oder einem beliebigen A³;
Z^{2b} H, (C1-6)-Alkyl, (C2-8)-Alkenyl, (C2-8)-Alkinyl ist;
Q¹ (C1)-Alkylen, (C2-8)-Alkenylen oder (C2-8)-Alkinylen ist;
A³ unabhängig ausgewählt ist unter PRT, H, -OH, -C(O)OH, -(CH₂)ₘ-, -C(O)O-, -NH-, Cyano, Alkyl, Alkenyl, Alkinyl, Amino, Amido, Imido, Imino, Halogen, CF₃, CH₂CF₃, Cycloalkyl, Nitro, Aryl, Aralkyl, Alkoxy, Aryloxy, Heterocyclus, Heteroaryl, -C(A²)₃, -C(A²)₂-C(O)A², -C(O)A², -C(O)OA², -O(A²), -N(A²)₂, -S(A²), -CH₂P(O)(A²)(OA²), -CH₂P(O)(A²)(N(A²)₂), -CH₂P(O)(OA²)(OA²), -OCH₂P(O)(OA²)(OA²), -OCH₂P(O)(A²)(OA²), -OCH₂P(O)(A²)(N(A²)₂), -C(O)OCH₂P(O)(OA²)(OA²), -C(O)OCH₂P(O)(A²)(OA²), -C(O)OCH₂P(O)(A²)(N(A²)₂), -CH₂P(O)(OA²)(N(A²)₂), -OCH₂P(O)(OA²)(N(A²)₂), -C(O)OCH₂P(O)(OA²)(N(A²)₂), -CH₂P(O)(N(A²)₂)(N(A²)₂), -C(O)OCH₂P(O)(N(A²)₂)(N(A²)₂), -OCH₂P(O)(N(A²)₂)(N(A²)₂), -(CH₂)ₘ-Heterocyclus, -(CH₂)ₘC(O)Oalkyl, -O-(CH₂)ₘ-O-C(O)-O-Alkyl, -O-(CH₂)ᵣ-O-C(O)-(CH₂)ₘ-Alkyl, -(CH₂)ₘO-C(O)-O-Alkyl, -(CH₂)ₘO-C(O)-O-Cycloalkyl, -N(H)C(Me)C(O)O-Alkyl oder Alkoxyarylsulfonamid,
wobei jedes A³ gegebenenfalls substituiert sein kann mit 1 bis 4 -R¹, -P(O)(OA²)(OA²), -P(O)(OA²)(N(A²)₂), -P(O)(A²)(OA²), -P(O)(A²)(N(A²)₂) oder -P(O)(N(A²)₂)(N(A²)₂), Halogen, Alkyl, Alkenyl, Alkinyl, Aryl, Carbocyclus, Heterocyclus, Aralkyl, Arylsulfonamid, Arylalkylsulfonamid, Aryloxysulfonamid, Aryloxyalkylsulfonamid, Aryloxyarylsulfonamid, Alkylsulfonamid, Alkyloxysulfonamid, Alkyloxyalkylsulfonamid, -(CH₂)ₘ-Heterocyclus, -(CH₂)ₘ-C(O)O-Alkyl, -O(CH₂)ₘOC(O)O-Alkyl, -O-(CH₂)ₘ-O-C(O) -(CH₂)ₘ-Alkyl, -(CH₂)ₘ-O-C(O)-O-Alkyl, -(CH₂)ₘ-O-C(O)-O-Cycloalkyl, -N(H)C(CH₃)C(O)O-Alkyl, Alkoxy, -N(R³)(R³) oder Alkoxyarylsulfonamid, gegebenenfalls substituiert mit -R¹, oder
A³ einen carbocyclischen oder heterocyclischen Ring mit einem beliebigen anderen A³ oder Q¹ bildet;
Y₁ O, S, N(R₂), N(OR₂) oder N(N(R₂)) ist;
A² unabhängig ausgewählt ist unter H, Alkyl, Alkenyl, Alkinyl, Amino, Aminosäure, Alkoxy, Aryloxy, Cyano, Halogenalkyl, Cycloalkyl, Aryl, Heteroaryl, Alkylsulfonamid oder Arylsulfonamid, gegebenenfalls substituiert mit A³;
PRT ausgewählt ist unter -CH₂OC(=O)R⁹ und -CH₂OC(=O)OR⁹, wobei R⁹ (C1-6)-Alkyl, substituiertes (C1-6)-Alkyl, (C6-20)-Aryl oder substituiertes (C6-20)-Aryl ist:
m 0 bis 6 ist;
r 1 oder 2 ist; und
q 1 bis 10 ist.

2. Verbindung nach Anspruch 1, ausgewählt aus der Gruppe, bestehend aus:

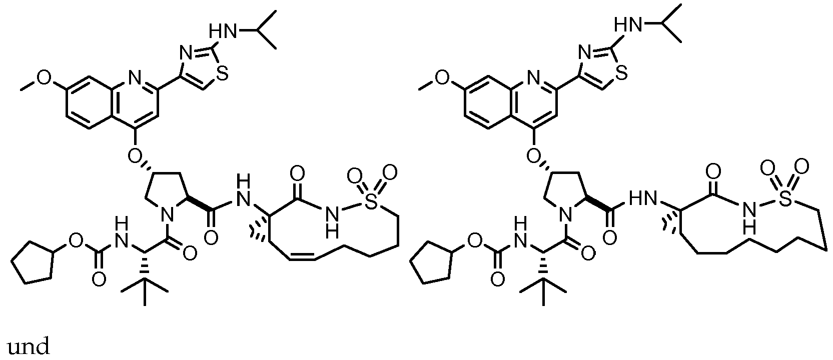

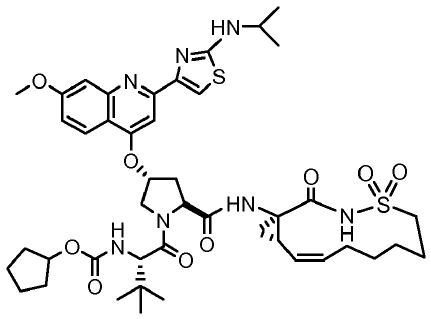

3. Verbindung der Formel II gemäß Anspruch 1, wobei R2 unter den Bedeutungen (b) und (c), wie in Anspruch 1 definiert, ausgewählt ist.

4. Pharmazeutische Zusammensetzung, umfassend die Verbindung nach einem der Ansprüche 1 bis 3 und mindestens einen pharmazeutisch verträglichen Träger.

5. Pharmazeutische Zusammensetzung nach Anspruch 4 zur Verwendung bei der Behandlung von Störungen, die mit HCV assoziiert sind.

6. Pharmazeutische Zusammensetzung nach Anspruch 5, weiterhin umfassend ein Nukleosid -Analogon.

7. Pharmazeutische Zusammensetzung nach Anspruch 6, weiterhin umfassend ein Interferon oder pegyliertes Interferon.

8. Pharmazeutische Zusammensetzung nach Anspruch 7, wobei das Nukleosid-Analogon ausgewählt ist unter Ribavirin, Viramidin Levovirin, einem L- Nukleosid und Isatoribin, und das Interferon α-Interferon oder pegyliertes Interferon ist.

9. Verbindung nach einem der Ansprüche 1 bis 3 zur Verwendung in einem Verfahren zur Behandlung von Störungen, die mit Hepatitis C assoziiert sind.

10. Verbindung nach einem der Ansprüche 1 bis 3 zur Verwendung in der medizinischen Therapie.

11. Verwendung einer Verbindung nach einem der Ansprüche 1 bis 3 zur Herstellung eines Medikaments zur Behandlung einer Störung bei einem Säuger, die mit Hepatitis C assoziiert ist.

## Revendications

1. Composé, comprenant des énantiomères de celui-ci, de formule II : 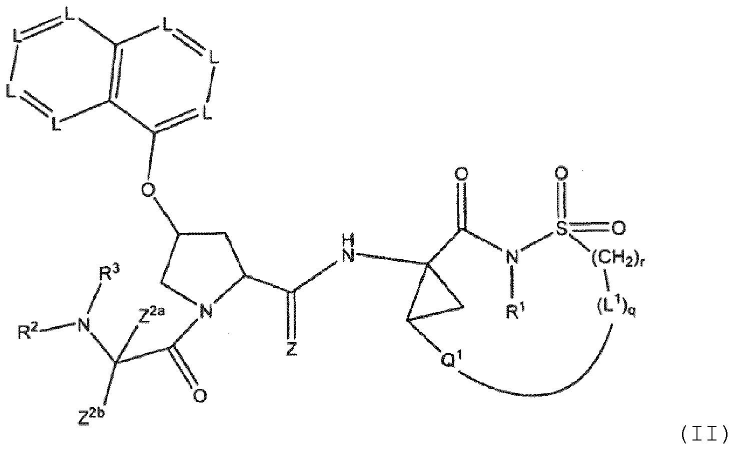 ou un sel pharmaceutiquement acceptable, ou un solvate de celui-ci,
dans lequel :
R¹ est indépendamment choisi parmi H, alkyle, alcényle, alcynyle, aryle, cycloalkyle, hétérocycle, halogène, halogénoalkyle, alkylsulfonamido, arylsulfonamido, - C(O)NHS(O)₂-, ou -S(O)₂-, facultativement substitué par un ou plusieurs A³ ;
R² est choisi parmi
a) -C(Y¹) (A³)
b) alkyle en C2-10, cycloalkyle en C3-7 ou (alkyle en C1-4)-(cycloalkyle en C3-7),
où lesdits cycloalkyle et alkyl-cycloalkyle peuvent être mono-, di- ou tri-substitués par alkyle en C1-3, ou
où lesdits alkyle, cycloalkyle et alkyl-cycloalkyle peuvent être mono- ou disubstitués par des substituants choisi parmi hydroxy et O-(alkyle en C1-4), ou
où chacun desdits groupes alkyle peut être mono-, di- ou tri-substitué par halogène, ou
où chacun desdits groupes cycloalkyle est de 5, 6 ou 7 chaînons, un ou deux groupes -CH2- n'étant pas directement liés l'un à l'autre peuvent être remplacés par -O- de sorte que l'atome 0 soit lié à l'atome N auquel R² est lié via au moins deux atomes C, ou
c) phényle, (alkyle en C1-3)-phényle, hétéroaryle ou (alkyle en C1-3)-hétéroaryle, les groupes hétéroaryle étant de 5 ou 6 chaînons ayant de 1 à 3 hétéroatomes choisis parmi N, 0 et S, lesdits groupes phényle et hétéroaryle pouvant être mono-, di- ou trisubstitués par des substituants choisis parmi halogène, -OH, alkyle en C1-4, O-(alkyle en C1-4), S-(alkyle en C1-4), -NH2, -NH(alkyle en C1-4) et -N(alkyle en C1-4)2, -CONH2 et -CONH-(alkyle en C1-4) ;
R³ est PRT, H ou alkyle en C1,-6 ;
L est indépendamment choisi parmi C ou N, à condition qu'il n'y ait pas plus de trois N consécutifs, chaque C étant substitué par un ou plusieurs A³;
L¹ est indépendamment choisi parmi C ou N, à condition qu'il n'y ait pas plus de trois N consécutifs, chacun substitué par un ou plusieurs A³ ;
Z est 0, S, C ou N, substitué par A³ ;
Z^{2a} est H, alkyle en C1-10, alcényle en C2-10, alcynyle en C2-10, où tout atome de carbone peut être remplacé par un hétéroatome choisi parmi 0, S ou N, ou
Z^{2a} forme éventuellement un carbocyle ou un hétérocycle avec Q¹, ou un A³ quelconque ;
Z^{2b} est H, alkyle en C1-6, alcényle en C2-8, alcynyle en C2-8 ;
Q¹ est alkylène en C1, alcénylène en C2-8, ou alcynylène en C2-8 ;
A³ est indépendamment choisi parmi PRT, H, -OH, -C(O)OH, -(CH₂)ₘ-, -C(O)O-, -NH-, cyano, alkyle, alcényle, alcynyle, amino, amido, imido, imino, halogène, CF₃, CH₂CF₃, cycloalkyle, nitro, aryle, aralkyle, alcoxy, aryloxy, hétérocycle, hétéroaryle, -C(A²)₃, -C(A²)₂-C(O)A², -C(O)A², -C(O) OA², -O(A²) ; -N(A²)₂, -S(A²), -CH₂P(O)(A²)(OA²), -CH ₂P(O)(A²)(N(A²)₂), -CH₂P(O)(OA²)(OA²), -OCH₂P(0) (OA²) (OA²), -OCH₂P(0) (A²)(OA²), -OCH₂P(0) (A²)(N(A²)₂), -C( 0) OCH₂P(0) (OA²)(OA²), -C(0) OCH₂P(0) (A²)(OA²), -C (0) 0 CH₂P(O)(A²)(N(A²)₂), -CH₂P(0) (OA²)(N(A²)₂) ,-OCH₂P(O)(OA²)(N(A²)₂), -C(O)OCH₂P(O)(OA²)(N(A²)₂), -CH ₂P(O)(N(A²)₂)(N(A²)₂), -C(O) OCH₂P(O) (N (A²)₂)(N(A²)₂), -OCH₂P(O)(N(A²)₂)(N(A²)₂), -(CH₂)ₘ-hétérocycle, -(CH ₂)ₘC(O)Oalkyle, -O-(CH₂)ₘ-O-C(O)-Oalkyle, -O-(CH₂)ᵣ-O-C(O)-(CH₂)ₘ-alkyle, -(CH₂)ₘO-C(0) -O-alkyle, -(CH₂)ₘO-C(0) -O-cycloalkyle, -N(H)C(Me)C(O)O-alkyle, ou alcoxy-arylsulfonamide,
chaque A³ pouvant être facultativement substitué par 1 à 4-R¹-P(0) (OA²)(OA²), -P(O)(OA²)(N(A²)₂), -P(O)(A²)(OA²) , -P(O)(A²)(N(A²)₂), ou -P(O)(N(A²)2) (N (A2) ₂), halogène, alkyle, alcényle, alcynyle, aryle, carbocycle, hétérocycle, aralkyle, arylsulfonamide, aryl alkylsulfonamide, aryloxysulfonamide, aryloxyalkylsulfonamide, aryloxyarylsulfonamide, alkylsulfonamide, alkyloxysulfonamide, alkyloxyalkylsulfonamide,-(CH₂)ₘhétérocycle, -(CH₂)ₘ-C(O)O-alkyle, -O(CH₂)ₘOC(O)Oalkyle, -O-(CH₂)ₘ-O-C(O)-(CH₂)ₘ-alkyle, -(CH₂)ₘ-O-C(O)-O-alkyle, -(CH₂)ₘ-O-C(O)-O-cycloalkyle, -N(H)C(CH₃)C(O)O-alkyle, alcoxy, -N(R³)(R³) ou alcoxy-arylsulfonamide, facultativement substitué par -R¹, ou
A³ forme un cycle carbocyclique ou hétérocyclique avec un autre A³ ou Q¹ quelconque ;
Y¹ est 0, S, N(R₂), N(OR₂) ou N(N(R₂))₂ ;
A² est indépendamment choisi parmi H, alkyle, alcényle, alcynyle, amino, amino acide, alcoxy, aryloxy, cyano, haloalkyle, cycloalkyle, aryle, hétéroaryle, alkylsulfonamide, ou arylsulfonamide, facultativement substitué par A³ ;
PRT est choisi parmi -CH₂OC(=O)R⁹ et -CH₂OC(=O)OR⁹ où R⁹ est alkyle en C1-C6, alkyle en C1-C6 substitué, aryle en C6-C20 ou aryle en C6-C20 substitué ;
m est 0 à 6 ;
r est 1 à 2 ; et
q est 1 à 10.

2. Composé de la revendication 1 choisi dans le groupe constitué de : 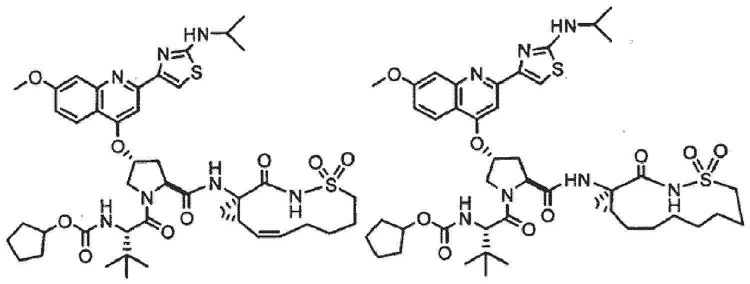 et

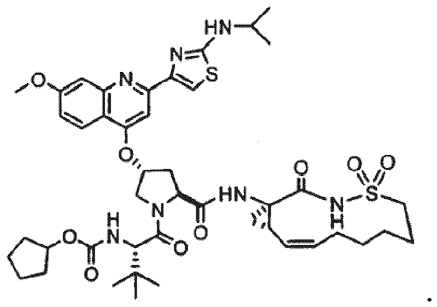

3. Composé de formule II selon la revendication 1, dans lequel R2 est choisi parmi les définitions (b) et (c) telles que définies dans la revendication 1.

4. Composition pharmaceutique comprenant le composé tel que décrit dans l'une quelconque des revendications 1 à 3 et au moins un véhicule pharmaceutiquement acceptable.

5. Composition pharmaceutique de la revendication 4 pour utilisation dans le traitement de troubles associés à VHC.

6. Composition pharmaceutique de la revendication 5 comprenant en outre un analogue de nucléoside.

7. Composition pharmaceutique de la revendication 6 comprenant en outre un interféron ou un interféron pégylé.

8. Composition pharmaceutique de la revendication 7, dans laquelle ledit analogue de nucléoside est choisi parmi la ribavirine, la viramidine la lévovirine, un L-nucléoside, et l'isatoribine et ledit interféron est l'interféron α ou un interféron pégylé.

9. Composé tel que décrit dans l'une quelconque des revendications 1 à 3 pour utilisation dans un procédé de traitement de troubles associés à l'hépatite C.

10. Composé tel que décrit dans l'une quelconque des revendications 1 à 3 pour utilisation en thérapie médicale.

11. Utilisation d'un composé tel que décrit dans l'une quelconque des revendications 1 à 3 pour préparer un médicament pour traiter un trouble associé à l'hépatite C chez un mammifère.
